# EUROPEAN PATENT APPLICATION

(11) **EP 4 549 533 A1**
(43) Date of publication of application: **07.05.2025**
(21) Application number: 25150422.1
(22) Date of filing: 24.12.2021
(51) Int. Cl.: C09K 9/02, G02C 7/10, C07D 311/78, G02B 1/04, G02B 5/23, C07D 311/94, C07D 407/04, C07D 409/04, C07D 497/04, C07F 7/04

(54) **PHOTOCHROMIC COMPOSITION, PHOTOCHROMIC ARTICLE, AND SPECTACLES**

(30) Priority: 24.12.2020 JP 2020215158; 24.12.2020 JP 2020215159; 08.02.2021 JP 2021018610
(62) Divisional of application: 21911103.6
(71) Applicant: HOYA Lens Thailand Ltd., Thanyaburi, Pathumthani 12130 (TH)
(72) Inventor: Kawakami, Hironori, Tokyo, 160-8347 (JP); Kobayashi, Kei, Tokyo, 160-8347 (JP); Matsue, Aoi, Tokyo, 160-8347 (JP); Shimada, Takuya, Tokyo, 160-8347 (JP); Yamashita, Teruo, Tokyo, 160-8347 (JP)
(74) Representative: Hoffmann Eitle

(57) **Abstract**

A photochromic composition containing one or more compounds represented by General Formula A, one or more compounds represented by General Formula B, and one or more compounds represented by General Formula C is provided. In General Formula A, R¹ to R⁶, B¹, and B² each independently represent a hydrogen atom or a substituent. In General Formula B, R⁷ to R¹², B³, and B⁴ each independently represent a hydrogen atom or a substituent, and R¹³ and R¹⁴ each independently represent an electron-donating group. In General Formula C, R¹⁵ to R²⁰, B⁵, and B⁶ each independently represent a hydrogen atom or a substituent, and one of R²¹ and R²² represents a hydrogen atom and the other represents an electron-donating group.

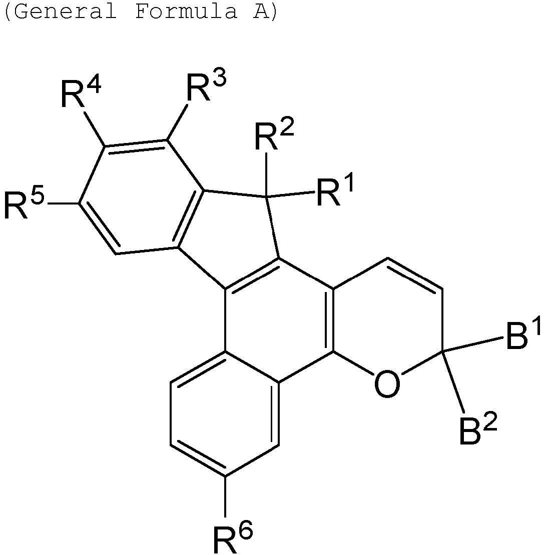

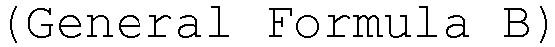

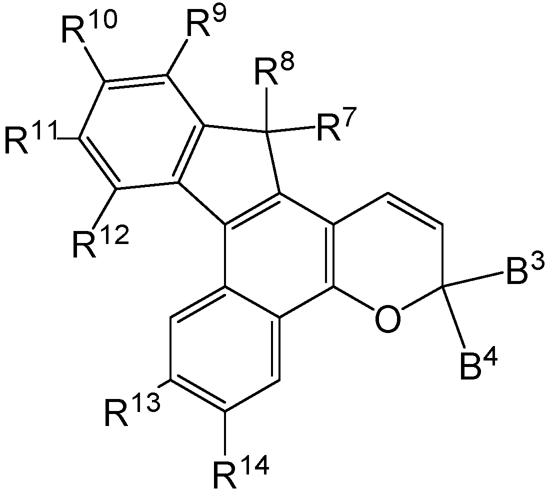

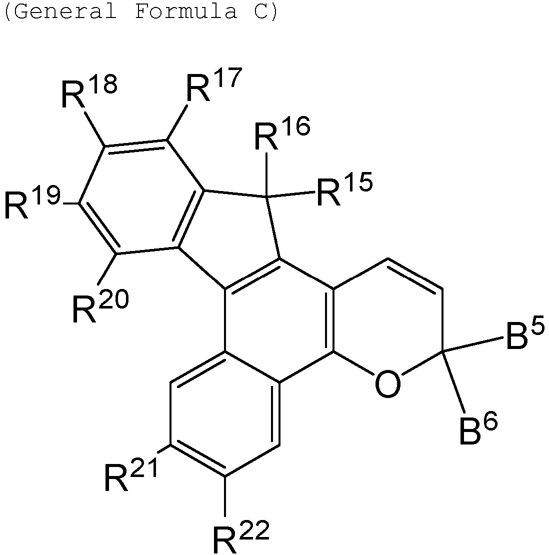

## Description

### [Technical Field]

The present invention relates to a photochromic composition, a photochromic article, and spectacles.

### [Background Art]

A photochromic compound is a compound having properties (photochromic properties) of coloring under irradiation with light in a wavelength range having photoresponsivity and fading under non-irradiation. For example, PTL 1 discloses a naphthopyran-based compound having photochromic properties.

### [Citation List]

### [Patent Literature]

[PTL 1] WO 2000/15631

### [Summary of Invention]

### [Technical Problem]

Examples of methods of imparting photochromic properties to optical articles such as spectacle lenses include a method of incorporating a photochromic compound into a substrate, and a method of forming a layer containing a photochromic compound. Examples of performance desired for such optical articles to which photochromic properties have been imparted include a high coloring density when coloring in the visible range (wavelength: 380 to 780 nm).

An object of one aspect of the present invention is to provide a photochromic article having a high coloring density when coloring in the visible range.

### [Solution to Problem]

One aspect of the present invention relates to a photochromic article containing: one or more compounds represented by General Formula A; one or more compounds represented by General Formula B; and one or more compounds represented by General Formula C.

Another aspect of the present invention relates to a photochromic composition containing: one or more compounds represented by General Formula A; and one or more compounds represented by General Formula B.

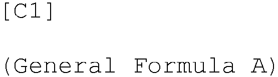

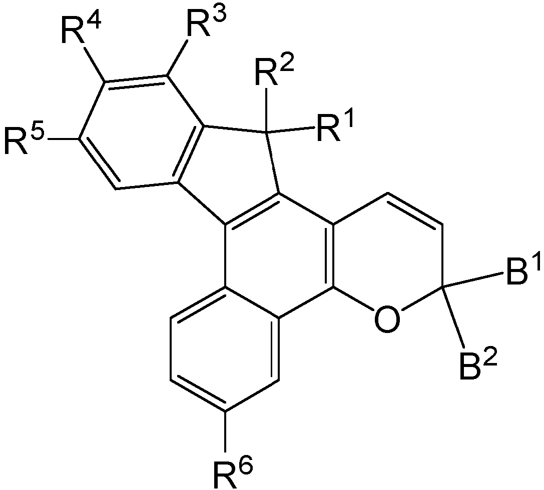

In General Formula A, R¹ to R⁶, B¹, and B² each independently represent a hydrogen atom or a substituent.

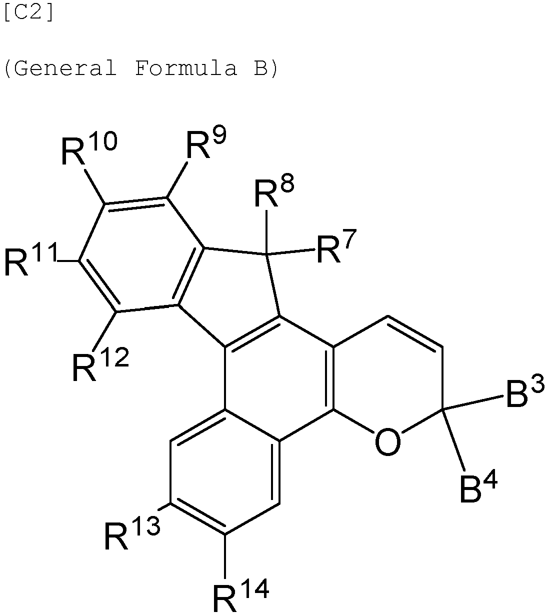

In General Formula B, R⁷ to R¹², B³, and B⁴ each independently represent a hydrogen atom or a substituent, and R¹³ and R¹⁴ each independently represent an electron-donating group.

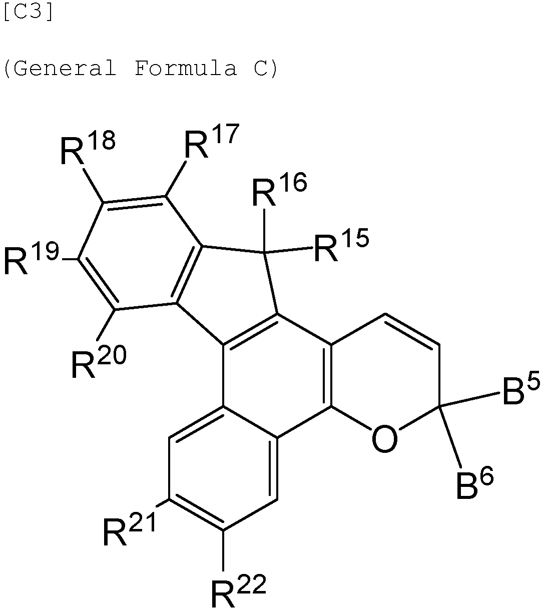

In General Formula C, R¹⁵ to R²⁰, B⁵, and B⁶ each independently represent a hydrogen atom or a substituent, and one of R²¹ and R²² represents a hydrogen atom and the other represents an electron-donating group.

As a result of intensive studies by the inventors of the present invention, they newly found that by combining the compound represented by General Formula A, the compound represented by General Formula B, and the compound represented by General Formula C, and a photochromic article that can color at a high density in the visible range can be provided. This is presumed to be because the compound represented by General Formula A, the compound represented by General Formula B, and the compound represented by General Formula C have different absorption peak positions and/or peak intensities in the visible range, and therefore, by combining these compounds, coloring at a high density in a wide wavelength range becomes possible. However, the present invention is not limited by the presumption described in the present specification. Furthermore, it became clear that by combining the compound represented by General Formula A, the compound represented by General Formula B, and the compound represented by General Formula C, a photochromic article exhibiting a fast fading speed can be provided.

### [Advantageous Effects of Invention]

According to one aspect of the present invention, a photochromic article having a high coloring density when coloring in the visible range can be provided.

### [Description of Embodiments]

As an example, a photochromic compound undergoes structural conversion into a colored product via an excited state upon irradiation with light such as sunlight. The structure after structural conversion via irradiation with light can be called a "colored product". In contrast, the structure before irradiation with light can be called a "colorless product". However, the term "colorless" in the colorless product is not limited to being completely colorless and also includes a case in which a color is lighter than that of the colored product. Each of the structure of General Formula A, the structure of General Formula B, and the structure of General Formula C is the structure of the colorless product.

In the present invention and the present specification, the term "photochromic article" refers to an article containing a photochromic compound. A photochromic article according to one aspect of the present invention contains, as photochromic compounds, one or more compounds represented by General Formula A, one or more compounds represented by General Formula B, and one or more compounds represented by General Formula C. The photochromic compound can be contained in a substrate of the photochromic article and/or can be contained in a photochromic layer in the photochromic article having a substrate and the photochromic layer. The "photochromic layer" is a layer containing a photochromic compound.

In the present invention and the present specification, the term "photochromic composition" refers to a composition containing the photochromic compound. The photochromic composition according to one aspect of the present invention contains, as photochromic compounds, one or more compounds represented by General Formula A, one or more compounds represented by General Formula B, and one or more compounds represented by General Formula C, and can be used for the production of the photochromic article according to one aspect of the present invention.

In the present invention and the present specification, various substituents, that is, substituents that can be represented by any of R¹, R², B¹, and B² in General Formula A and R⁷ to R¹², B³, and B⁴ in General Formula B and substituents that can be represented by any of R¹⁵ to R²⁰, R⁵, and R⁶ in General Formula C and substituents when each group described later has a substituent may each independently be the following substituent:
a substituent R^{m} selected from the group consisting of a linear or branched alkyl group having 1 to 18 carbon atoms such as a hydroxy group, a methyl group, an ethyl group, a propyl group, a butyl group, a pentyl group, and a hexyl group; a cycloaliphatic alkyl group of a single-ring type or a multi-ring type such as a bicyclic ring which has 5 to 18 carbon atoms such as a cyclopentyl group and a cyclohexyl group; a linear or branched alkoxy group having 1 to 24 constituent atoms such as a methoxy group, an ethoxy group, and a butoxy group; a non-aromatic cyclic substituent having 1 to 24 constituent atoms; a linear or branched perfluoroalkyl group having 1 to 18 carbon atoms such as a trifluoromethyl group; a linear or branched perfluoroalkoxy group such as a trifluoromethoxy group; a linear or branched alkylsulfide group having 1 to 24 constituent atoms such as a methylsulfide group, an ethylsulfide group, and a butylsulfide group; an aryl group such as a phenyl group, a naphthyl group, an anthracenyl group, a fluoranthenyl group, a phenanthryl group, a pyranyl group, a perylenyl group, a styryl group, and a fluorenyl group; an aryloxy group such as a phenyloxy group; an arylsulfide group such as a phenylsulfide group; a heteroaryl group such as a pyridyl group, a furanyl group, a thienyl group, a pyrrolyl group, a benzofuranyl group, a benzothiophenyl group, an indolyl group, a dibenzofuranyl group, a dibenzothiophenyl group, a carbazolyl group, a diazolyl group, a triazolyl group, a quinolinyl group, a phenothiazinyl group, a phenoxazinyl group, a phenazinyl group, a thianthryl group, and an acridinyl group; an amino group (-NH₂); a monoalkylamino group such as a monomethylamino group; a dialkylamino group such as a dimethylamino group; a monoarylamino group such as a monophenylamino group; a diarylamino group such as a diphenylamino group; a cyclic amino group such as a piperidino group, a morpholino group, a thiomorpholino group, a tetrahydroquinolino group, and a tetrahydroisoquinolino group; an ethynyl group; a mercapto group; a silyl group; a sulfonic acid group; an alkylsulfonyl group; a formyl group; a carboxy group; a cyano group; and a halogen atom such as a fluorine atom, a chlorine atom, a bromine atom, and an iodine atom, or
a substituent in which R^{m} is further substituted with one or more same or different R^{m}'s.

Examples of the above-mentioned substituent in which R^{m} is further substituted with one or more same or different R^{m}'s include a structure in which a carbon atom at the terminal of an alkoxy group is further substituted with an alkoxy group, and a carbon atom at the terminal of this alkoxy group is further substituted with an alkoxy group. In addition, other examples of the above-mentioned substituent in which R^{m} is further substituted with one or more same or different R^{m}'s include a structure in which two or more positions of the five substitutable positions of a phenyl group are substituted with the same or different R^{m}'s. However, the above-mentioned substituent is not limited to such examples.

In the present invention and the present specification, the terms "the number of carbon atoms" and "the number of constituent atoms" refer to the numbers including the number of carbon atoms or the number of atoms of a substituent when referring to a group having a substituent.

In addition, in the present invention and the present specification, various substituents, that is, substituents that can be represented by any of R¹, R², B¹, and B² in General Formula A and R⁷ to R¹², B³, and B⁴ in General Formula B and substituents that can be represented by any of R¹⁵ to R²⁰, R⁵, and R⁶ in General Formula C and substituents when each group described later has a substituent may each independently be a solubilizing group. In the present invention and the present specification, the term "solubilizing group" refers to a substituent that can contribute to enhancing compatibility with an arbitrary liquid or a specific liquid. As the solubilizing group, a substituent is suitable, the substituent capable of contributing to promoting thermal motion of the molecule of a compound by having the following substituent: an alkyl group having a linear, branched, or cyclic structure and having 4 to 50 carbon atoms; a linear, branched, or cyclic alkoxy group having 4 to 50 constituent atoms; a linear, branched, or cyclic silyl group having 4 to 50 constituent atoms; a substituent in which a part of the above-mentioned group has been substituted with a silicon atom, a sulfur atom, a nitrogen atom, a phosphorus atom, or the like; a substituent in which two or more of the above-mentioned groups have been combined; and the like. A compound having the solubilizing group as a substituent can be made to have a molecular association state close to that in a liquid by inhibiting the distance between solute molecules from becoming closer to prevent the solidification of a solute, or by lowering the melting point and/or glass transition temperature of a solute. Thereby, the solubilizing group can liquefy a solute or can increase the solubility of a compound having this substituent in a liquid. In one aspect, as the solubilizing group, a n-butyl group, a n-pentyl group, a n-hexyl group, and a n-octyl group which are linear alkyl groups; a tert-butyl group which is a branched alkyl group; and a cyclopentyl group and a cyclohexyl group which are cyclic alkyl groups are preferable.

The above-mentioned substituent is preferably a substituent selected from the group consisting of a methoxy group, an ethoxy group, a phenoxy group, a methylsulfide group, an ethylsulfide group, a phenylsulfide group, a trifluoromethyl group, a phenyl group, a naphthyl group, a dibenzofuranyl group, a dibenzothiophenyl group, a carbazolyl group, a phenothiazinyl group, a phenoxazinyl group, a phenazinyl group, an acridinyl group, a dimethylamino group, a diphenylamino group, a piperidino group, a morpholino group, a thiomorpholino group, a cyano group, and a solubilizing group, and is more preferably a substituent selected from the group consisting of a methoxy group, a phenoxy group, a methylsulfide group, a phenylsulfide group, a trifluoromethyl group, a phenyl group, a dimethylamino group, a diphenylamino group, a piperidino group, a morpholino group, a thiomorpholino group, a cyano group, and a solubilizing group.

In the present invention and the present specification, the term "electron-withdrawing group" refers to a substituent that more easily attracts electrons from a bonding atom side, as compared to a hydrogen atom. The electron-withdrawing group can attract electrons as a result of substituent effects such as an inductive effect and a mesomeric effect (or resonance effects). Specific examples of electron-withdrawing groups include a halogen atom (fluorine atom: -F, chlorine atom: -Cl, bromine atom: -Br, iodine atom: -I), a trifluoromethyl group: -CF₃, a nitro group: -NO₂, a cyano group: -CN, a formyl group: -CHO, an acyl group: -COR (where R is a substituent), an alkoxycarbonyl group: -COOR, a carboxy group: -COOH, a substituted sulfonyl group: -SO₂R (where R is a substituent), and a sulfo group: -SO₃H. Examples of suitable electron-withdrawing groups include a fluorine atom that is an electron-withdrawing group having a high electronegativity, and an electron-withdrawing group in which a substituent constant σₚ for para-positions based on the Hammett equation is a positive value.

In the present invention and the present specification, the term "electron-donating group" refers to a substituent that more easily donates electrons to a bonding atom side, as compared to a hydrogen atom. The electron-donating group can be a substituent that easily donates electrons due to the sum of the inductive effect, the mesomeric effect (or resonance effect), and the like. Specific examples of electron-donating groups include a hydroxy group: -OH, a thiol group: -SH, an alkoxy group: -OR (where R is an alkyl group), an alkylsulfide group: -SR (where R is an alkyl group), an arylsulfide group, an acetyl group: -OCOCH₃, an amino group: -NH₂, an alkylamide group: -NHCOCH₃, a dialkylamino group: -N(R)₂ (where two R's are the same or different alkyl groups), and a methyl group. Examples of suitable electron-donating groups include an electron-donating group in which a substituent constant σₚ for para-positions based on the Hammett equation is a negative value.

Specific examples of substituent constants σₚ for para-positions based on the Hammett equation (source: Organic Chemistry for Graduate School (Volume 1) (1988) by Hiizu IWAMURA, Ryoji NOYORI, Takeshi NAKAI, and Isao KITAGAWA) are described below.
-N(CH₃)₂: -0.83
-OCH₃: -0.27
-t-C₄H₉: -0.20
-CH₃: -0.17
-C₂H₅: -0.15
-C₆H₅: -0.01
(-H: 0) -F: +0.06
-Cl: +0.27
-Br: +0.23
-CO₂C2H5: +0.45
-CF₃: +0.54
-CN: +0.66
-SO₂CH₃: +0.72
-NO₂: +0.78

Hereinbelow, the compound represented by General Formula A, the compound represented by General Formula B, and the compound represented by General Formula C will be described in more detail.

### <Compound Represented by General Formula A>

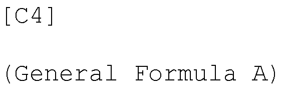

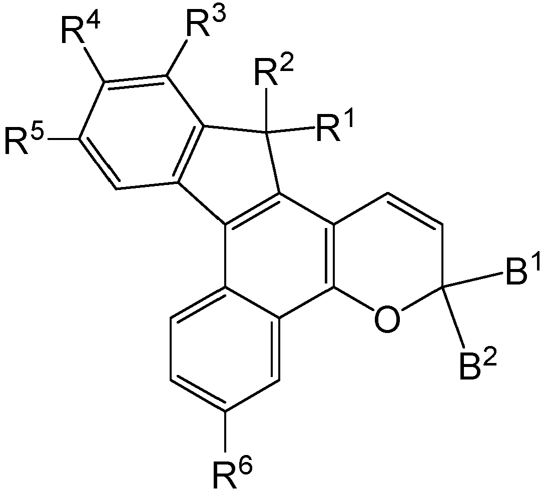

In General Formula A, R¹ to R⁶, B¹, and B² each independently represent a hydrogen atom or a substituent.

Preferably, R¹ and R² each independently represent a substituted or unsubstituted alkyl group having 1 to 20 carbon atoms, and more preferably, R¹ and R² each independently represent a methyl group, an ethyl group, a propyl group, a butyl group, a pentyl group, or a hexyl group. Further preferably, R¹ and R² each independently represent a methyl group or an ethyl group, and still further preferably, both R¹ and R² represent a methyl group or both R¹ and R² represent an ethyl group.

Preferably, B¹ and B² each independently represent a substituted or unsubstituted phenyl group. When the phenyl group has multiple substituents, two or more of these substituents may be bonded to form a ring. Specific examples of rings to be formed include rings included in exemplary compounds to be described later. The substitution position of a substituent in a substituted phenyl group is preferably a position that is the para-position with respect to the carbon atom to which B¹ and B² are bonded. Specific examples of substituents of the substituted phenyl group include substituents, which are included in exemplary compounds to be described later, such as a morpholino group, a piperidino group, a halogen atom, an alkoxy group, and the following substituents. In the present invention and the present specification, the symbol "*" relating to a partial structure of a compound indicates a bonding position with the atom to which such a partial structure is bonded.

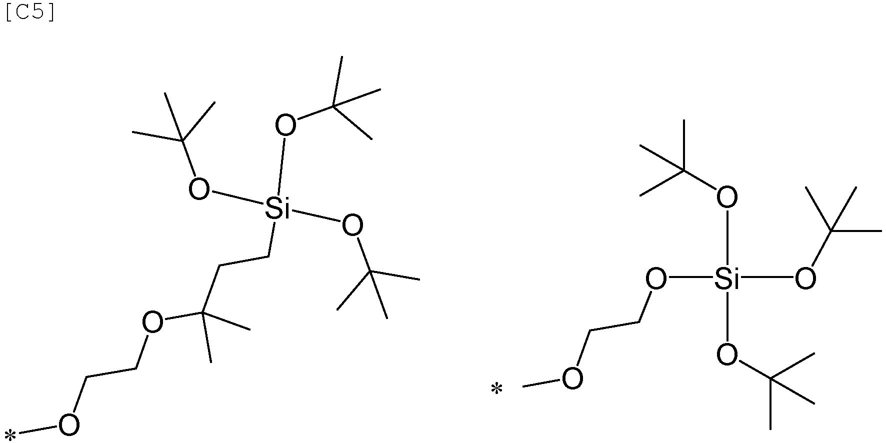

In General Formula A, R³ to R⁶ each independently represent a hydrogen atom or a substituent. In one aspect, R³ to R⁶ can all be hydrogen atoms. In another aspect, R³ to R⁶ each independently represent a hydrogen atom or an electron-withdrawing group, provided that one or more of R³ to R⁶ represent an electron-withdrawing group. As the electron-withdrawing group, a halogen atom, a perfluoroalkyl group having 1 to 6 carbon atoms, a perfluorophenyl group, a perfluoroalkylphenyl group, or a cyano group is preferable. As the halogen atom, a fluorine atom is preferable. As the perfluoroalkyl group having 1 to 6 carbon atoms, a trifluoromethyl group is preferable.

In one aspect, the compound represented by General Formula A can be the following compounds.

A compound in which only R⁴ among R³ to R⁶ is an electron-withdrawing group, and R³, R⁵, and R⁶ are hydrogen atoms.

A compound in which R⁴ and R⁶ are the same or different electron-withdrawing groups and R³ and R⁵ are hydrogen atoms among R³ to R⁶.

A compound in which R³ and R⁵ are the same or different electron-withdrawing groups and R⁴ and R⁶ are hydrogen atoms among R³ to R⁶.

Examples of the compounds represented by General Formula A include the following compounds. However, the present invention is not limited to the compounds exemplified below.

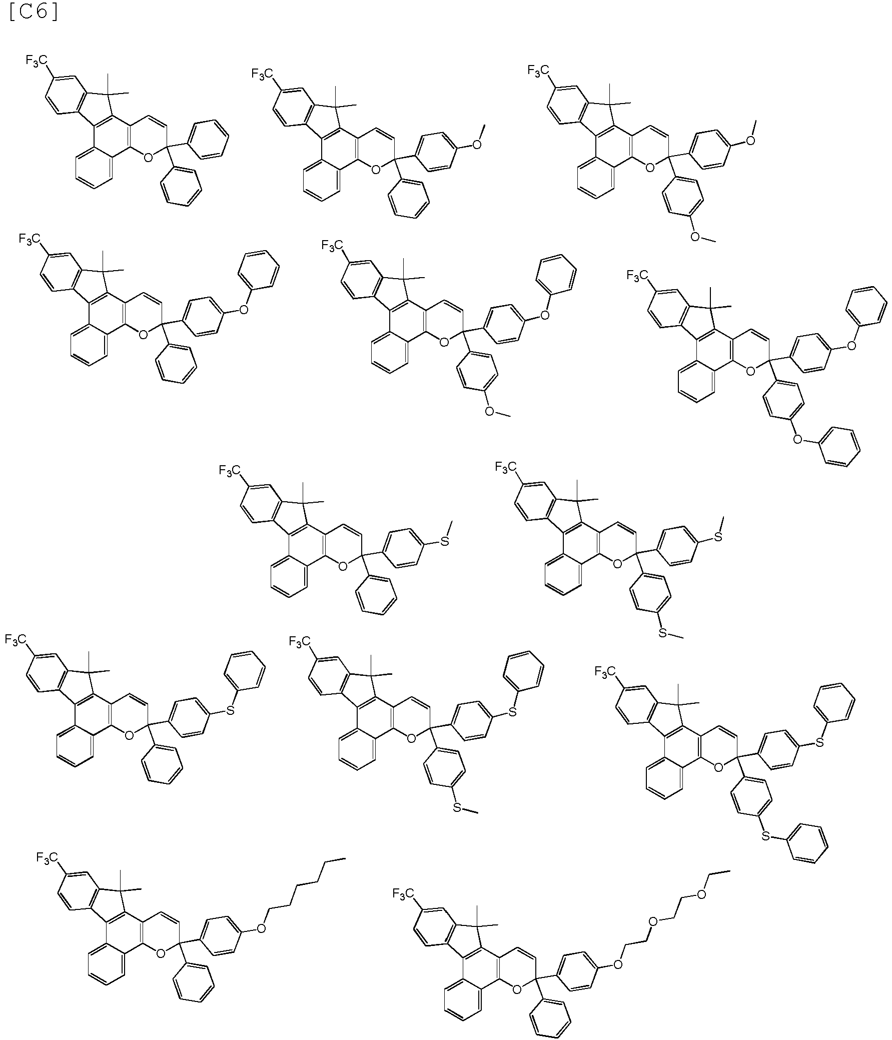

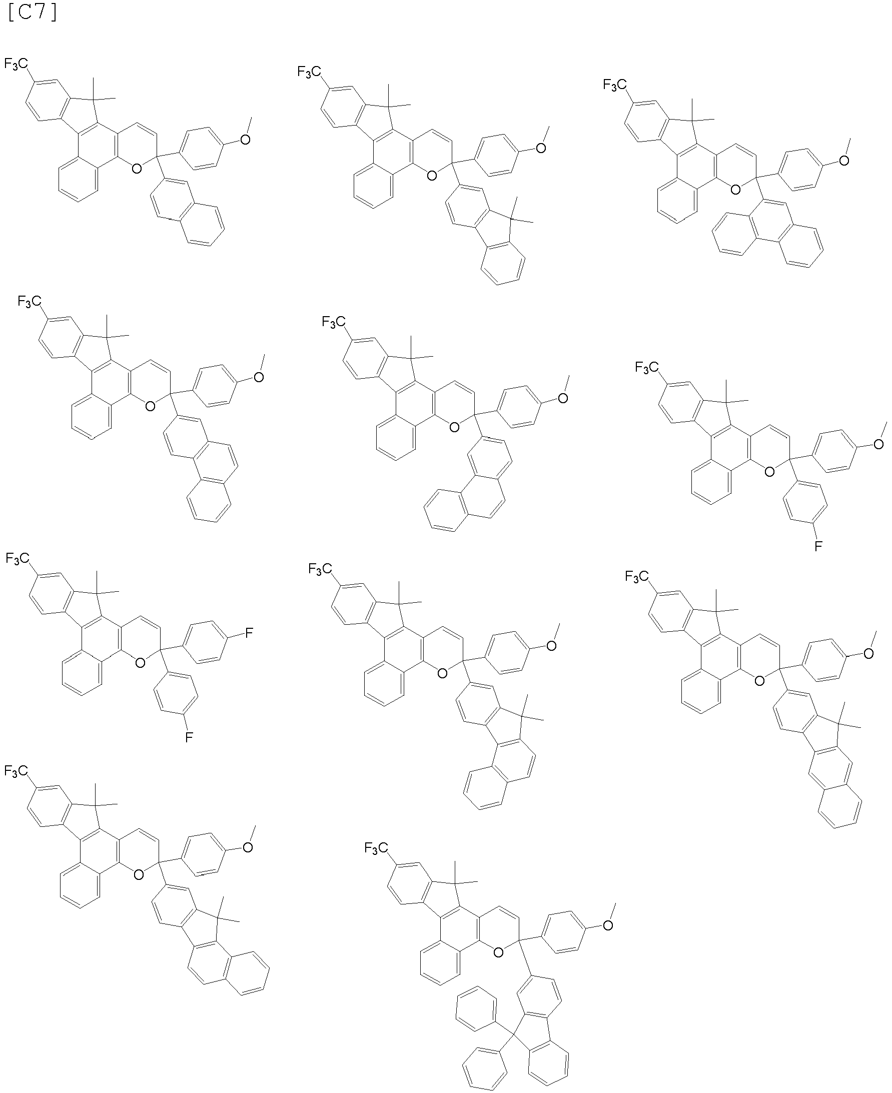

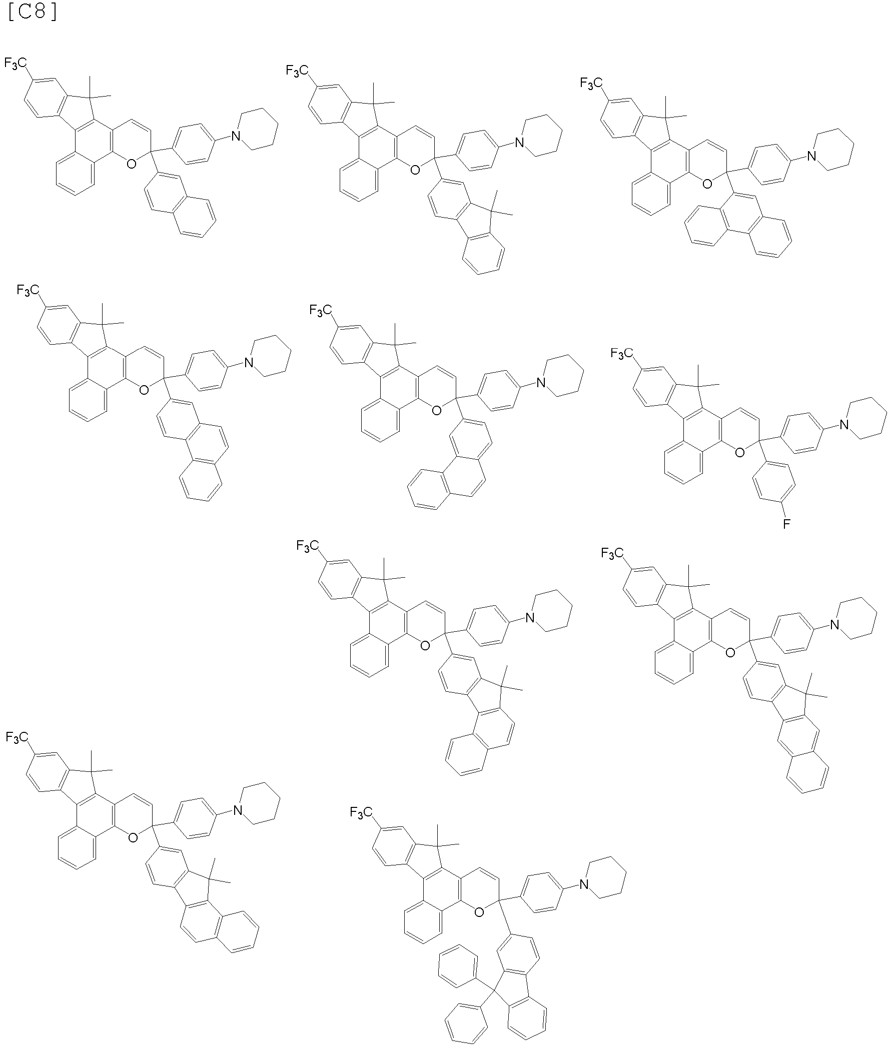

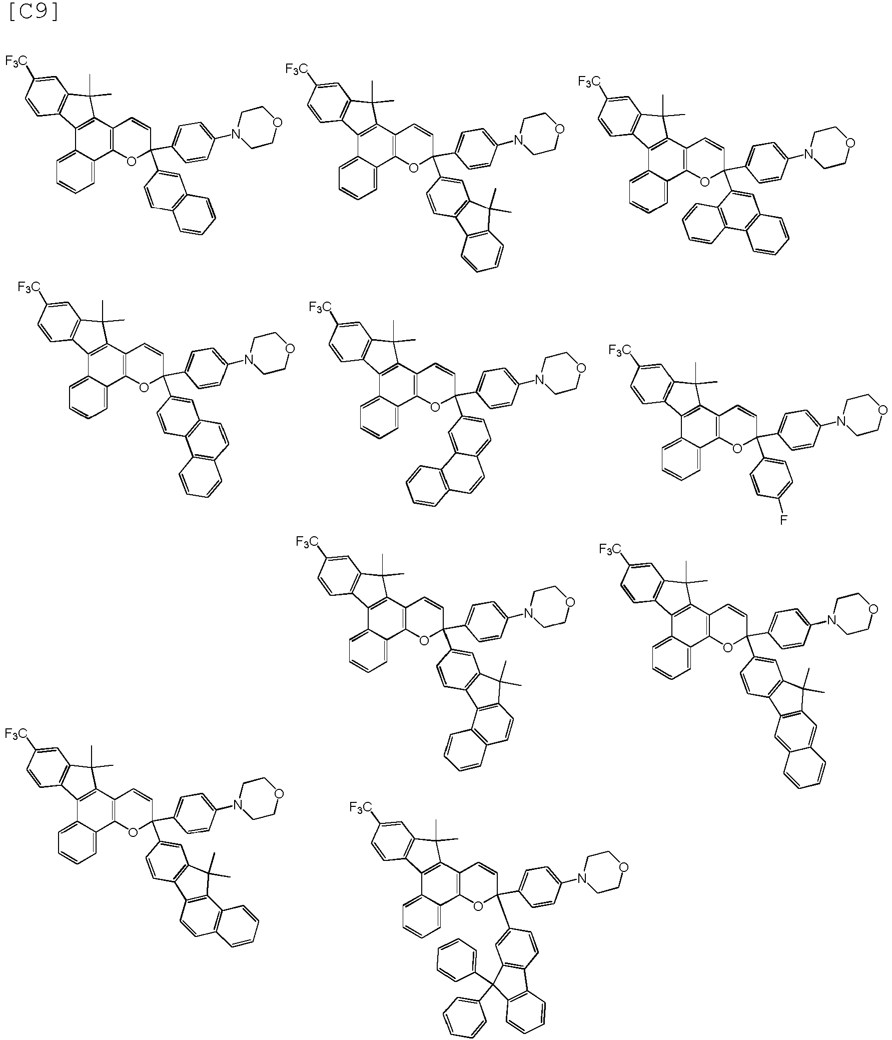

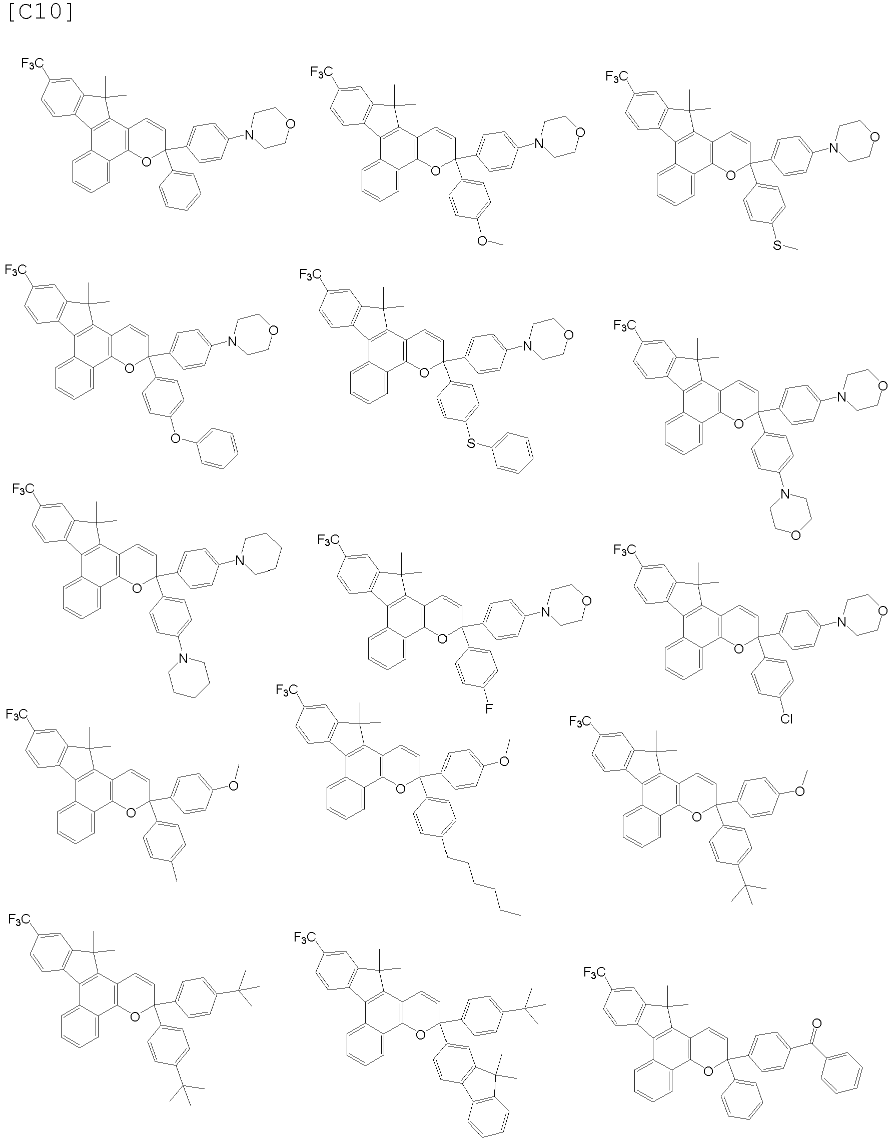

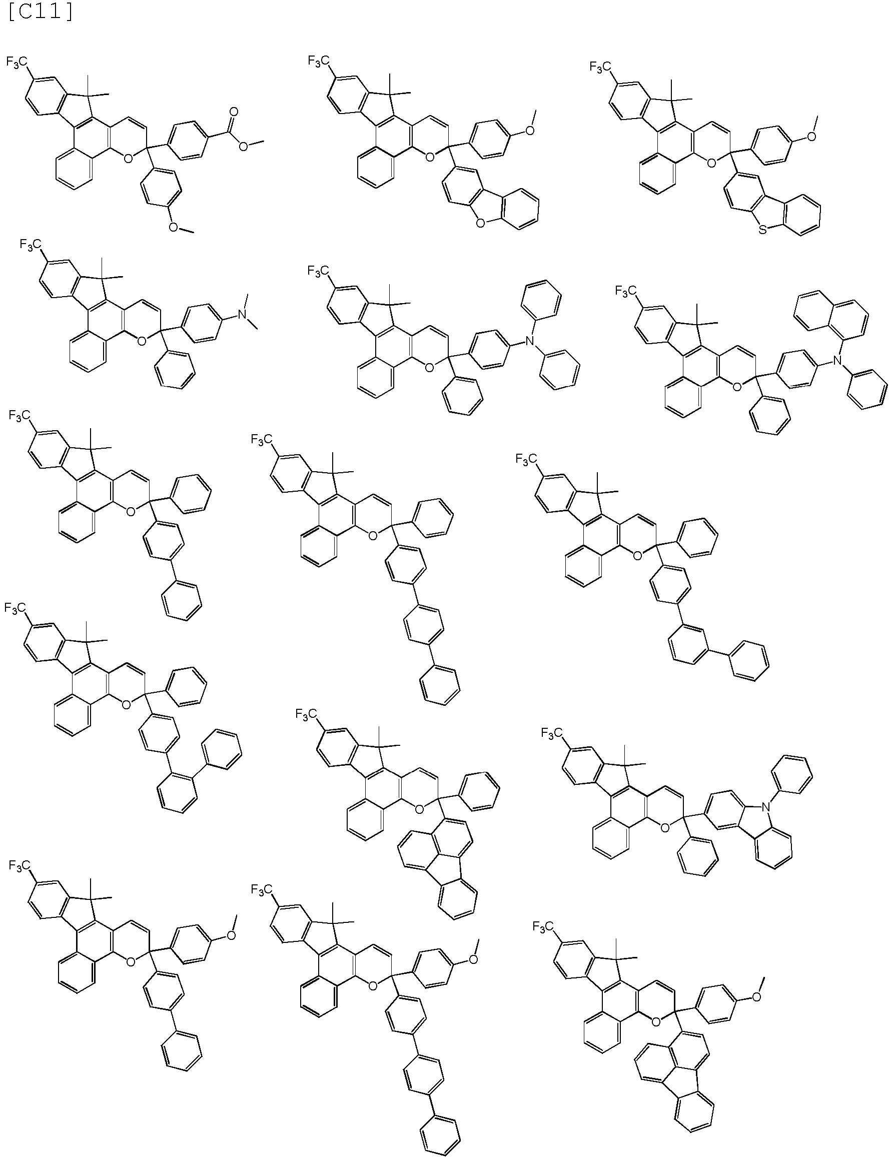

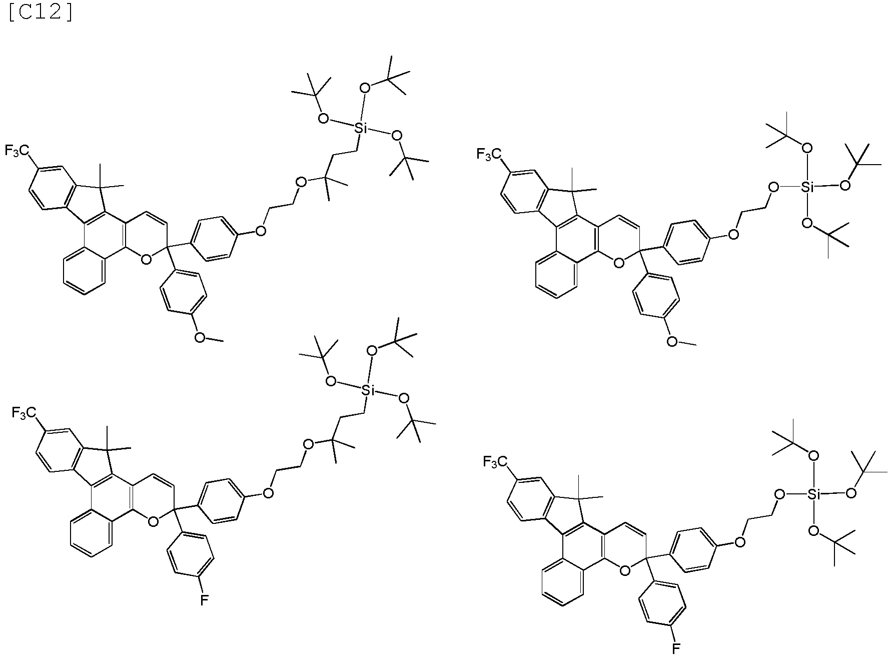

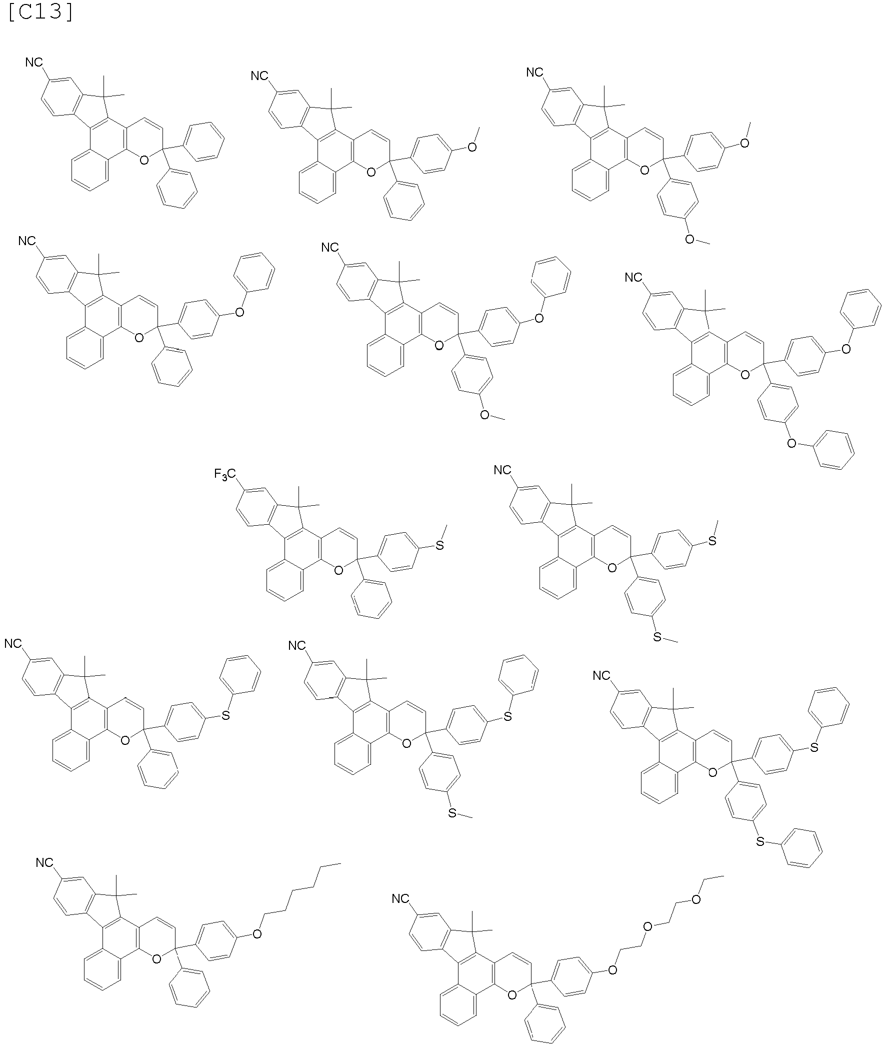

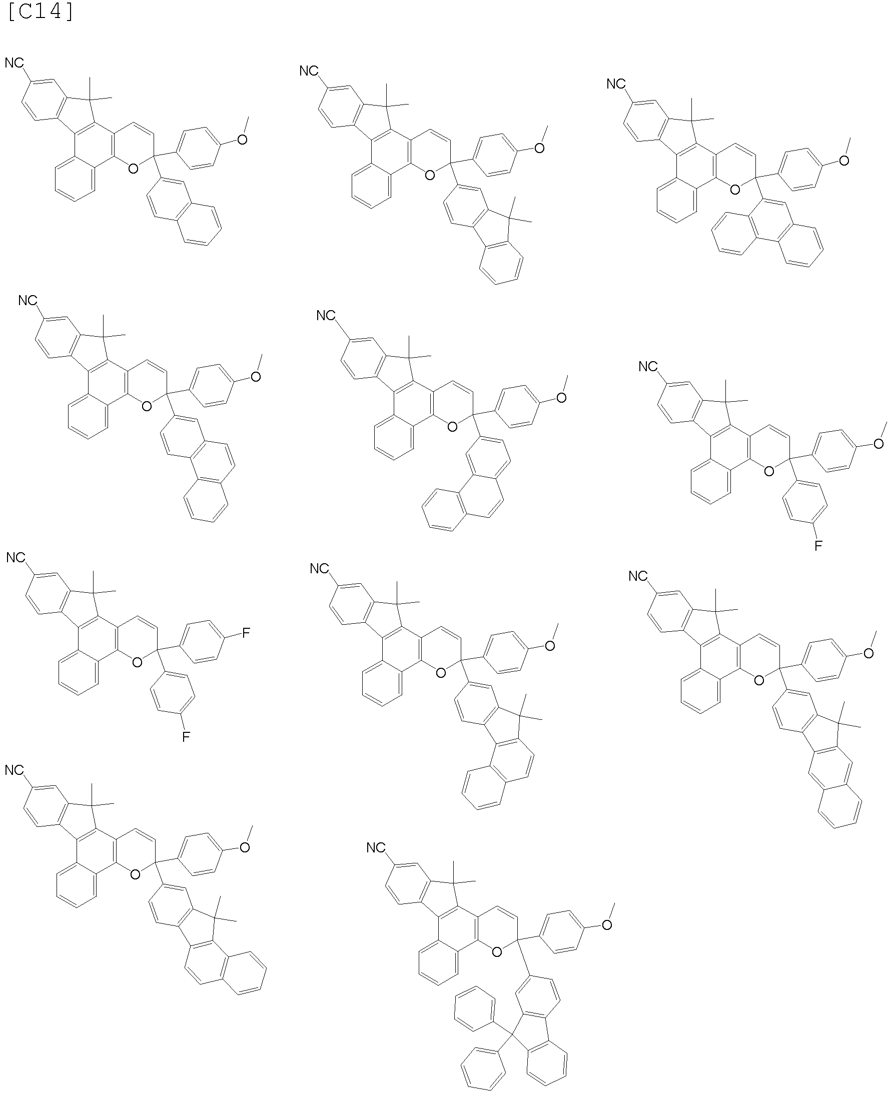

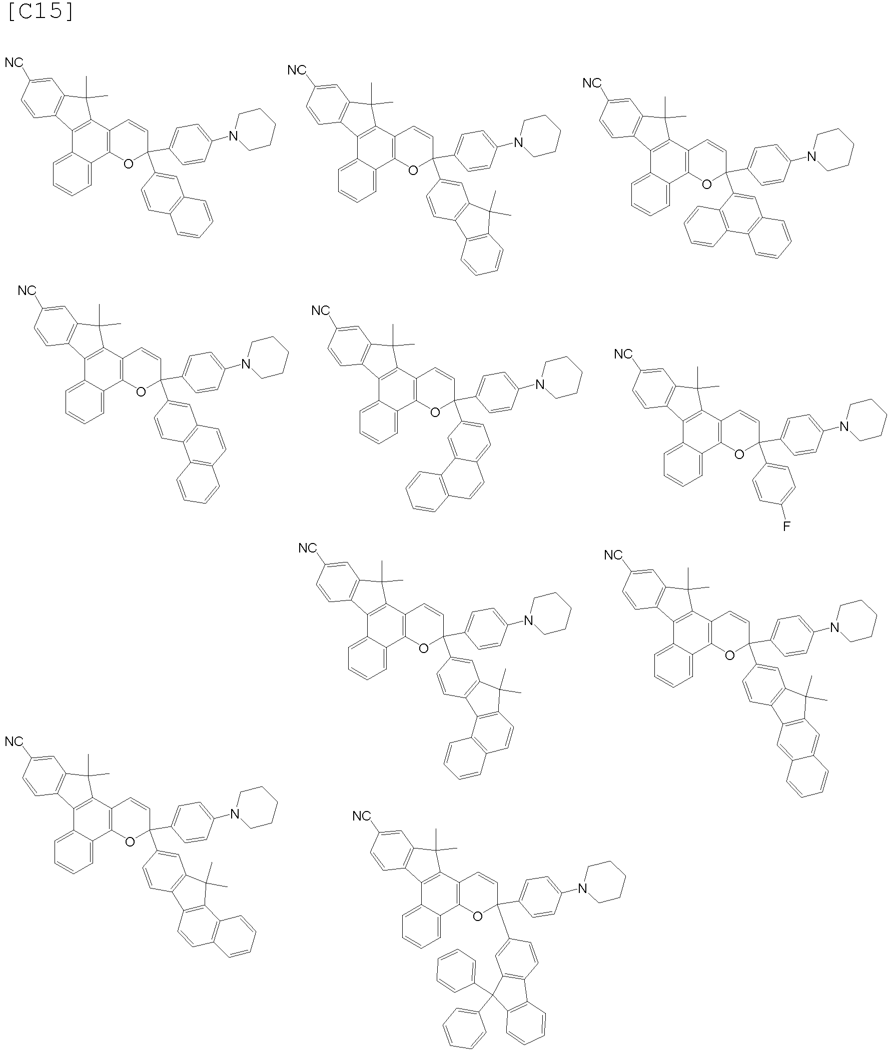

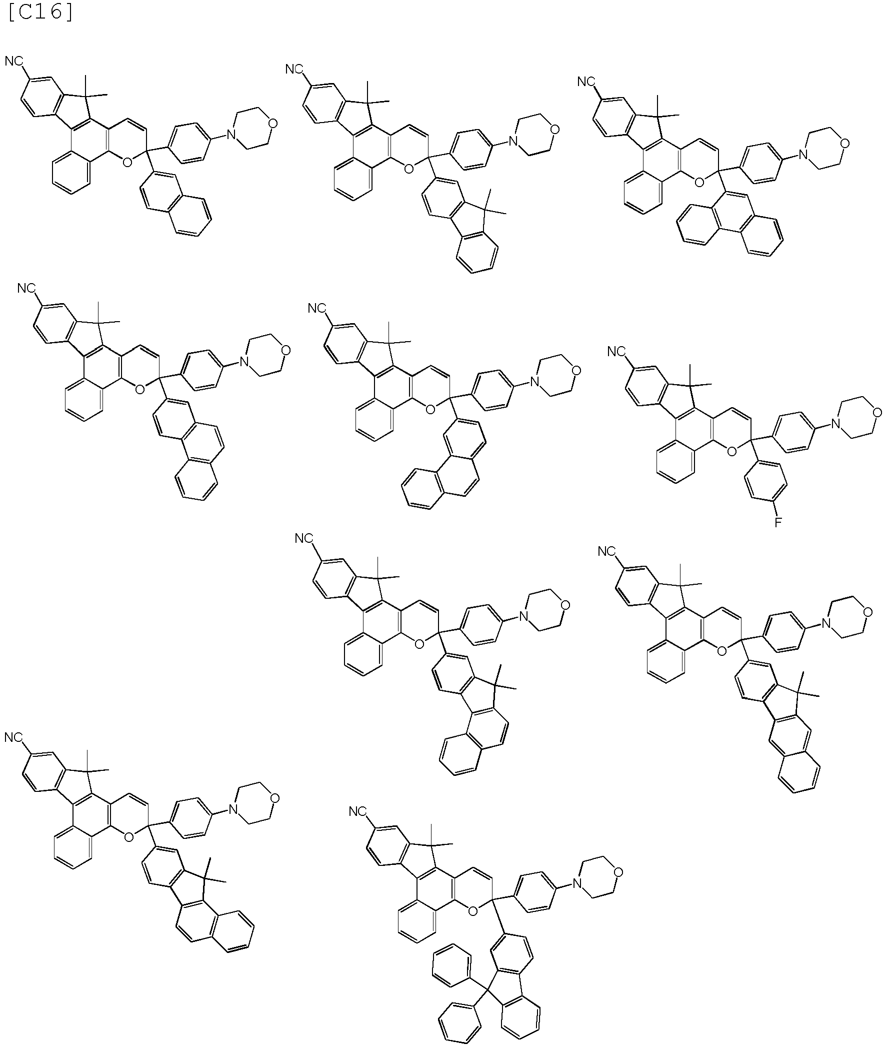

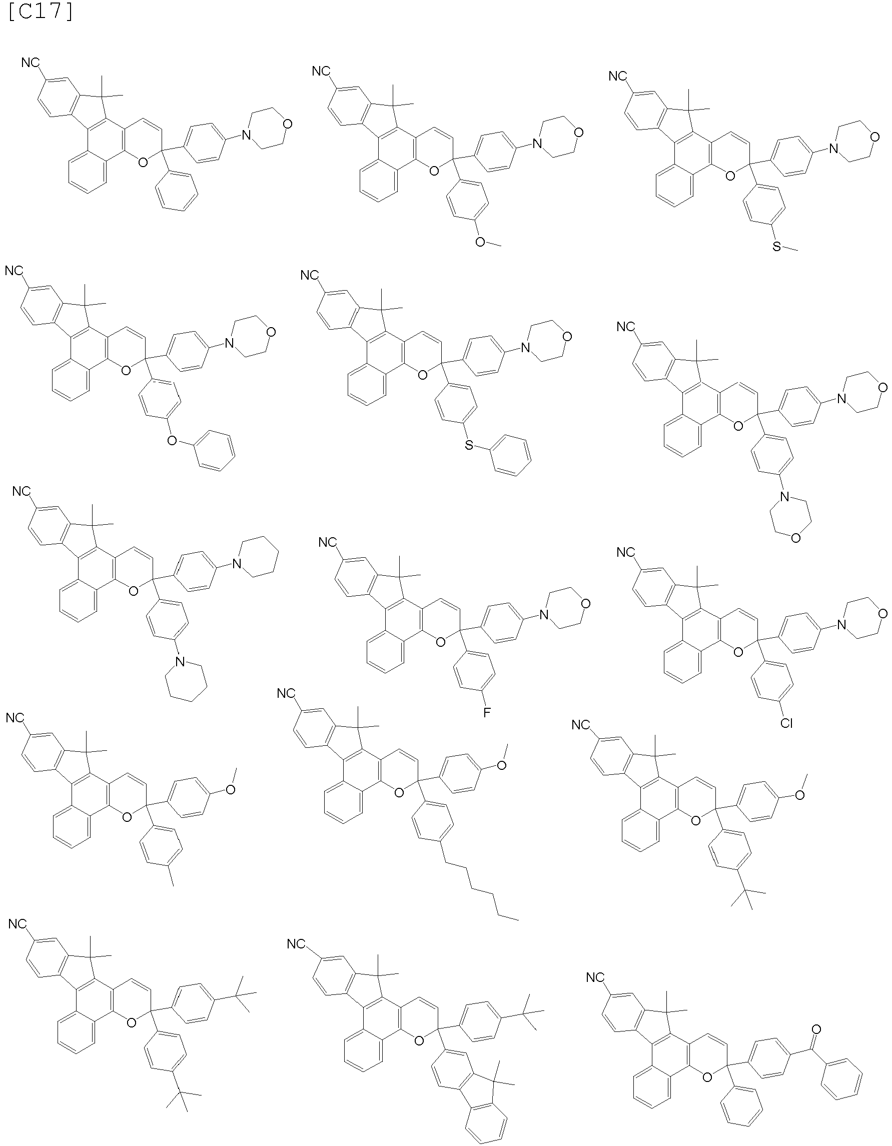

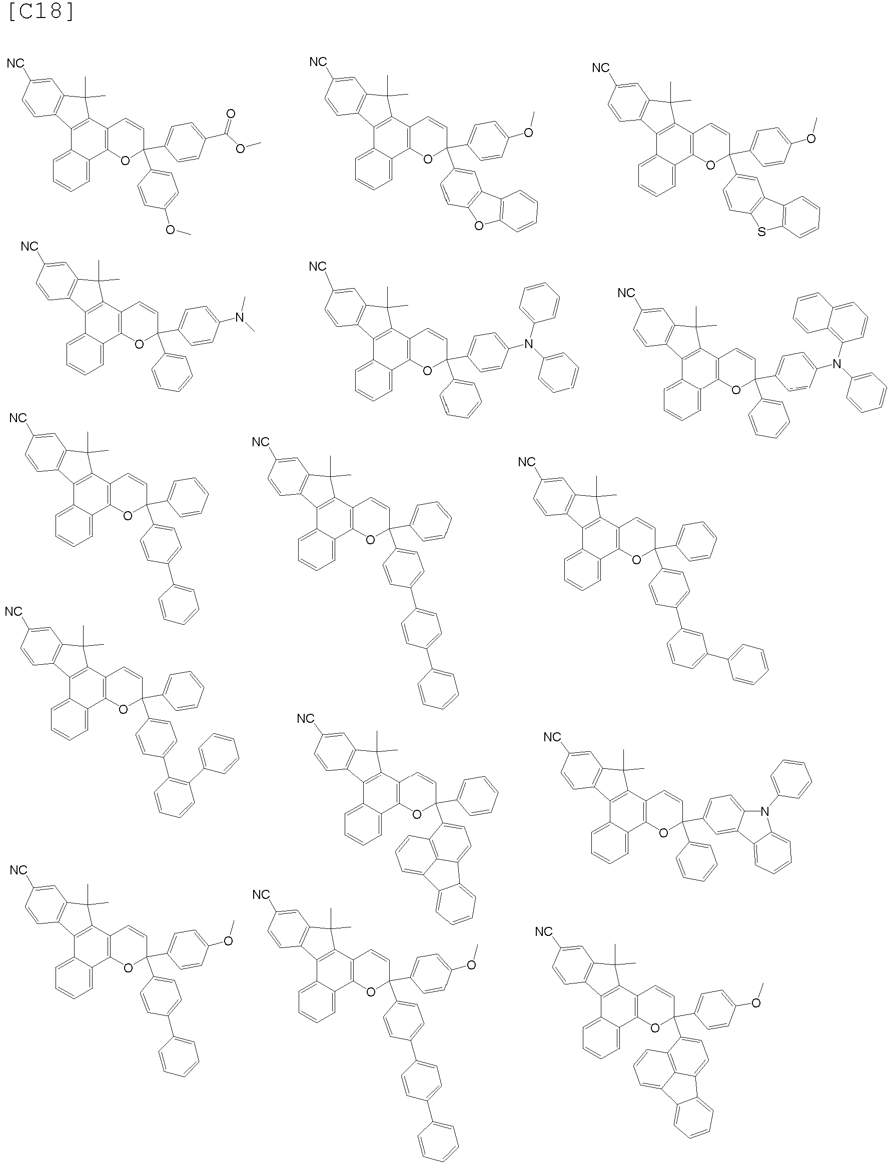

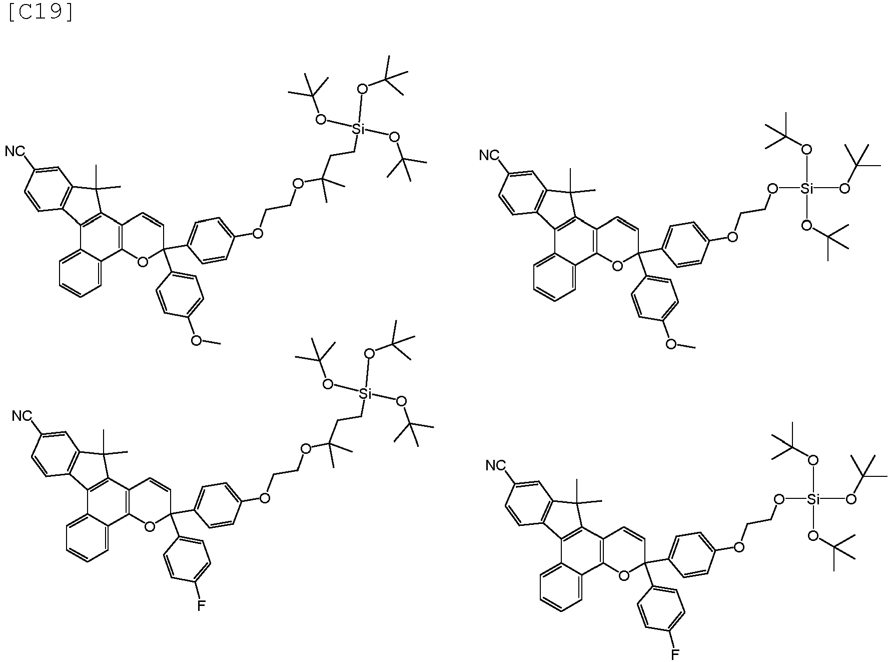

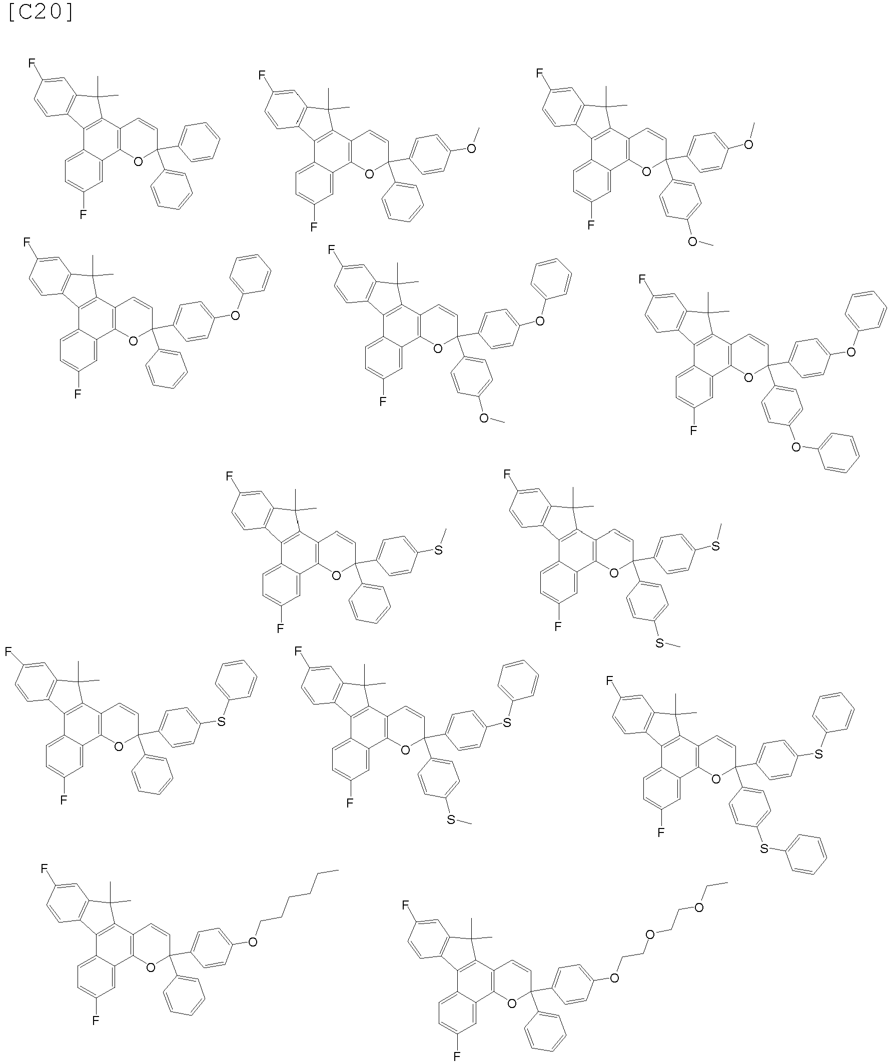

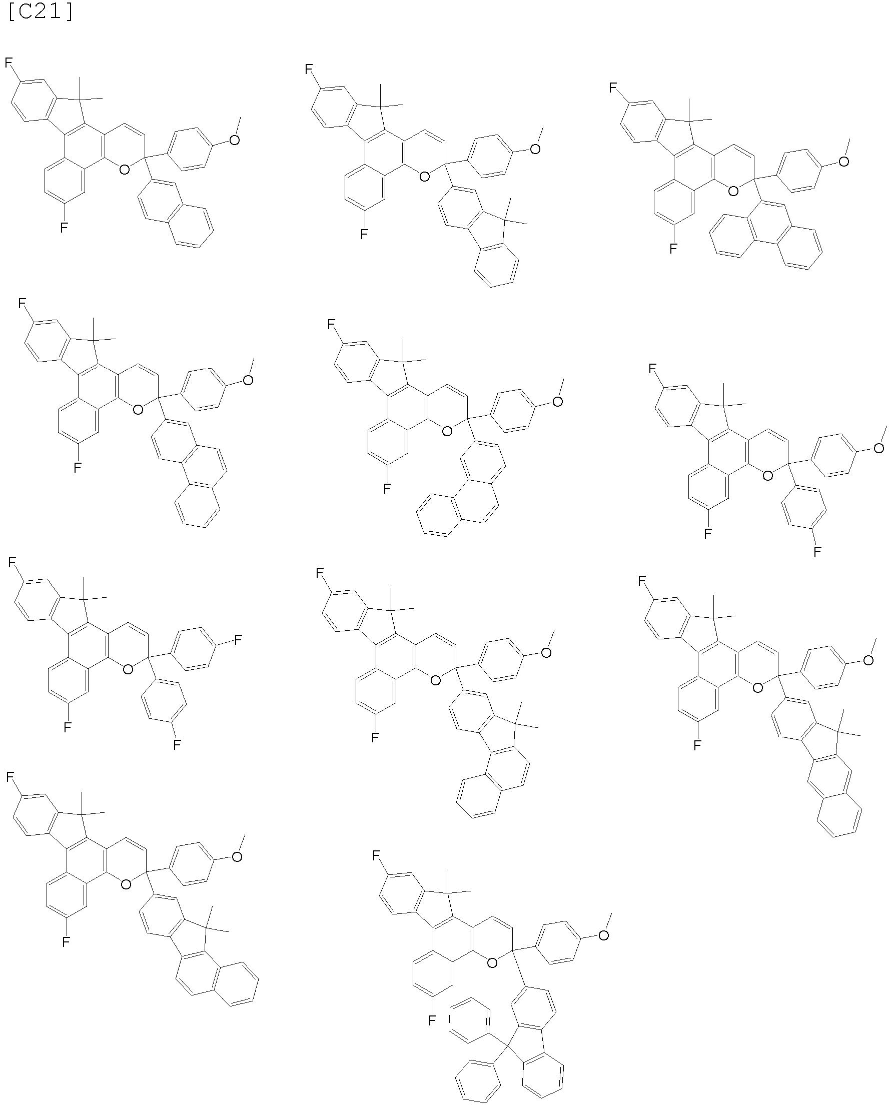

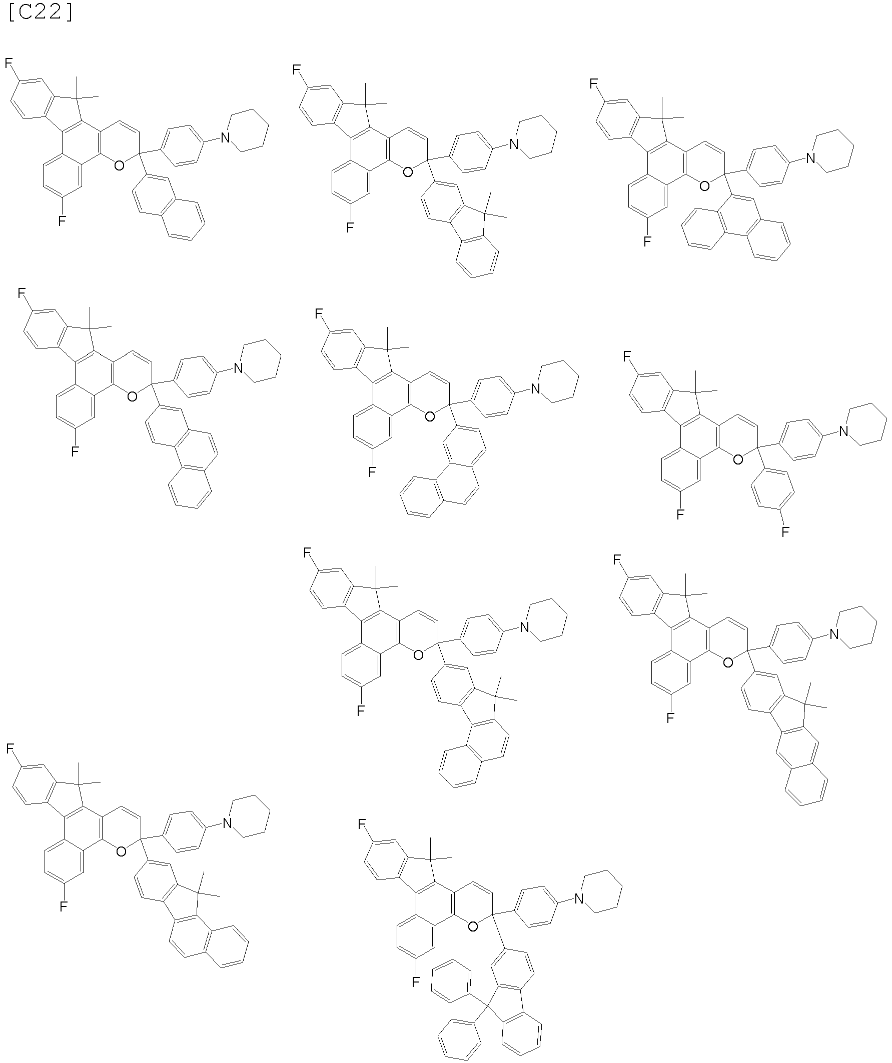

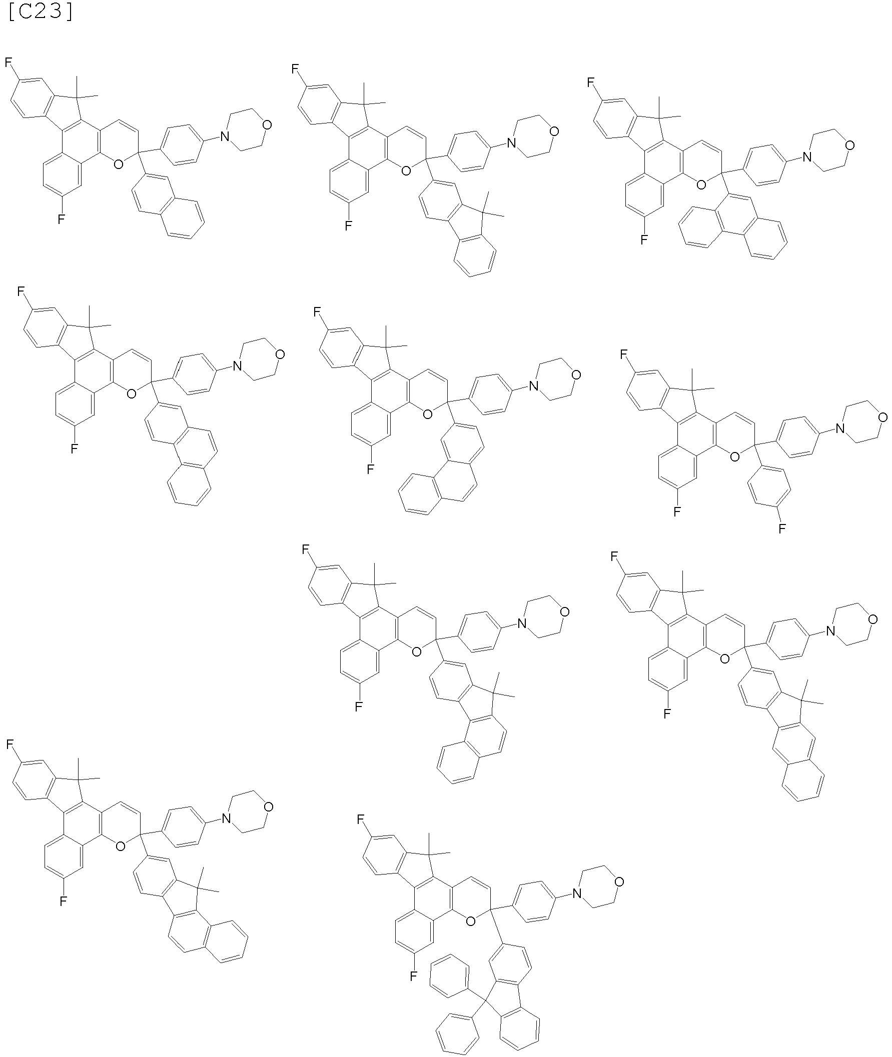

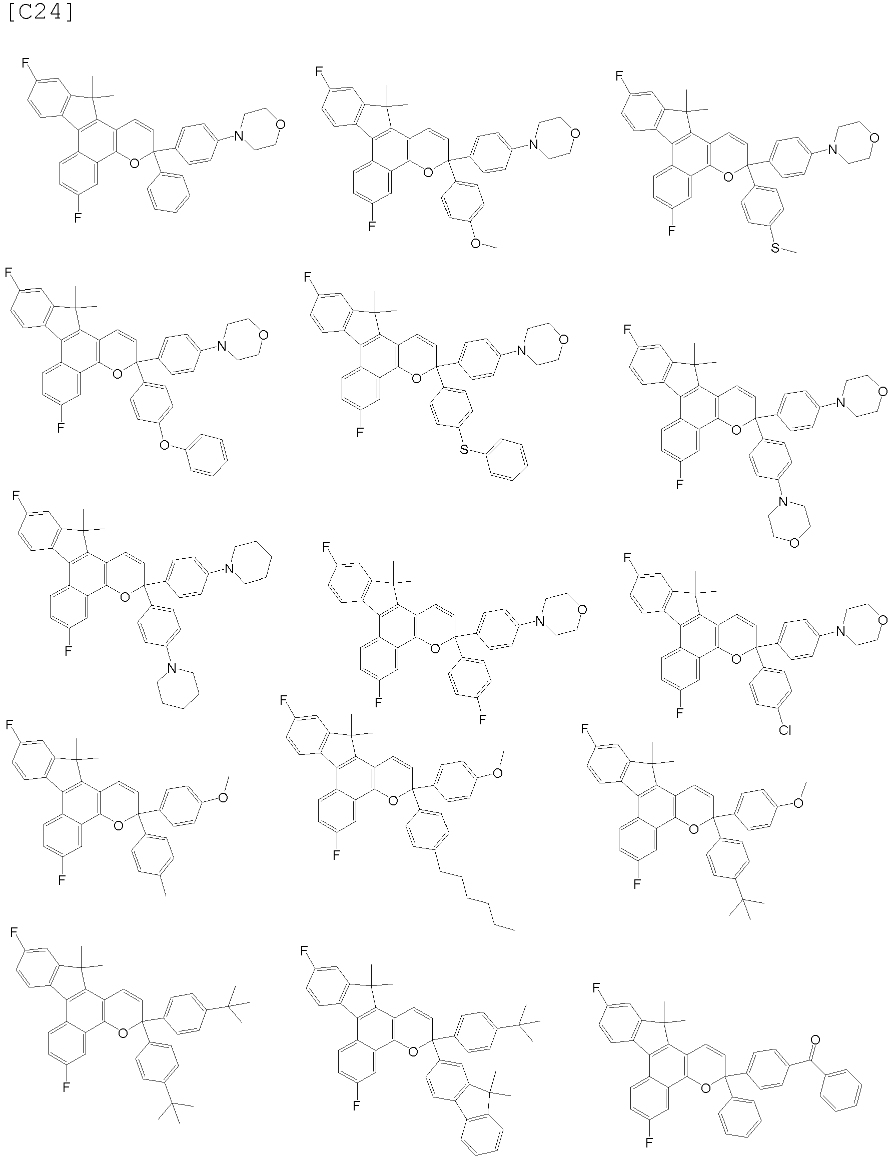

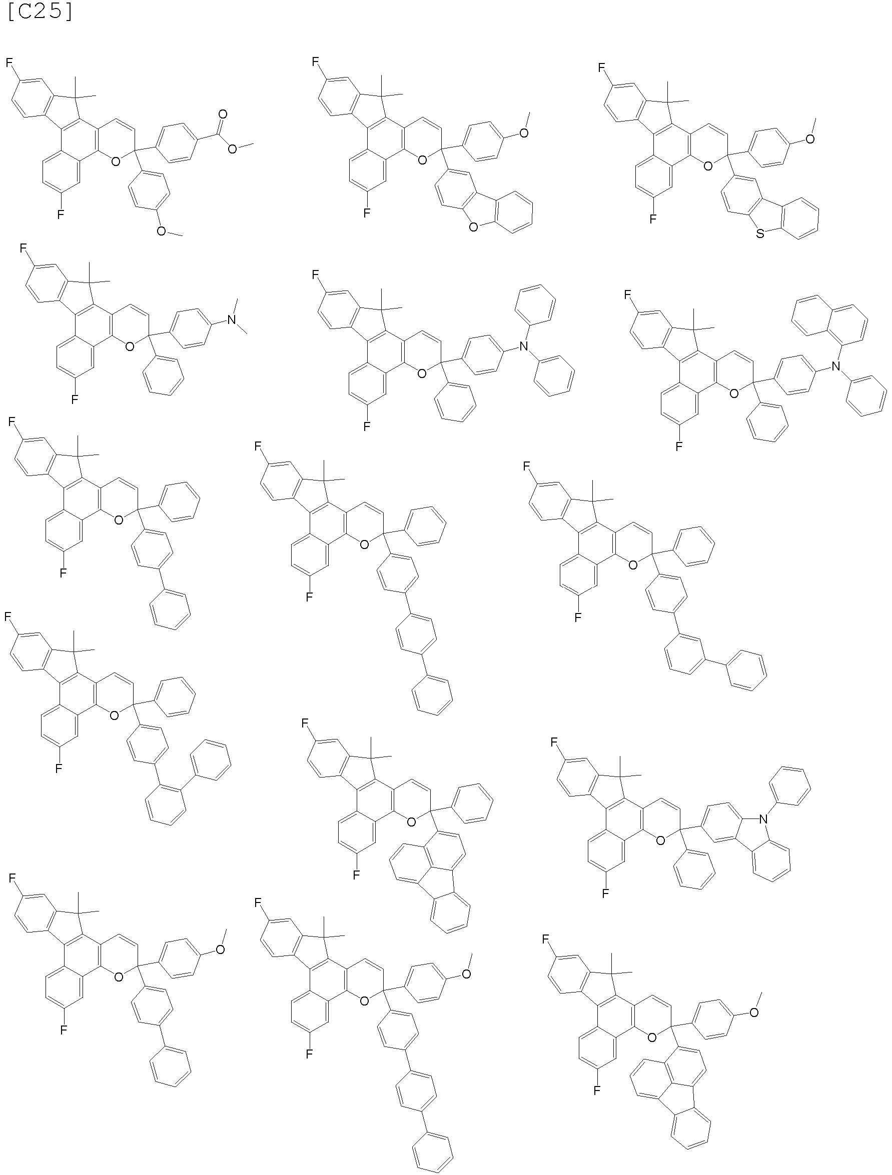

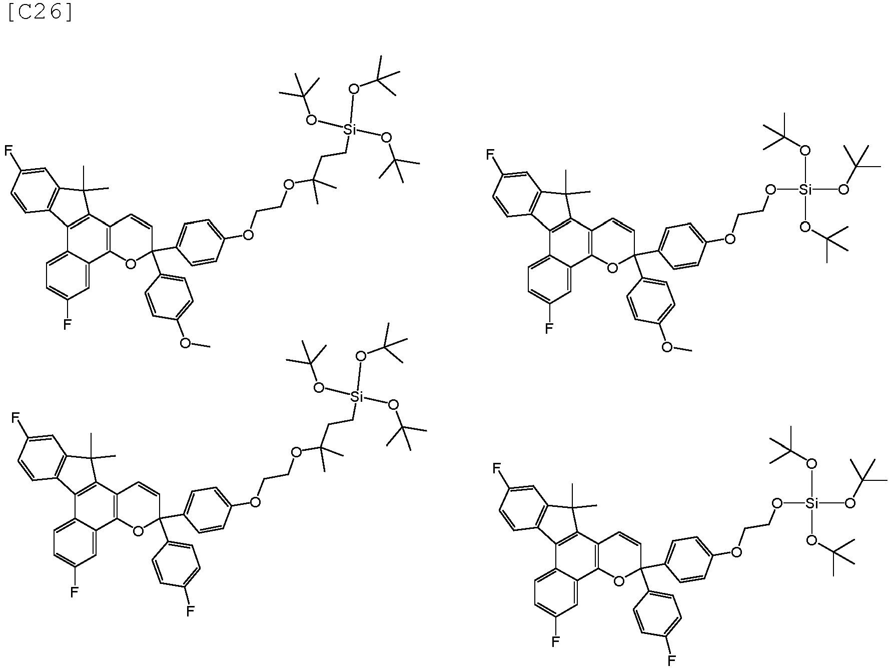

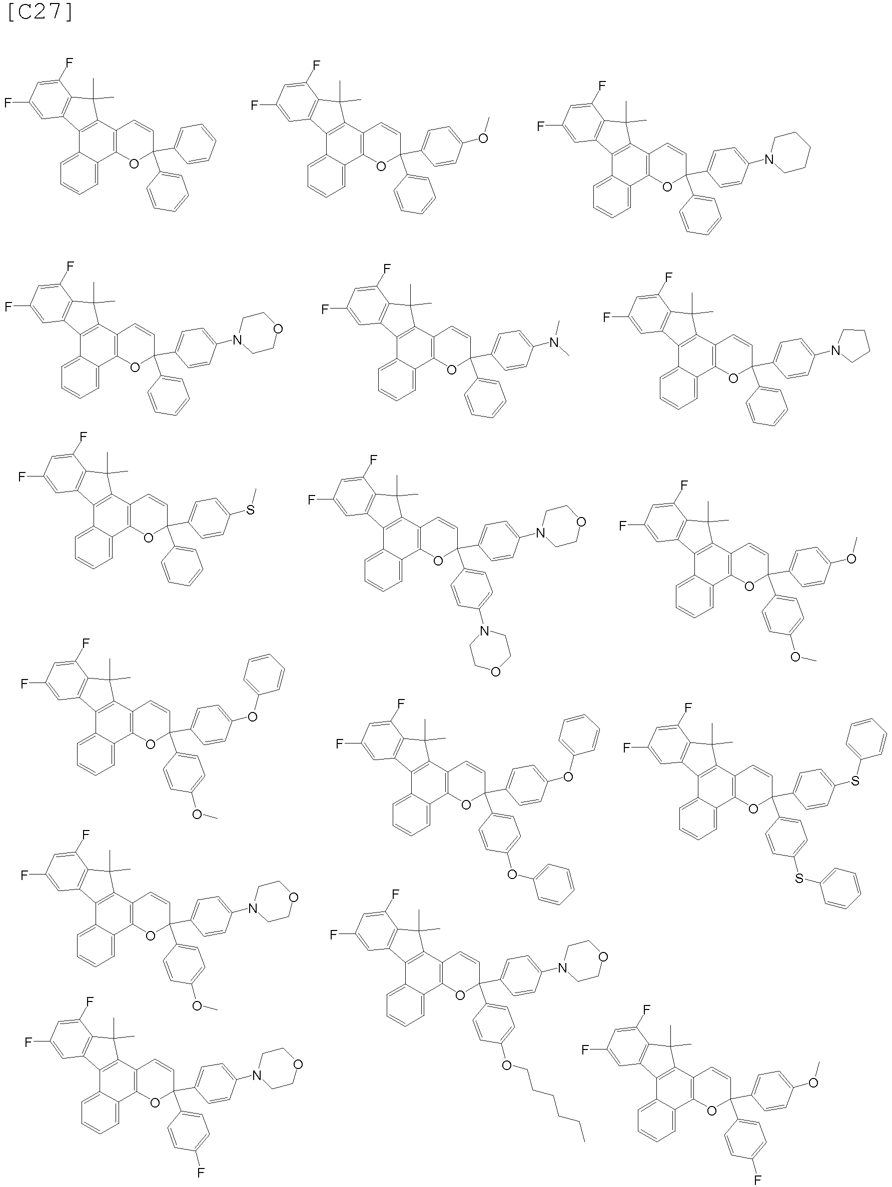

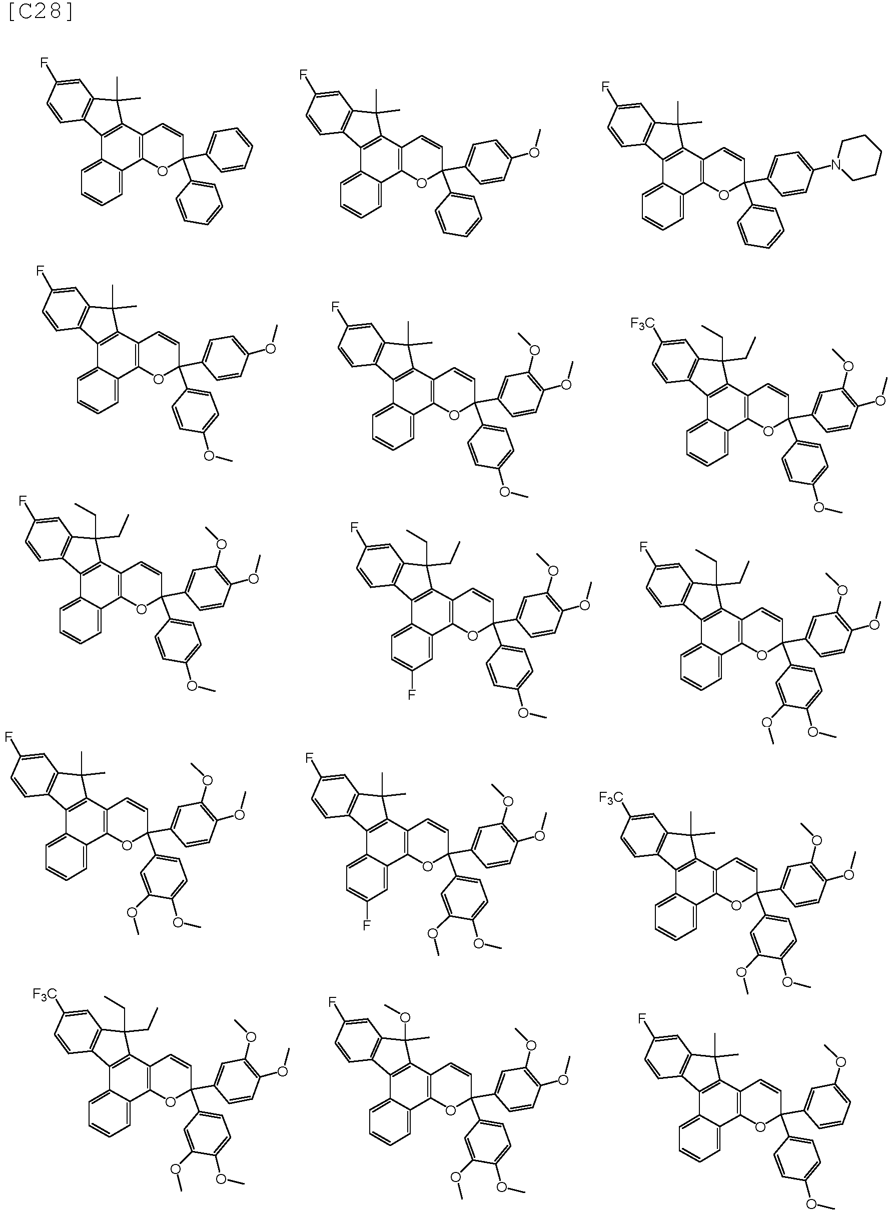

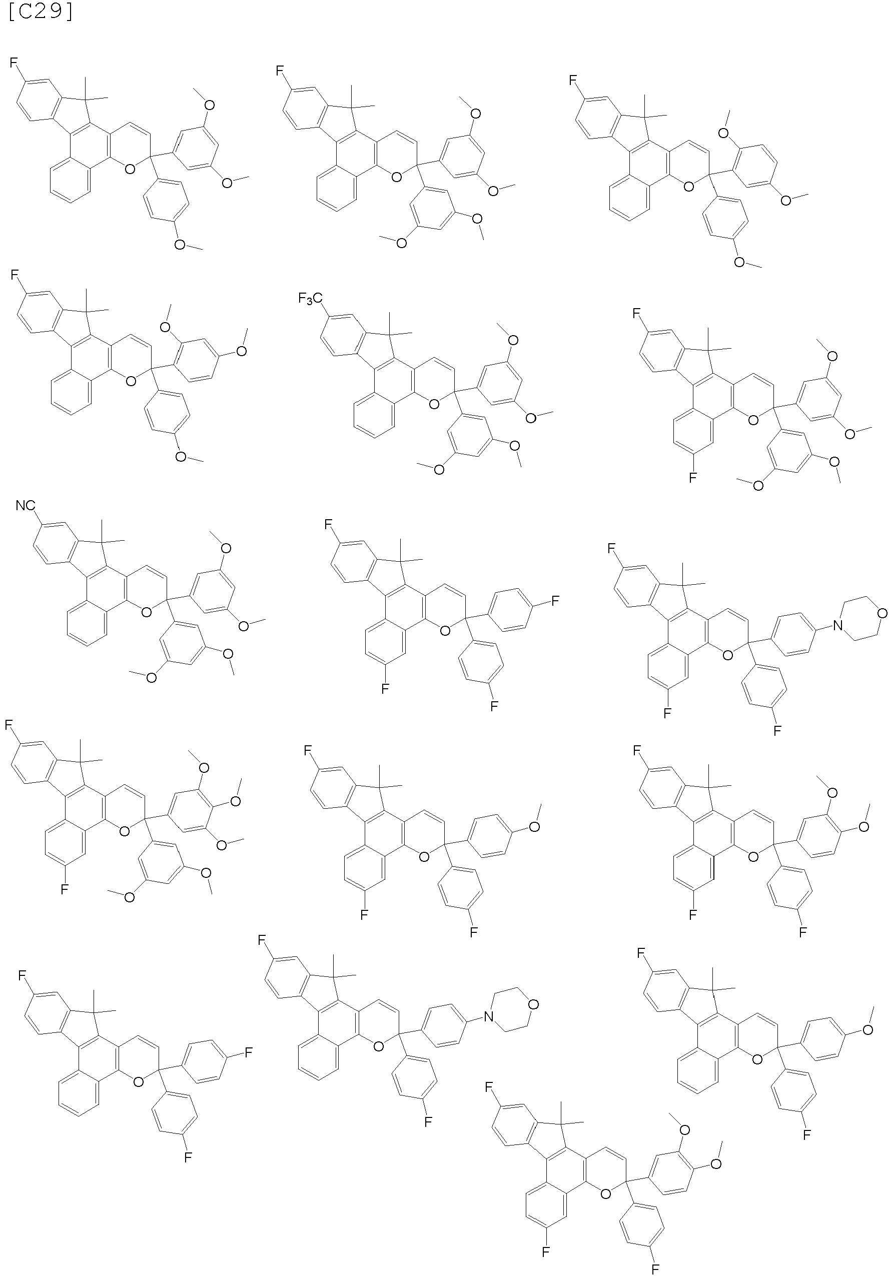

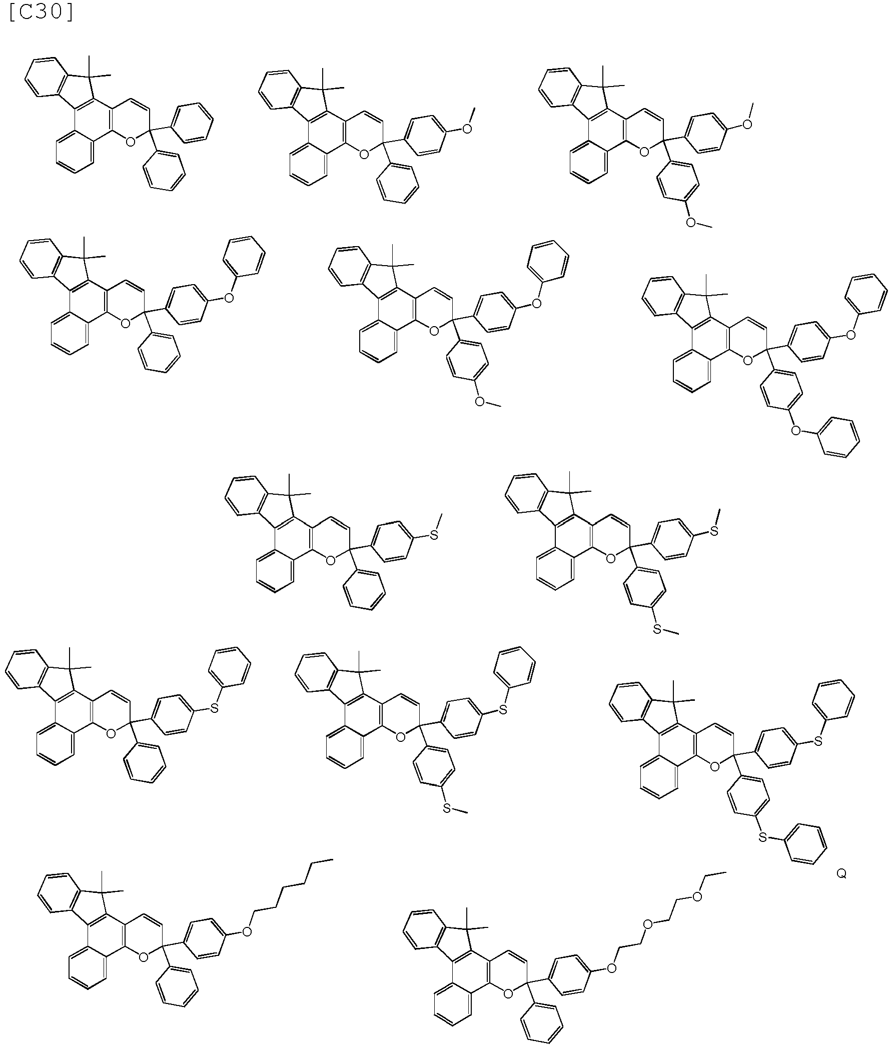

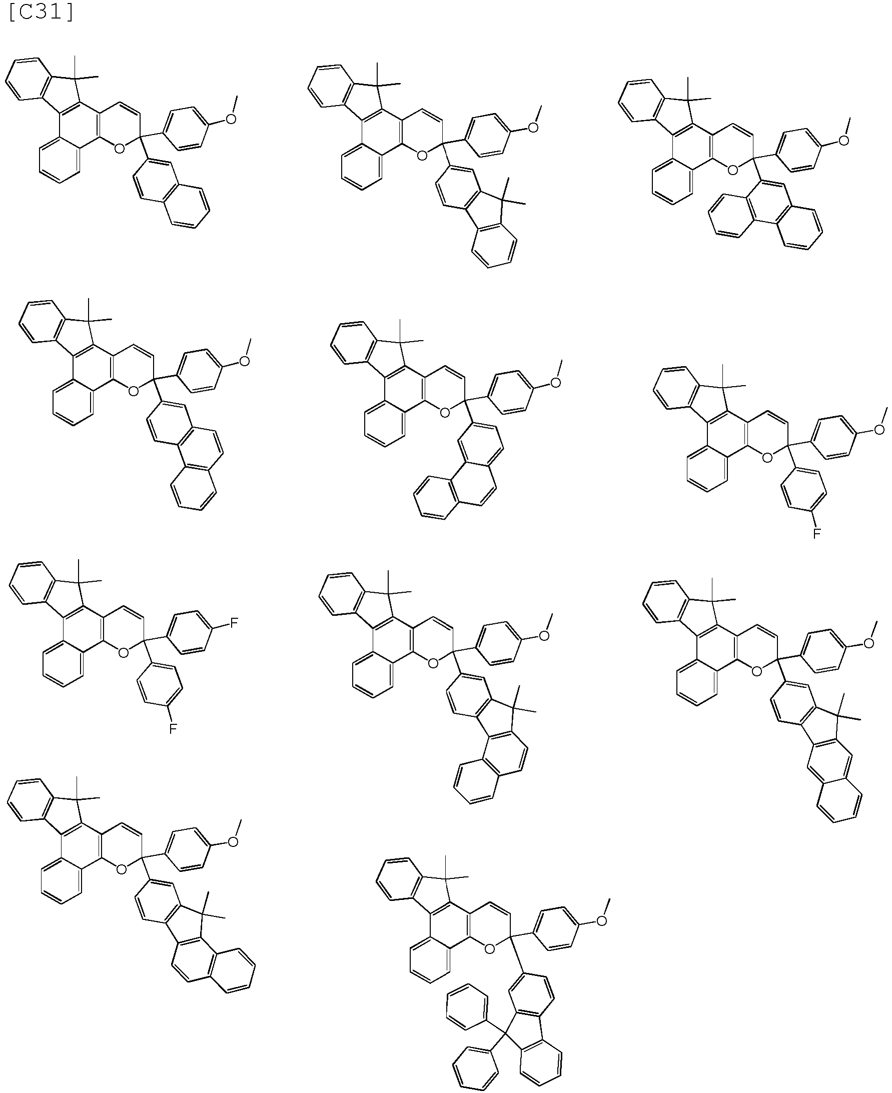

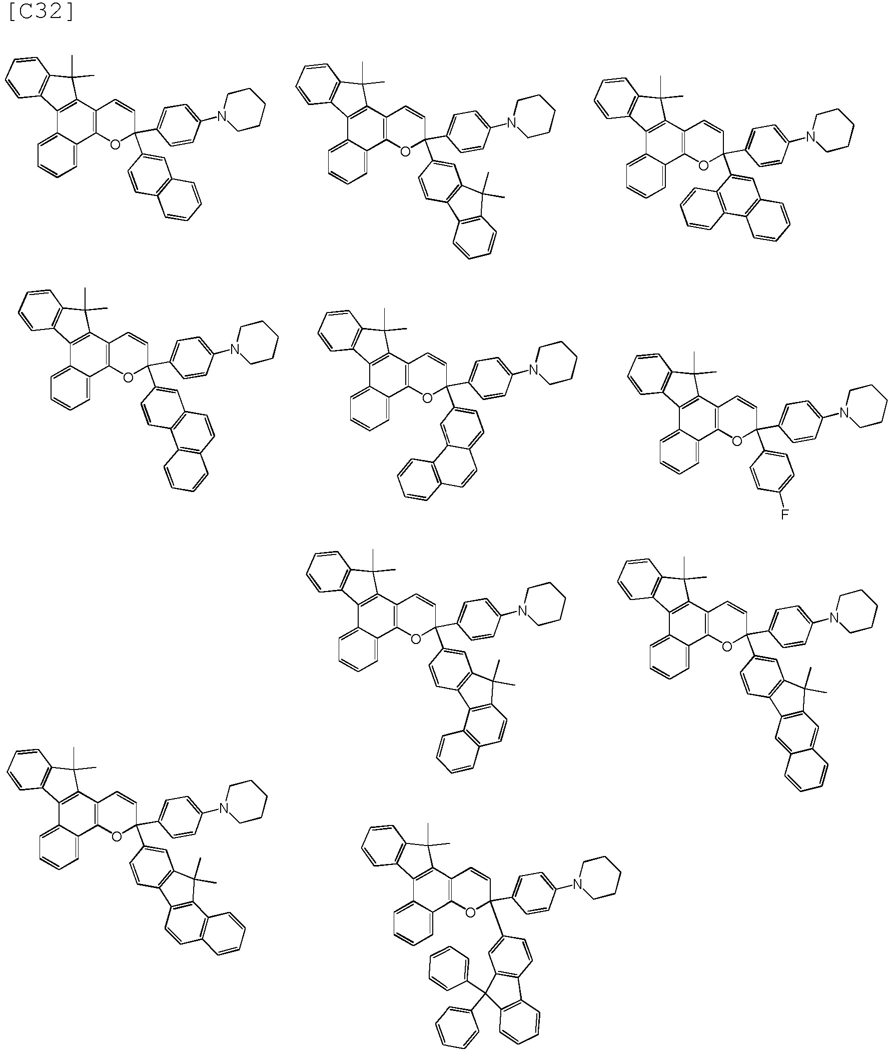

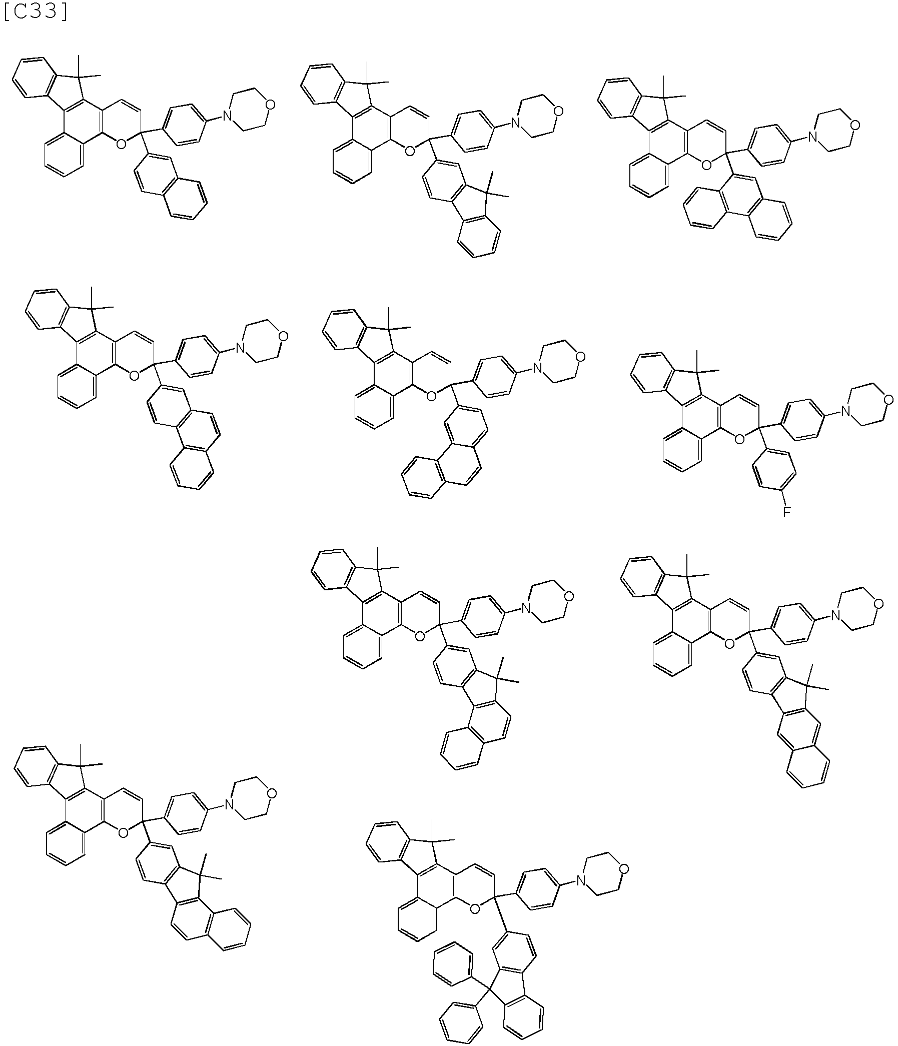

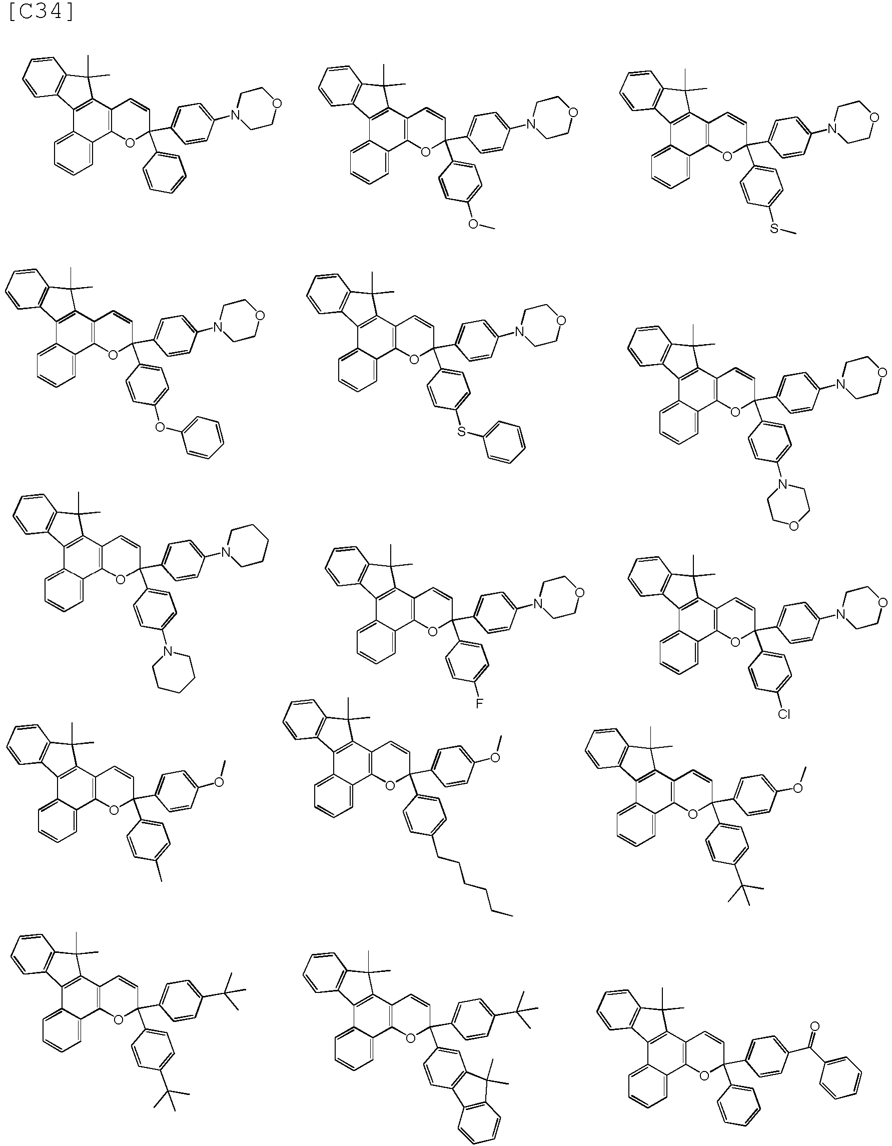

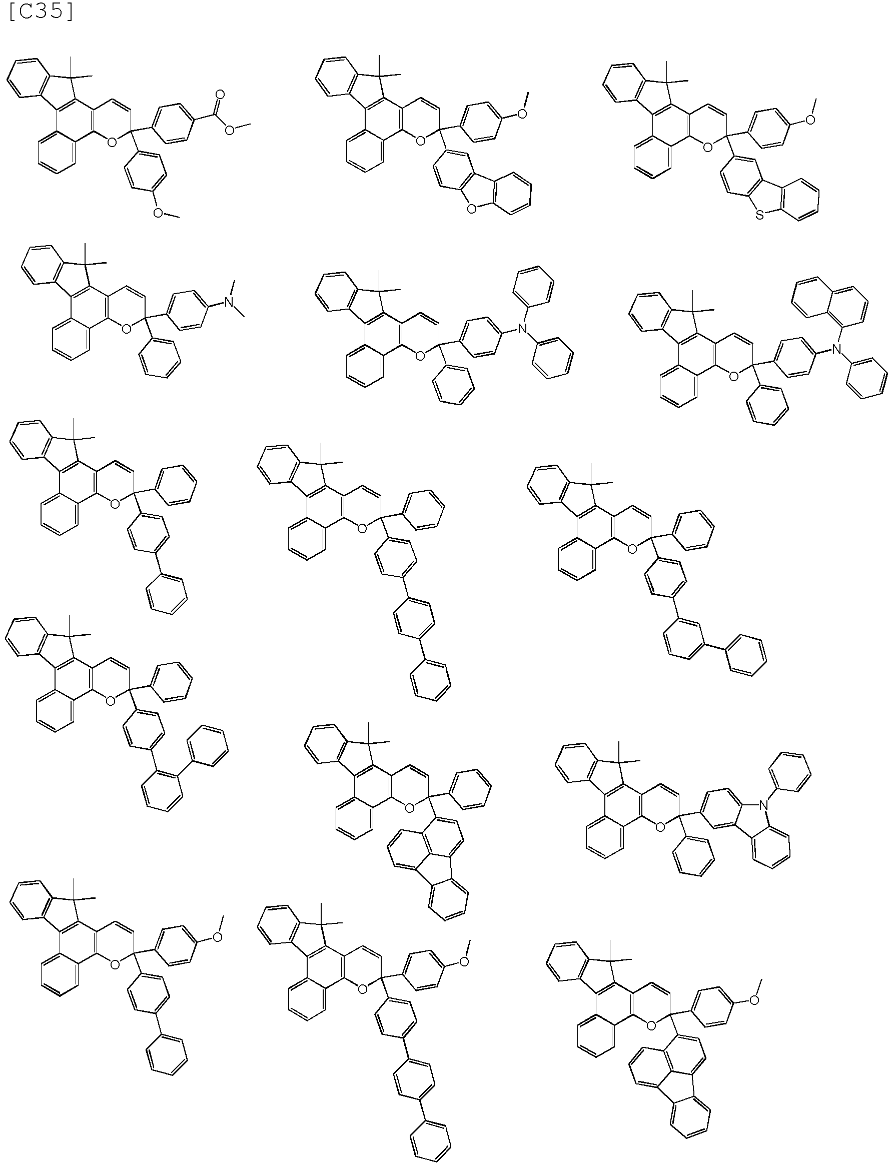

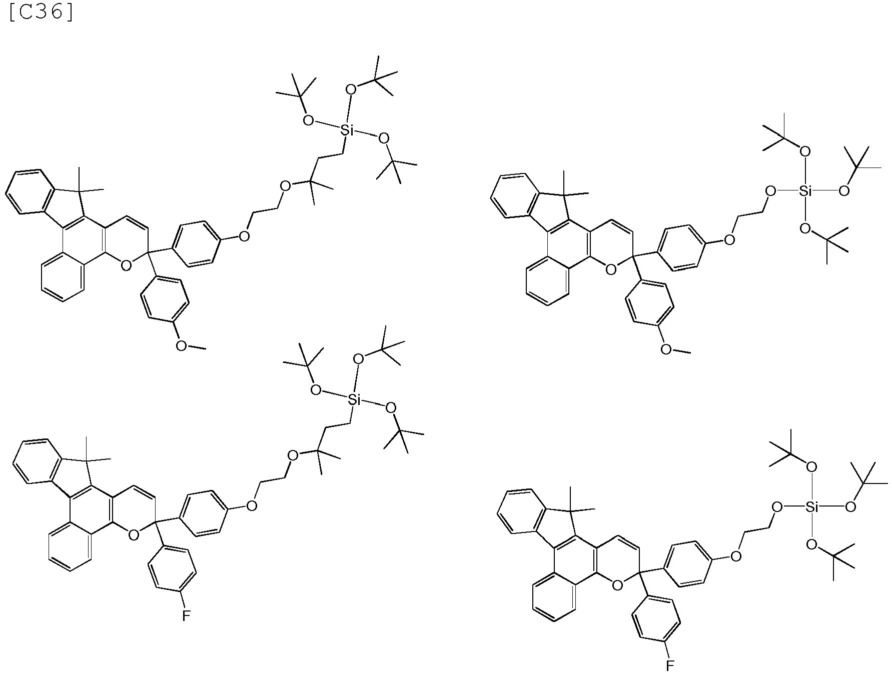

### <Compound Represented by General Formula B>

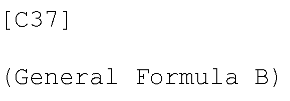

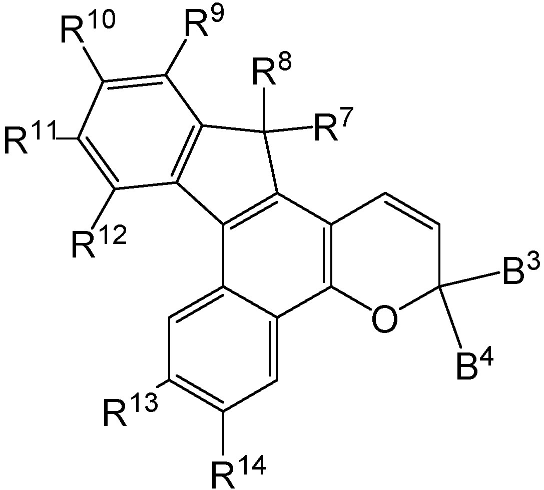

In General Formula B, R⁷ to R¹², B³, and B⁴ each independently represent a hydrogen atom or a substituent.

Preferably, R⁷ and R⁸ each independently represent a substituted or unsubstituted alkyl group having 1 to 20 carbon atoms, and more preferably, R⁷ and R⁸ each independently represent a methyl group, an ethyl group, a propyl group, a butyl group, a pentyl group, or a hexyl group. Further preferably, R⁷ and R⁸ each independently represent a methyl group or an ethyl group, and still further preferably, both R⁷ and R⁸ represent a methyl group or both R¹ and R² represent an ethyl group.

Preferably, B³ and B⁴ each independently represent a substituted or unsubstituted phenyl group. When the phenyl group has multiple substituents, two or more of these substituents may be bonded to form a ring. Specific examples of rings to be formed include rings included in exemplary compounds to be described later. The substitution position of a substituent in a substituted phenyl group is preferably a position that is the para-position with respect to the carbon atom to which B³ and B⁴ are bonded. Specific examples of substituents of the substituted phenyl group include substituents, which are included in exemplary compounds to be described later, such as a morpholino group, a piperidino group, a halogen atom, an alkoxy group, and the following substituents.

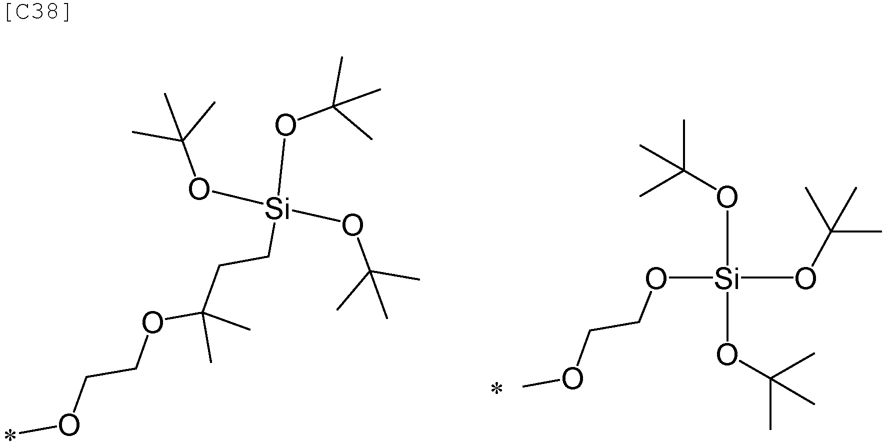

R⁹ to R¹² each independently represent a hydrogen atom or a substituent. In one aspect, R⁹ to R¹² can all be hydrogen atoms. In another aspect, R¹⁰ can be an electron-withdrawing group, and R⁹, R¹¹, and R¹² can all be hydrogen atoms. In another aspect, R⁹ and R¹¹ can each independently be an electron-withdrawing group, and R10 and R12 can be hydrogen atoms. As the electron-withdrawing group, a halogen atom, a perfluoroalkyl group having 1 to 6 carbon atoms, a perfluorophenyl group, a perfluoroalkylphenyl group, or a cyano group is preferable. As the halogen atom, a fluorine atom is preferable. As the perfluoroalkyl group having 1 to 6 carbon atoms, a trifluoromethyl group is preferable.

In one aspect, R¹⁰ can be a substituted or unsubstituted phenyl group, and preferably, R¹⁰ is a substituted or unsubstituted phenyl group and R⁹, R¹¹, and R¹² are hydrogen atoms. Specific examples of such substituted phenyl groups include a phenyl group that has been substituted with one or more halogen atoms and/or one or more cyano groups, for example, a phenyl group in which all five substitution positions of the phenyl group have been substituted with halogen atoms (preferably fluorine atoms), and a monosubstituted phenyl group in which a position that is the para-position with respect to the carbon atom to which R¹⁰ is bonded has been substituted with a cyano group.

R¹³ and R¹⁴ each independently represent an electron-donating group. That is, R¹³ and R¹⁴ represent the same or different electron-donating groups. It is preferable that R¹³ and R¹⁴ each independently represent an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group. Among them, for R¹³ and R¹⁴, it is preferable that R¹³ be a morpholino group and R¹⁴ be an alkoxy group (preferably a methoxy group), that R¹³ be a morpholino group and R¹⁴ be a methylsulfide group (-S-CH₃), that both R¹³ and R¹⁴ be alkoxy groups (preferably methoxy groups), and that both R¹³ and R¹⁴ be methylsulfide groups.

Examples of the compounds represented by General Formula B include the following compounds. However, the present invention is not limited to the compounds exemplified below.

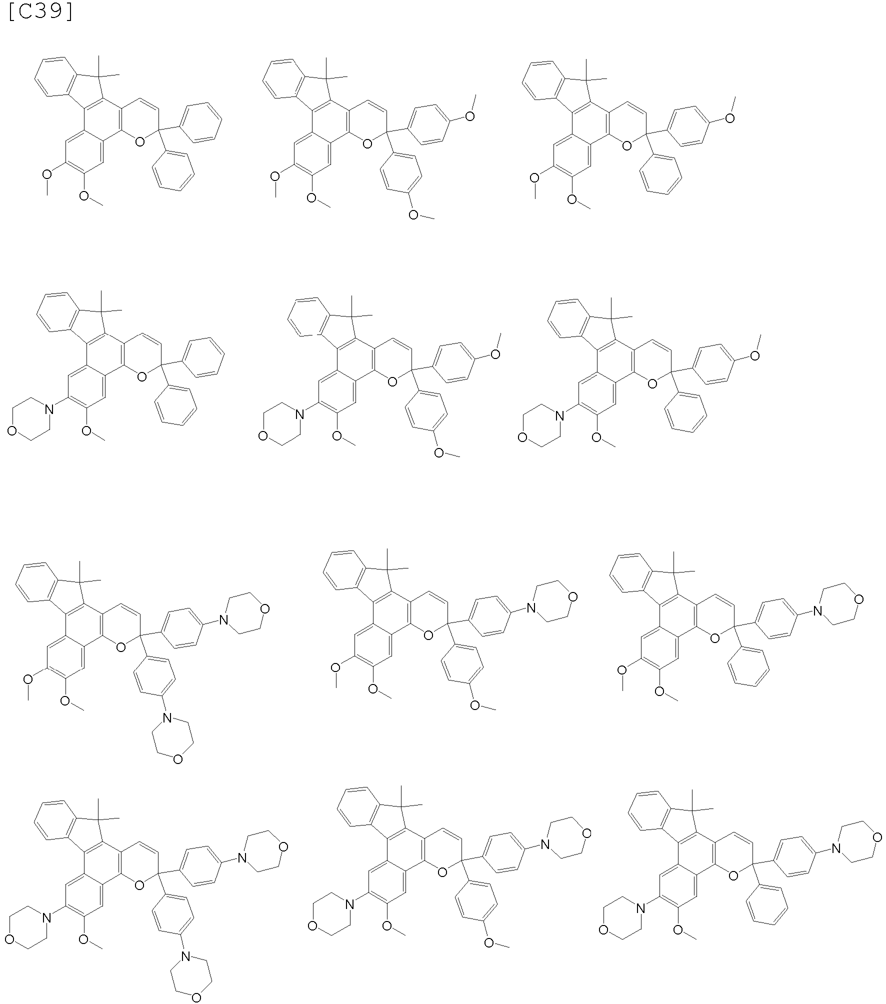

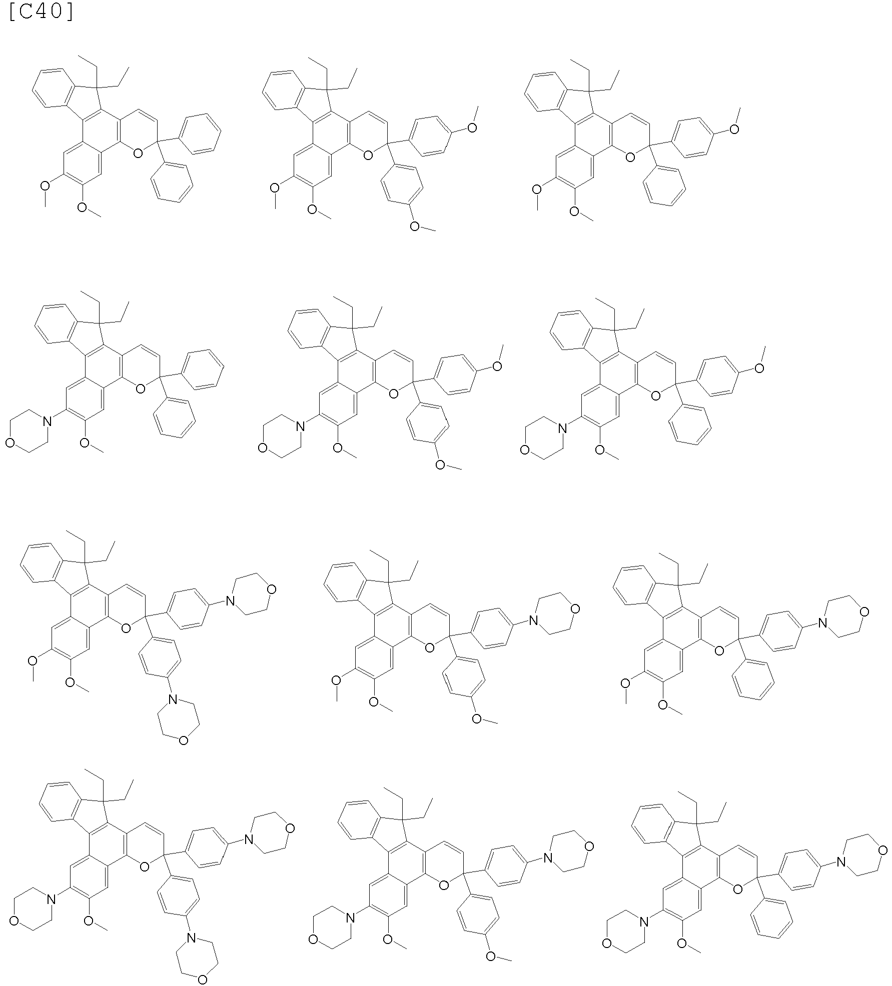

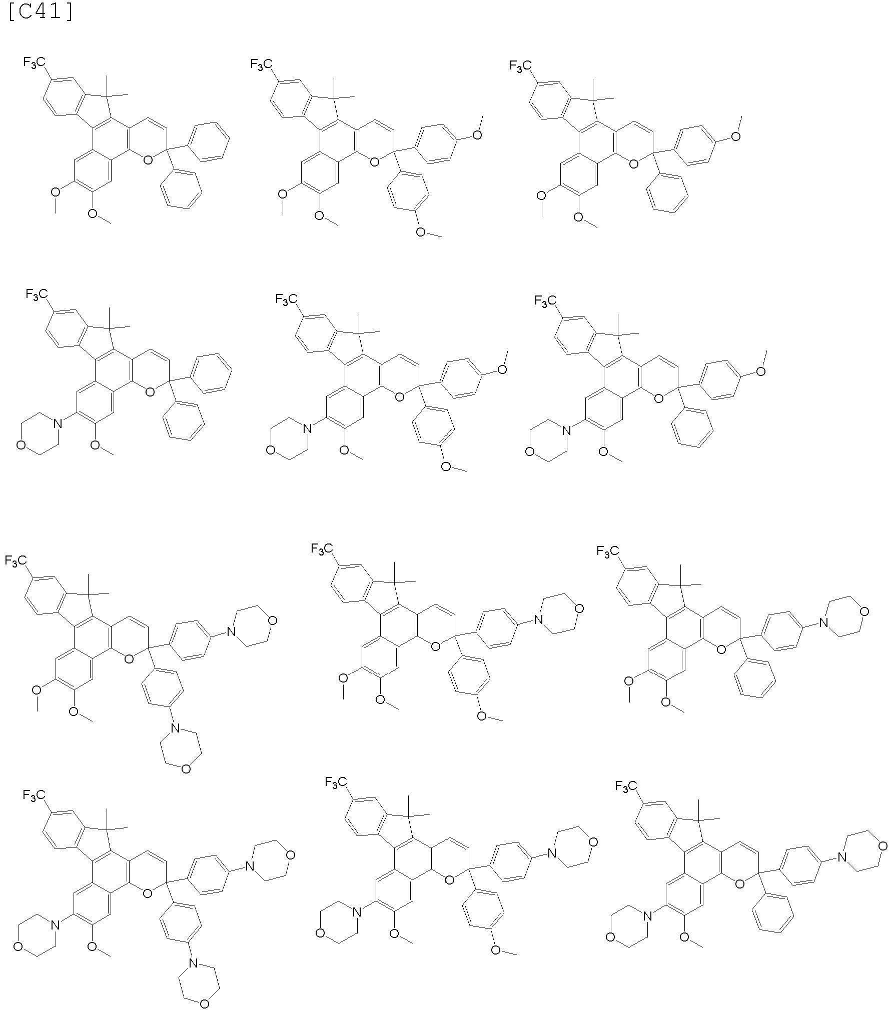

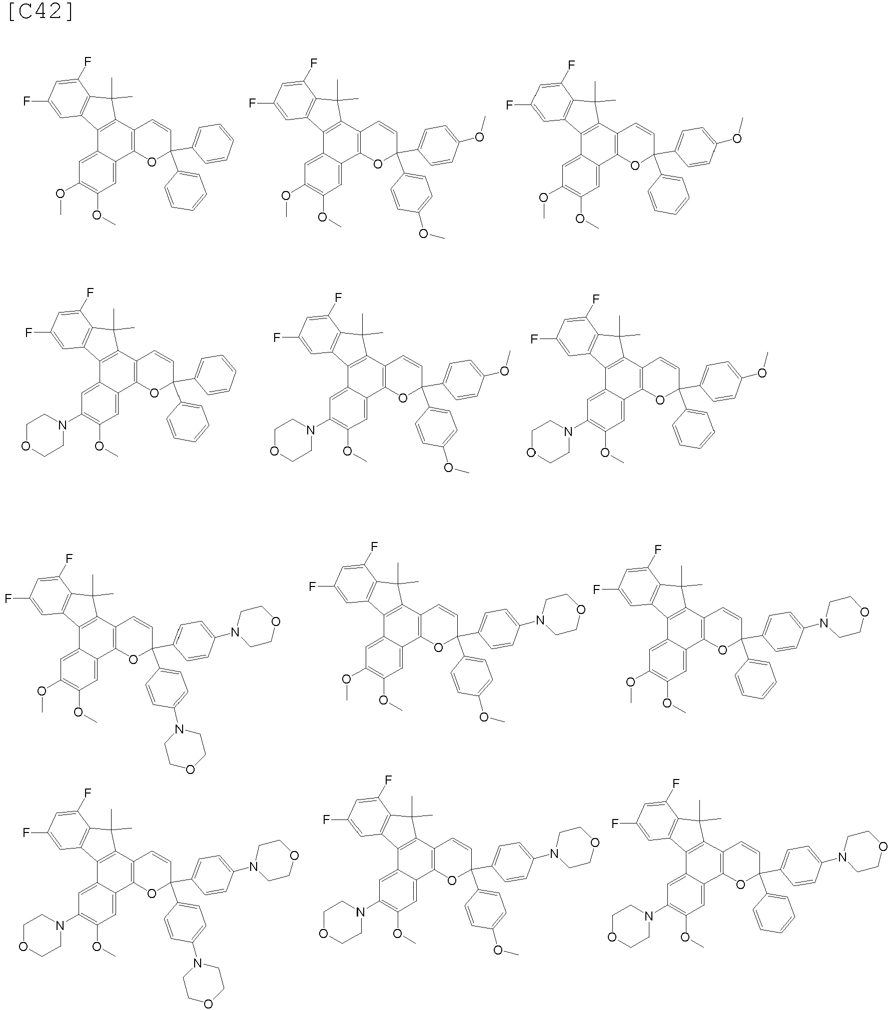

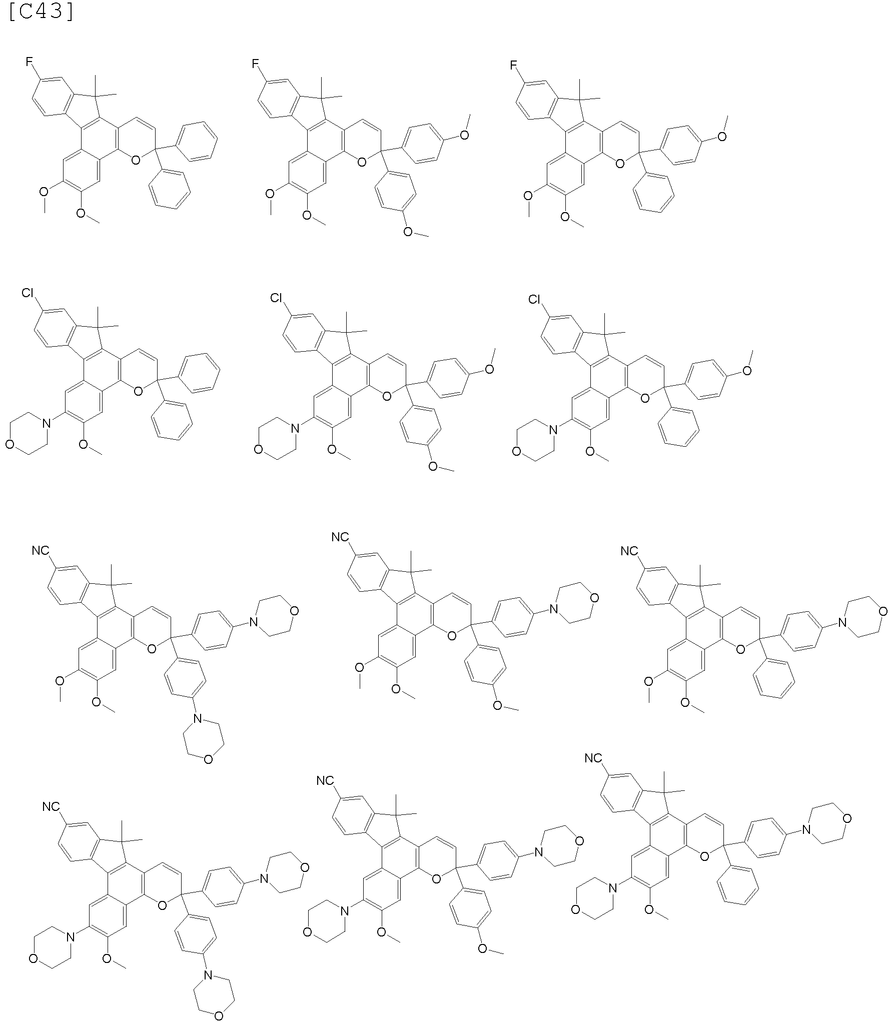

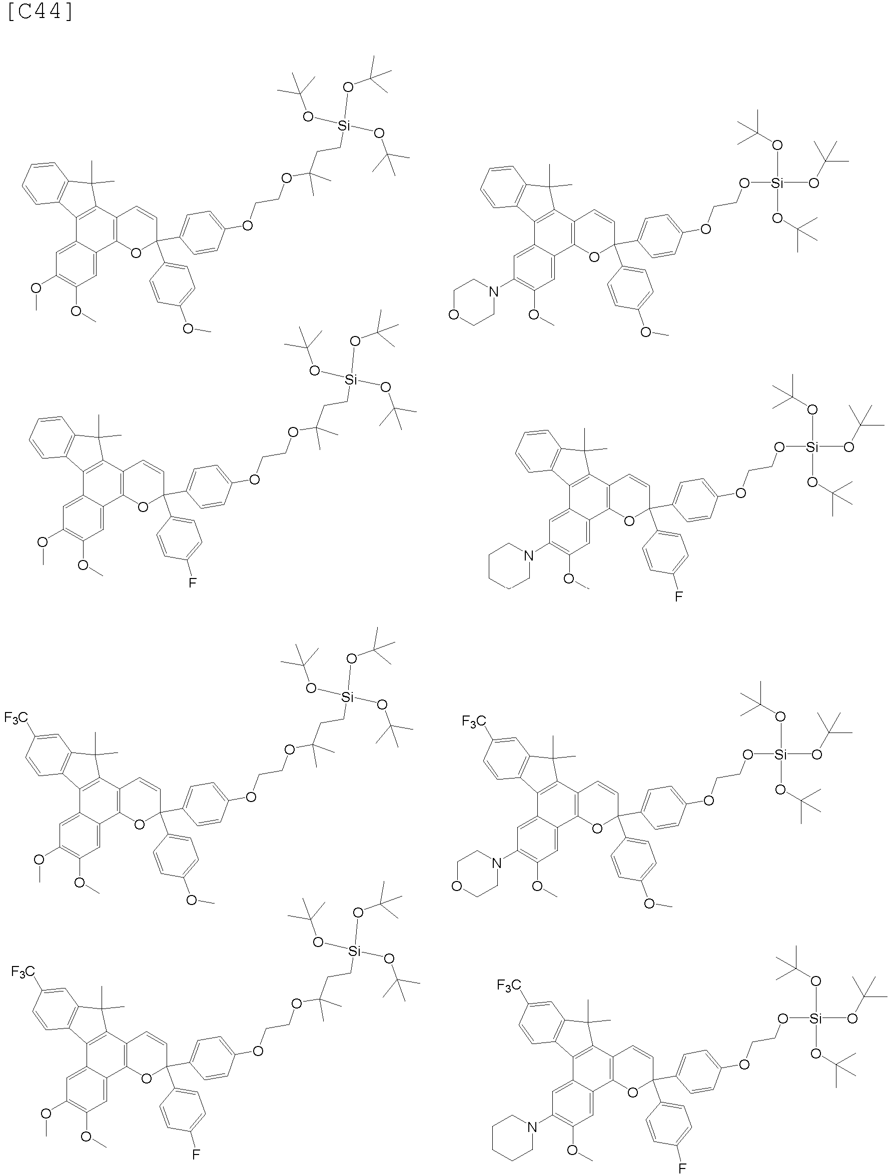

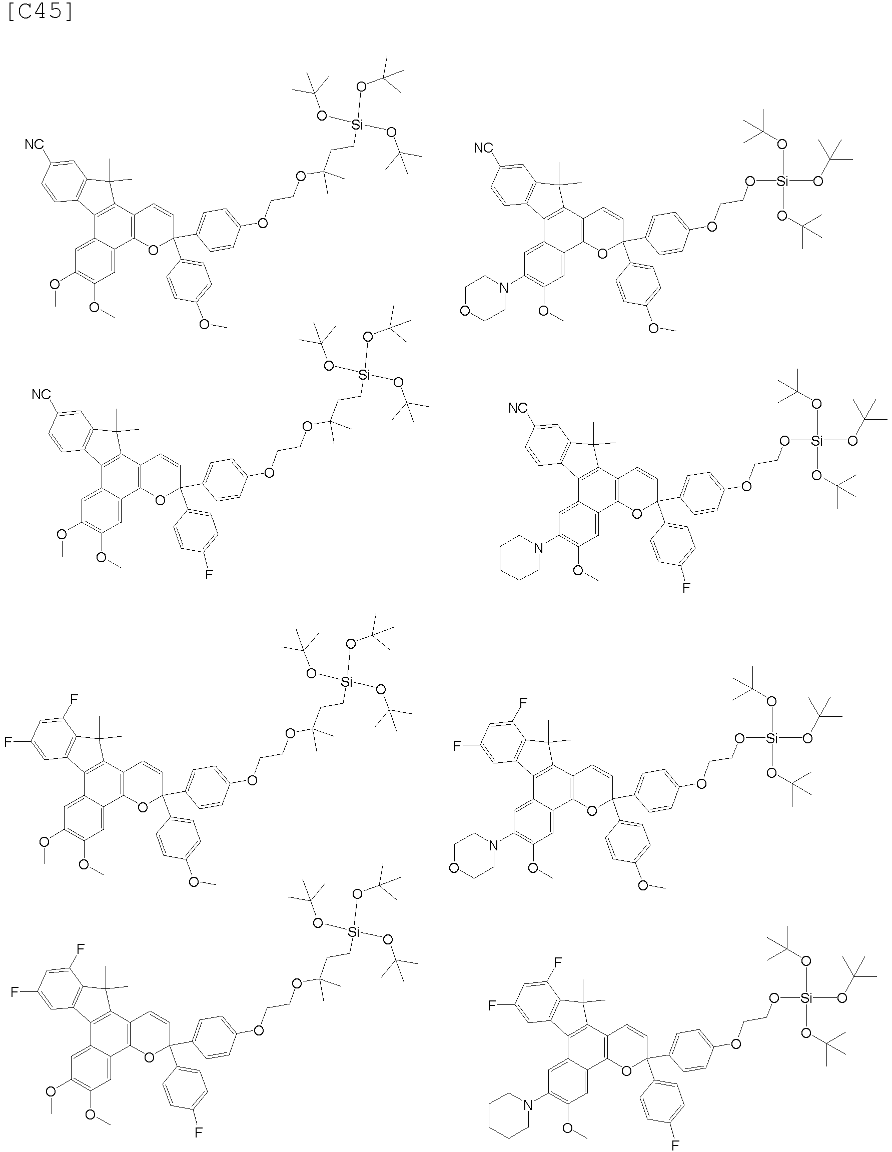

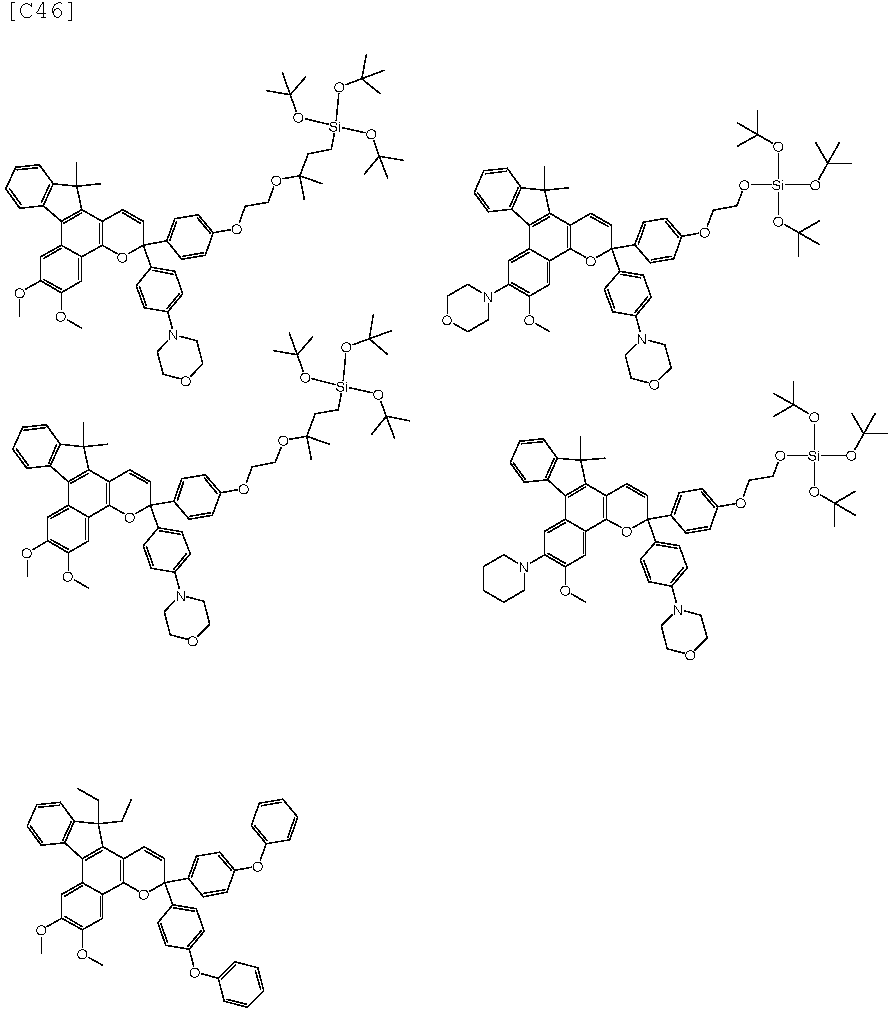

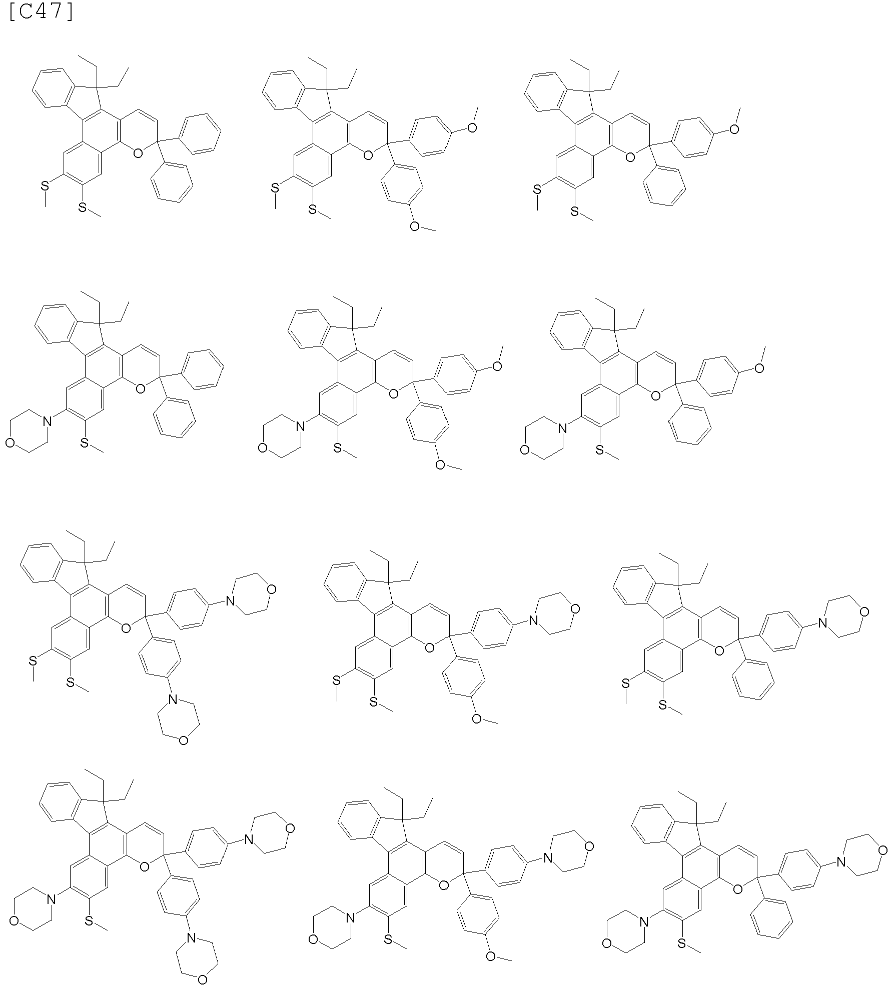

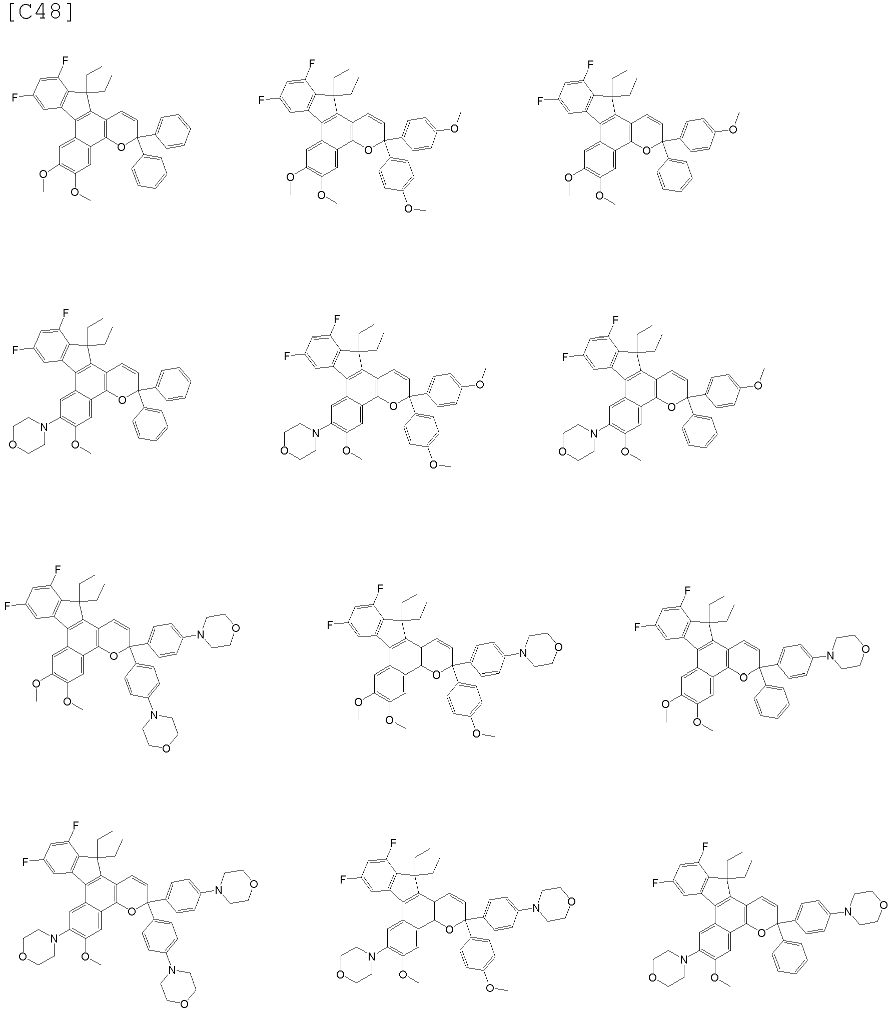

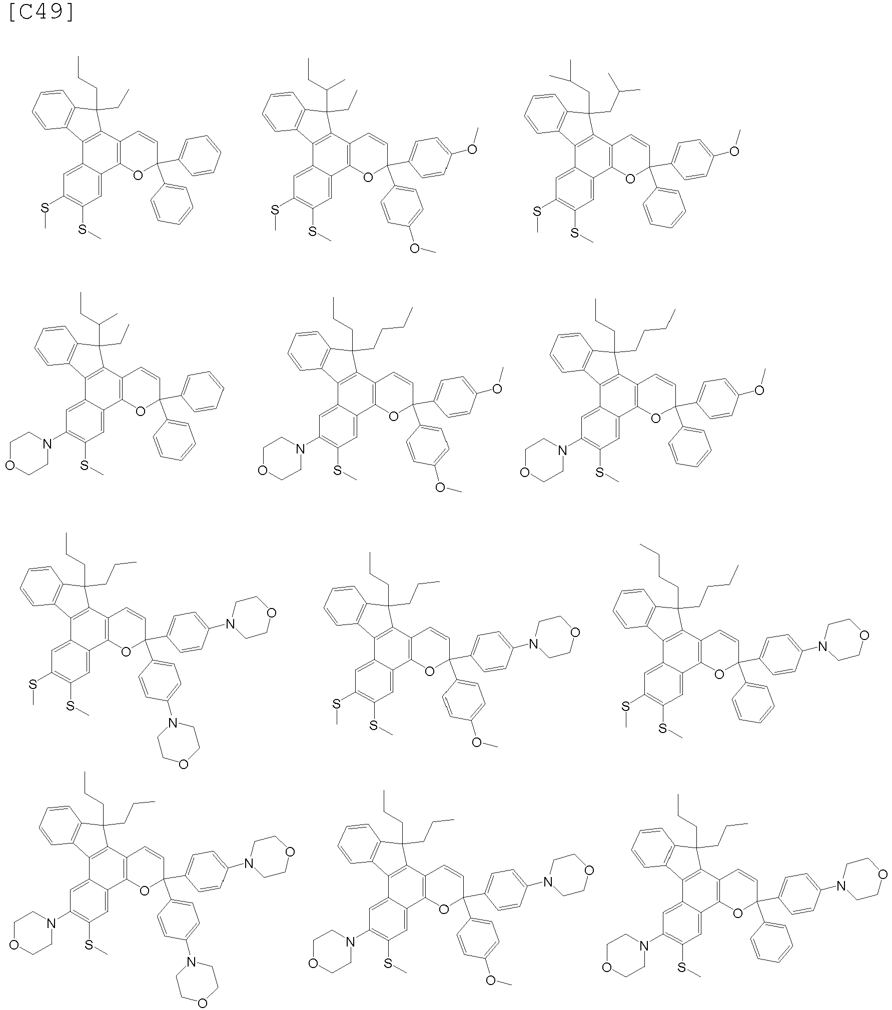

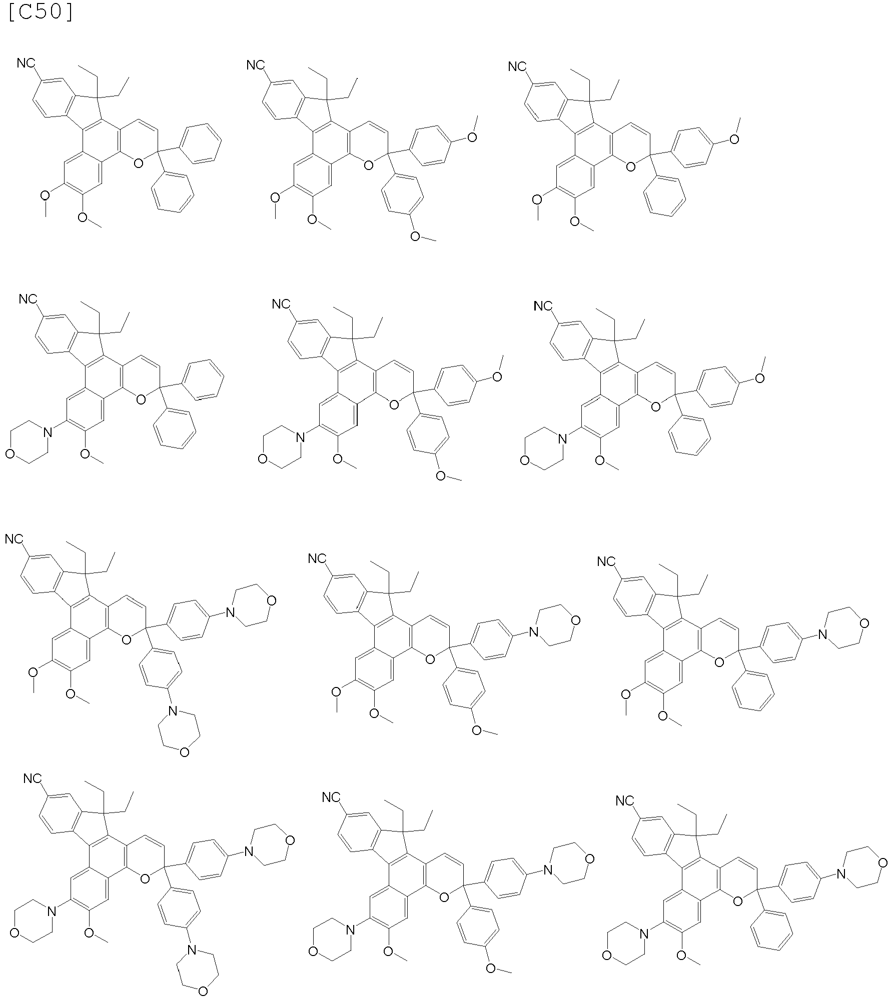

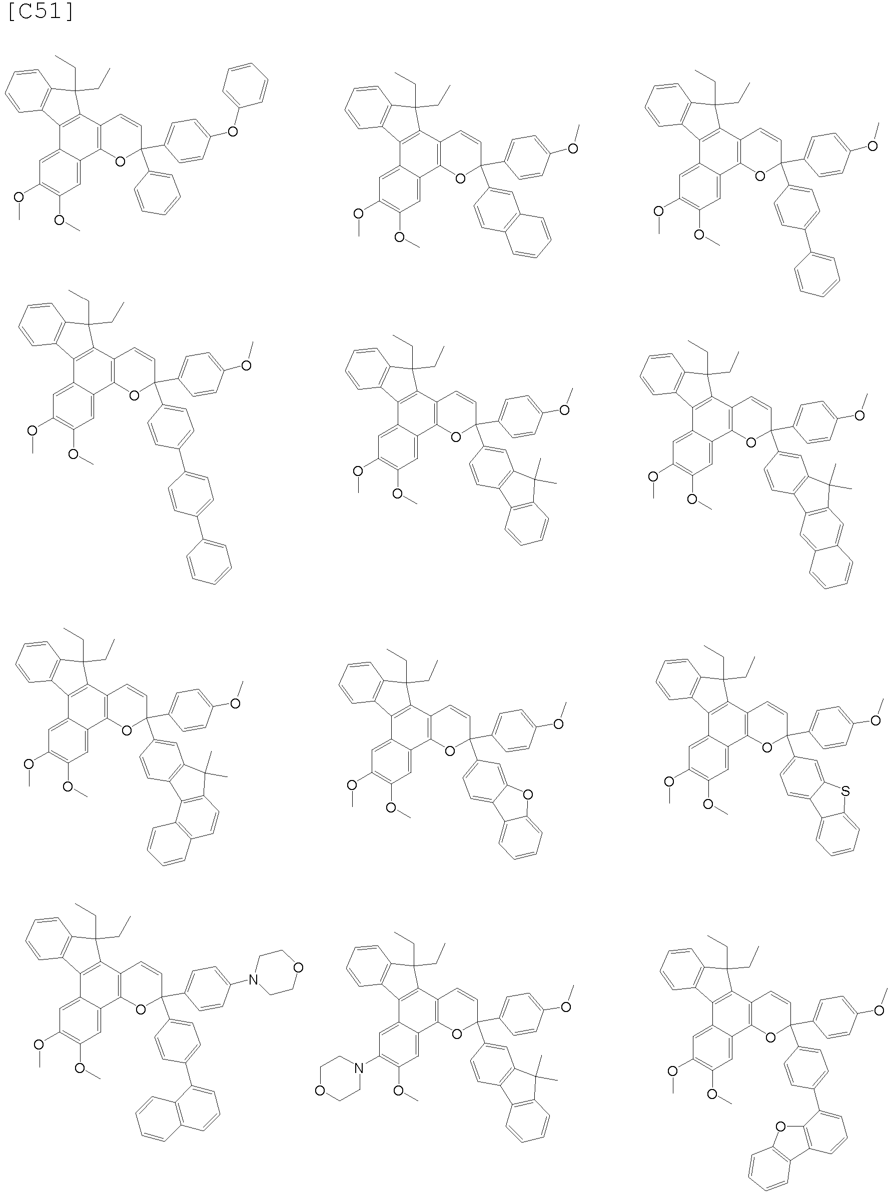

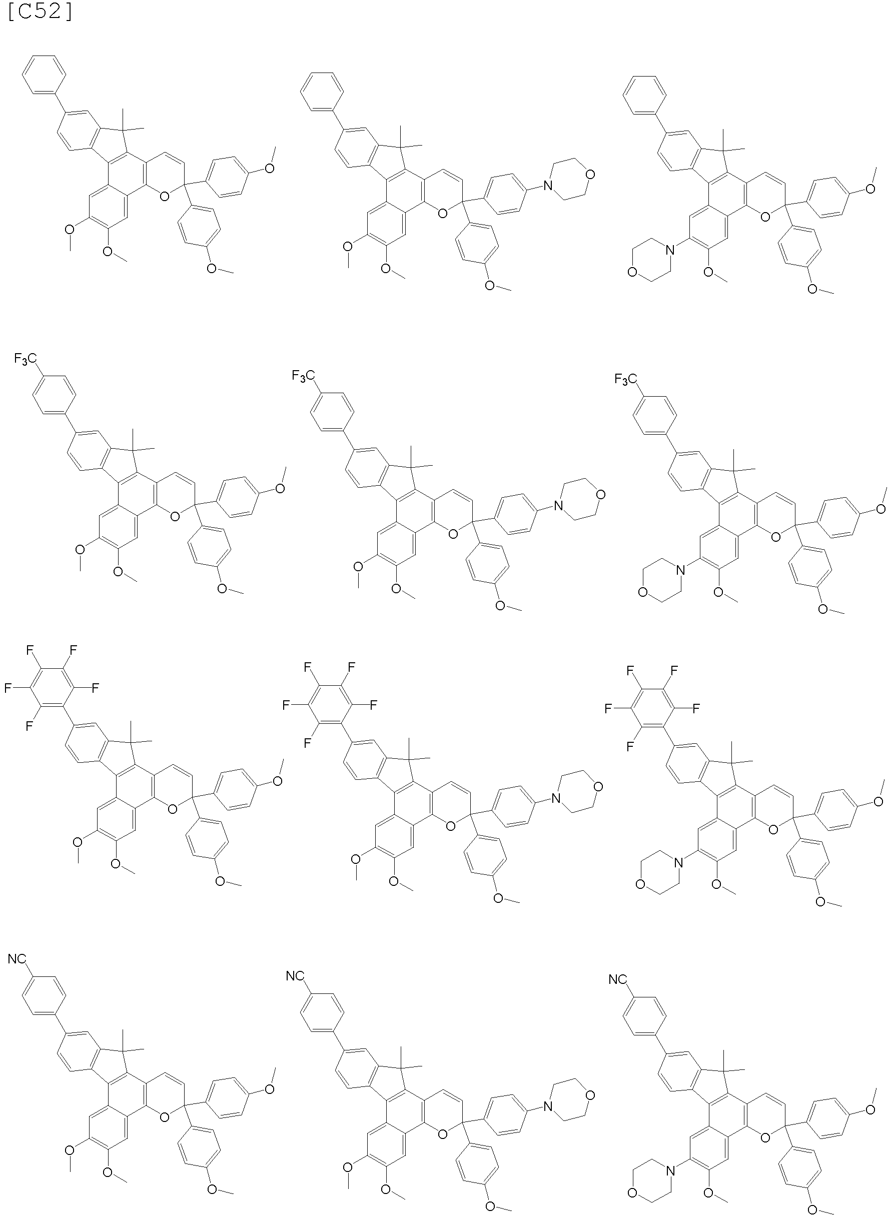

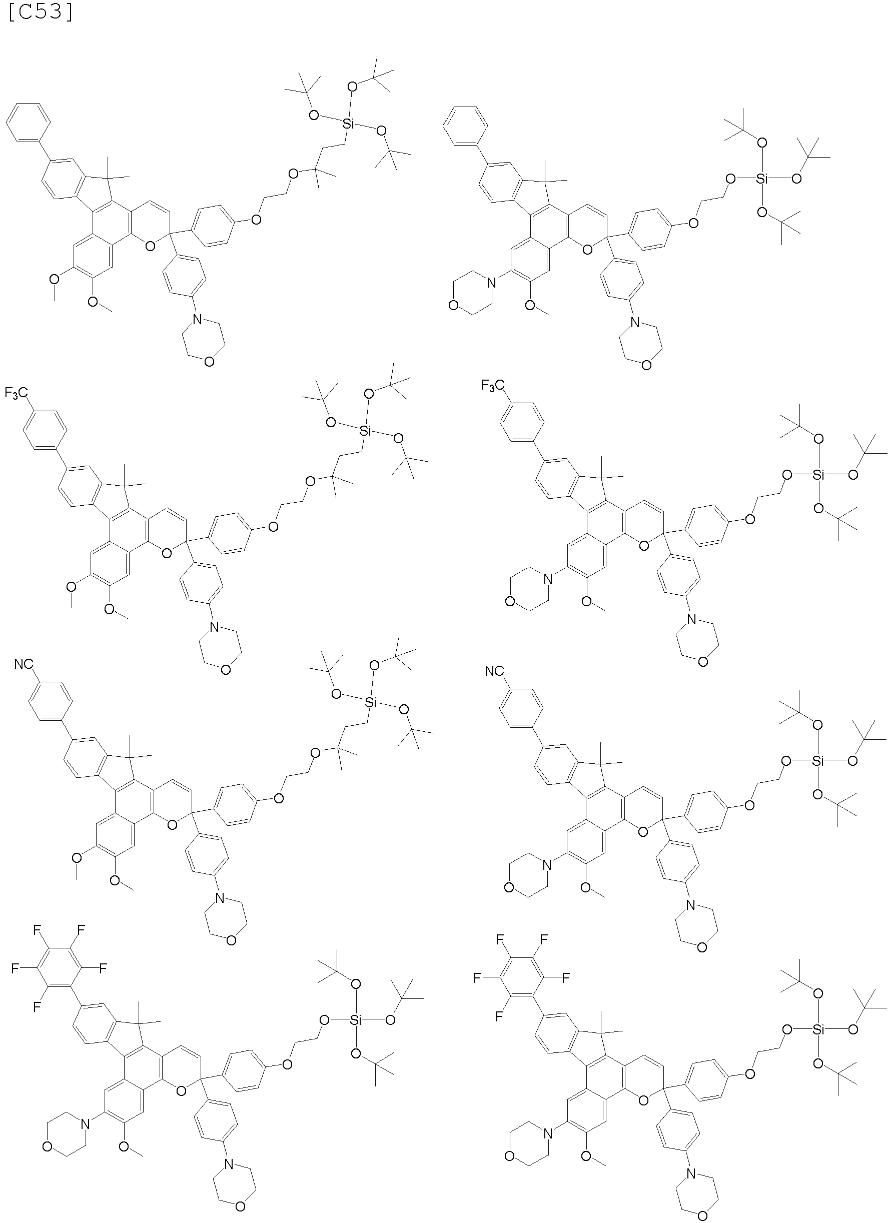

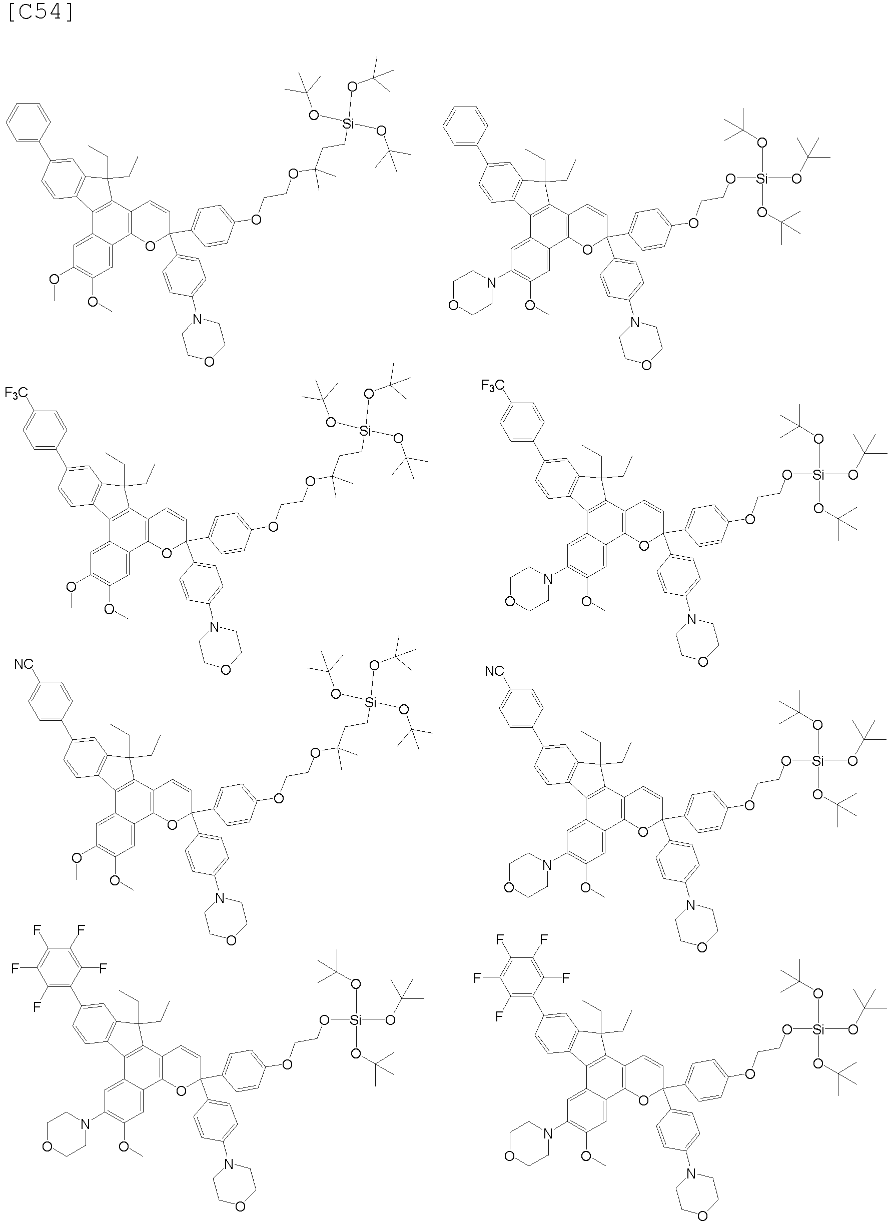

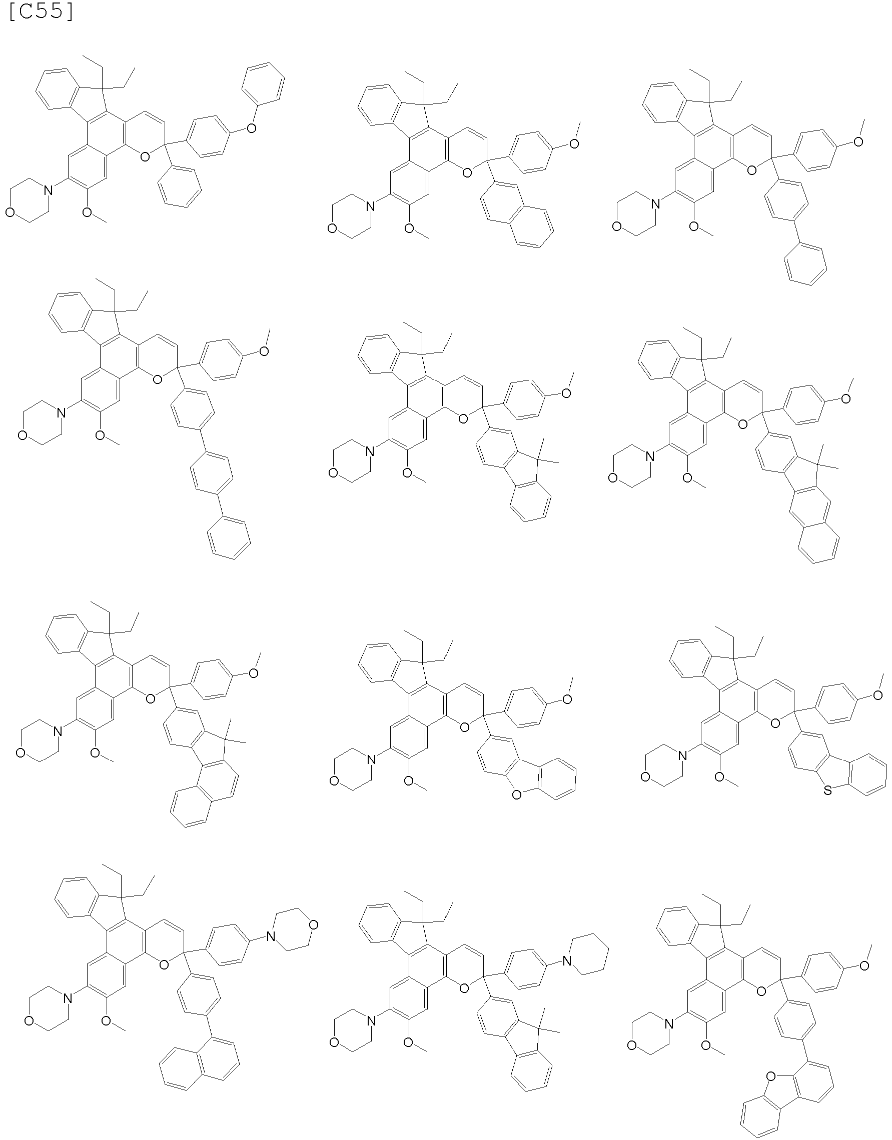

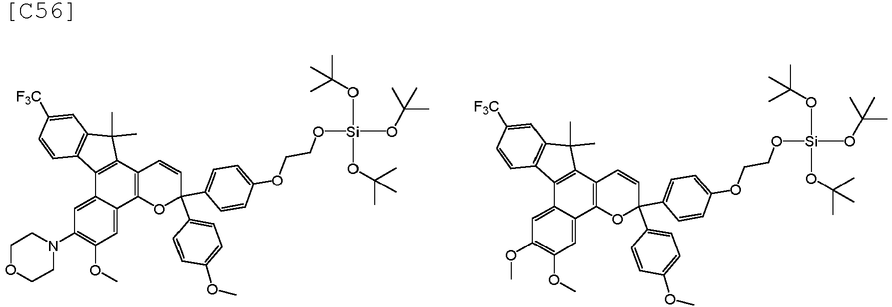

### <Compound Represented by General Formula C>

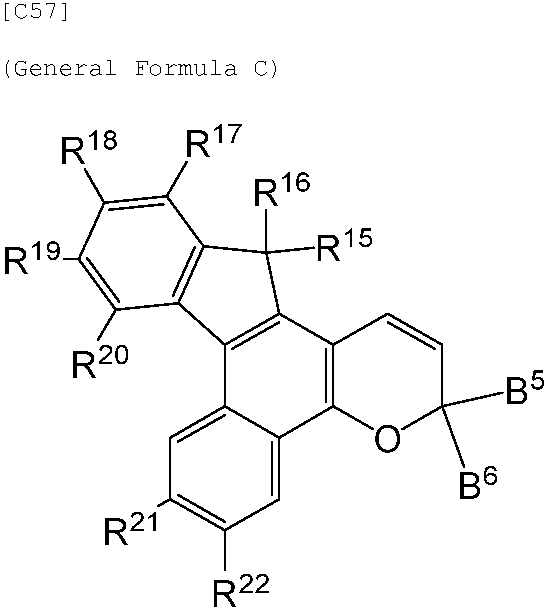

In General Formula C, R¹⁵ to R²⁰, B⁵, and B⁶ each independently represent a hydrogen atom or a substituent. One of R²¹ and R²² represents a hydrogen atom and the other represents an electron-donating group.

Preferably, R¹⁵ and R¹⁶ in General Formula C each independently represent a substituted or unsubstituted alkyl group having 1 to 20 carbon atoms, and more preferably, R¹⁵ and R¹⁶ each independently represent a methyl group, an ethyl group, a propyl group, a butyl group, a pentyl group, or a hexyl group. Further preferably, R¹⁵ and R¹⁶ each independently represent a methyl group or an ethyl group, and still further preferably, both R¹⁵ and R¹⁶ represent a methyl group or both R¹⁵ and R¹⁶ represent an ethyl group.

Preferably, B⁵ and B⁶ each independently represent a substituted or unsubstituted phenyl group. When the phenyl group has multiple substituents, two or more of these substituents may be bonded to form a ring. Specific examples of rings to be formed include rings included in exemplary compounds to be described later. The substitution position of a substituent in a substituted phenyl group is preferably a position that is the para-position with respect to the carbon atom to which B⁵ and B⁶ are bonded. Specific examples of substituents of the substituted phenyl group include a morpholino group, a piperidino group, a halogen atom, an alkoxy group, and the following substituents.

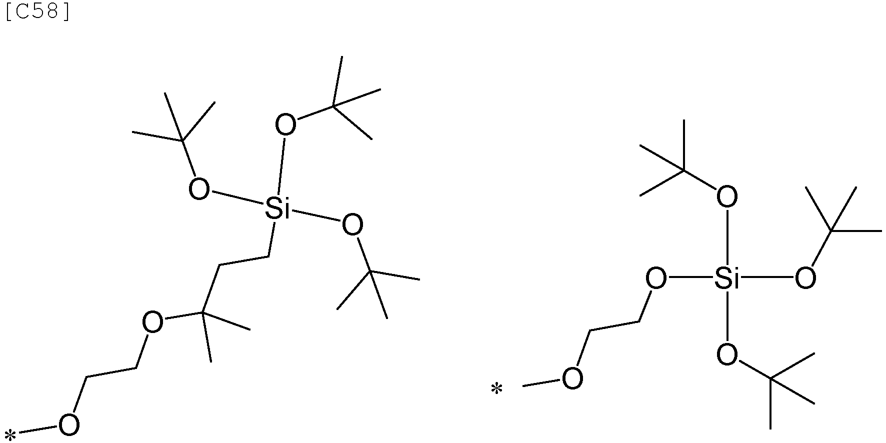

R¹⁷ to R²⁰ each independently represent a hydrogen atom or a substituent. In one aspect, R¹⁷ to R²⁰ can all be hydrogen atoms. In another aspect, R¹⁸ can be an electron-withdrawing group, and R¹⁷, R¹⁹, and R²⁰ can all be hydrogen atoms. In another aspect, R¹⁷ and R¹⁹ can each independently be an electron-withdrawing group, and R¹⁸ and R²⁰ can be hydrogen atoms. As the electron-withdrawing group, a halogen atom, a perfluoroalkyl group having 1 to 6 carbon atoms, a perfluorophenyl group, a perfluoroalkylphenyl group, or a cyano group is preferable. As the halogen atom, a fluorine atom is preferable. As the perfluoroalkyl group having 1 to 6 carbon atoms, a trifluoromethyl group is preferable.

In one aspect, R¹⁸ can be a substituted or unsubstituted phenyl group, and preferably, R¹⁸ is a substituted or unsubstituted phenyl group and R¹⁷, R¹⁹, and R²⁰ are hydrogen atoms. Specific examples of such substituted phenyl groups include a phenyl group that has been substituted with one or more halogen atoms and/or one or more cyano groups, for example, a phenyl group in which all five substitution positions of the phenyl group have been substituted with halogen atoms (preferably fluorine atoms), and a monosubstituted phenyl group in which a position that is the para-position with respect to the carbon atom to which R¹⁰ is bonded has been substituted with a cyano group.

One of R²¹ and R²² represents a hydrogen atom and the other represents an electron-donating group. Preferably, R²¹ is a hydrogen atom, and R²² is an electron-donating group. The electron-donating group represented by R²¹ or R²² (preferably R22) more preferably represents an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

Examples of the compounds represented by General Formula C include the following compounds. However, the present invention is not limited to the compounds exemplified below.

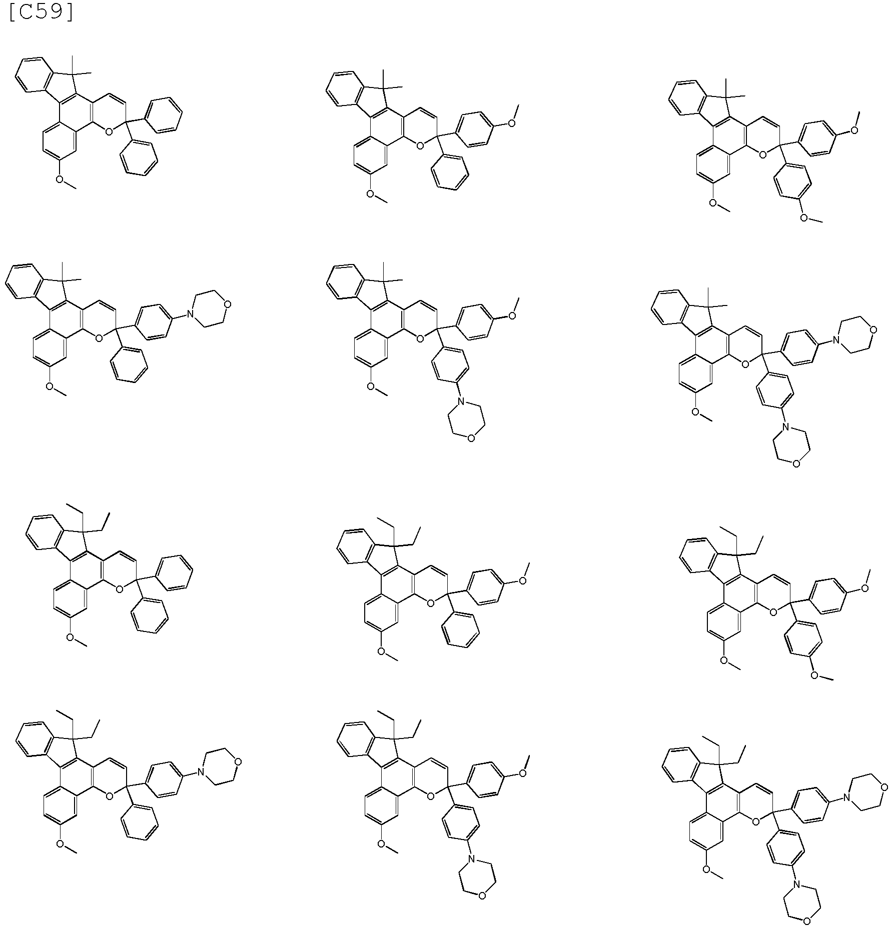

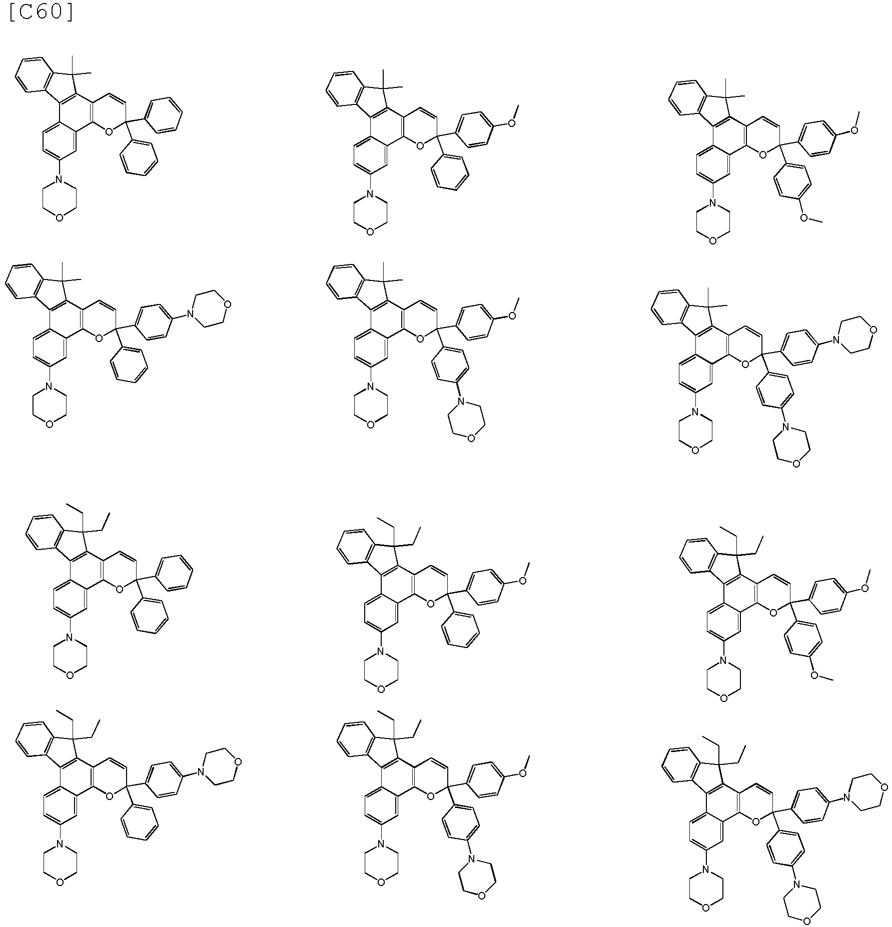

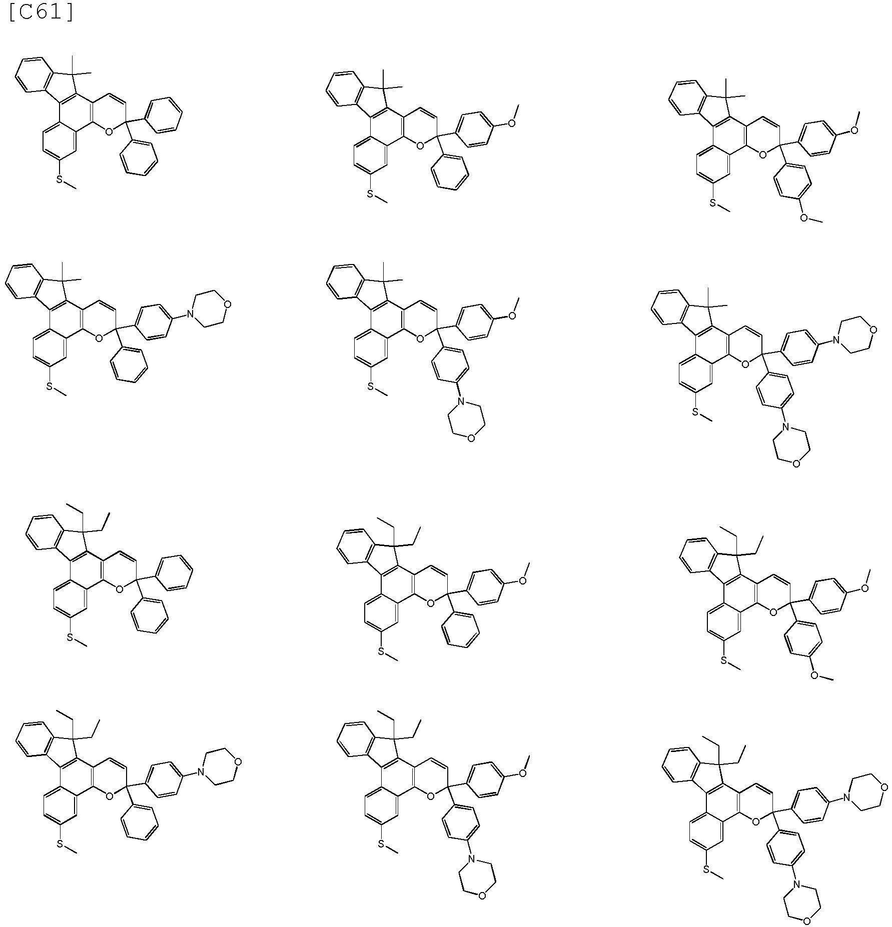

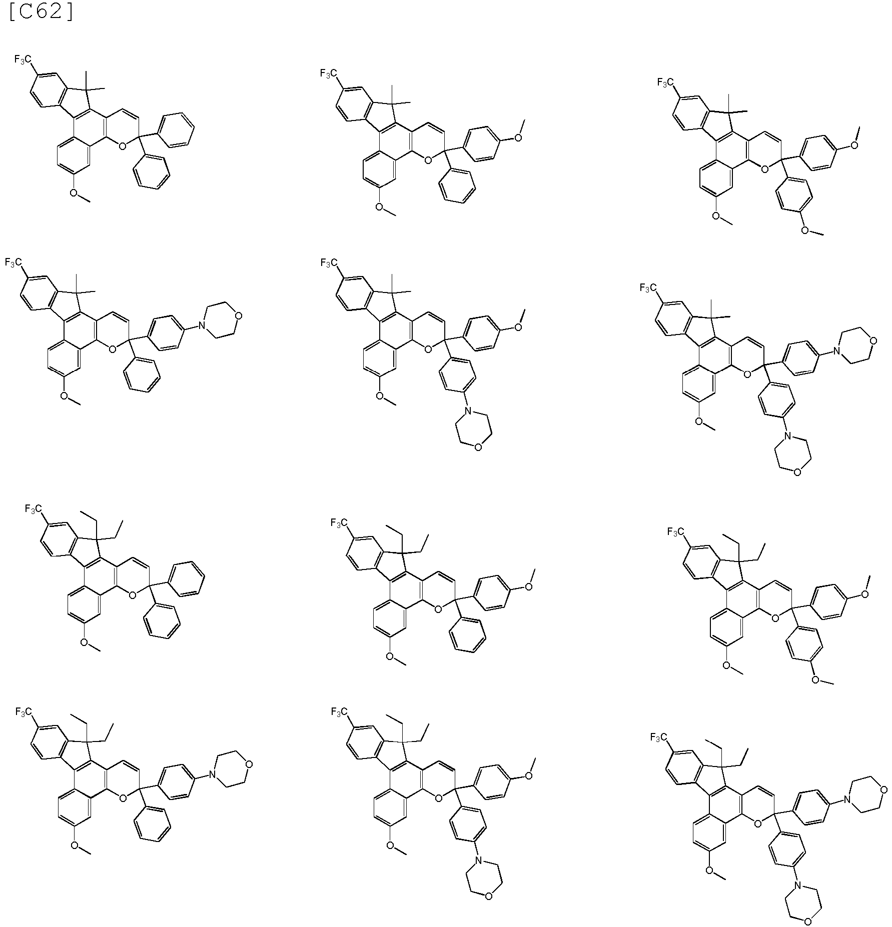

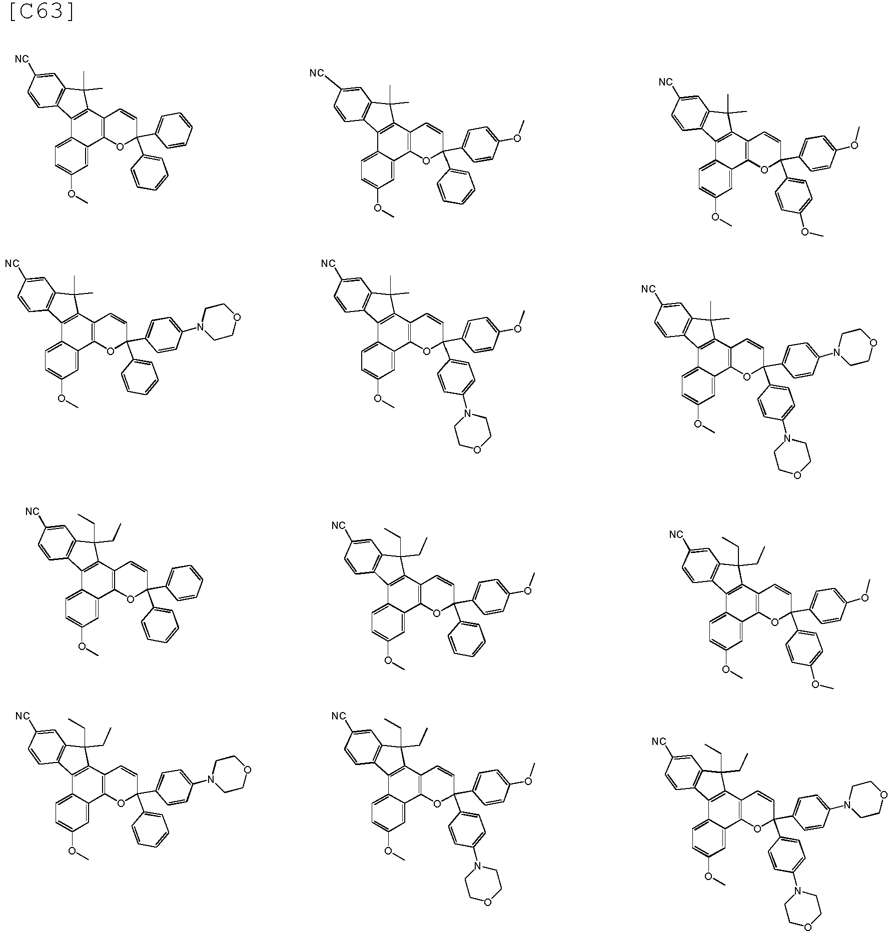

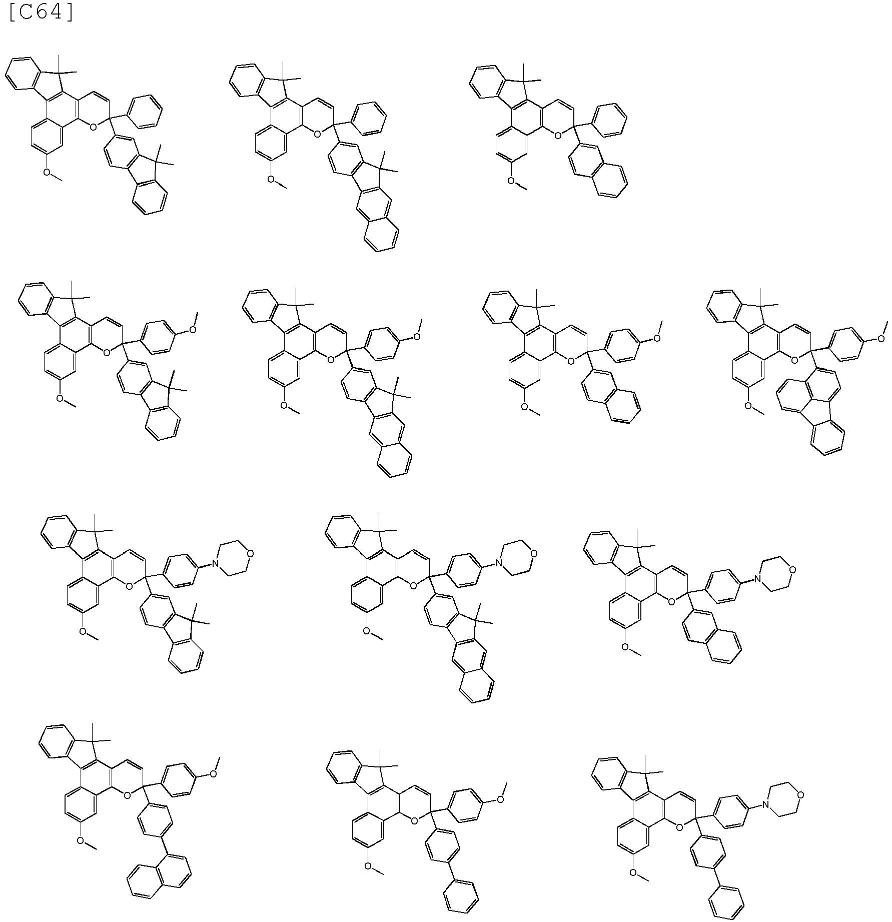

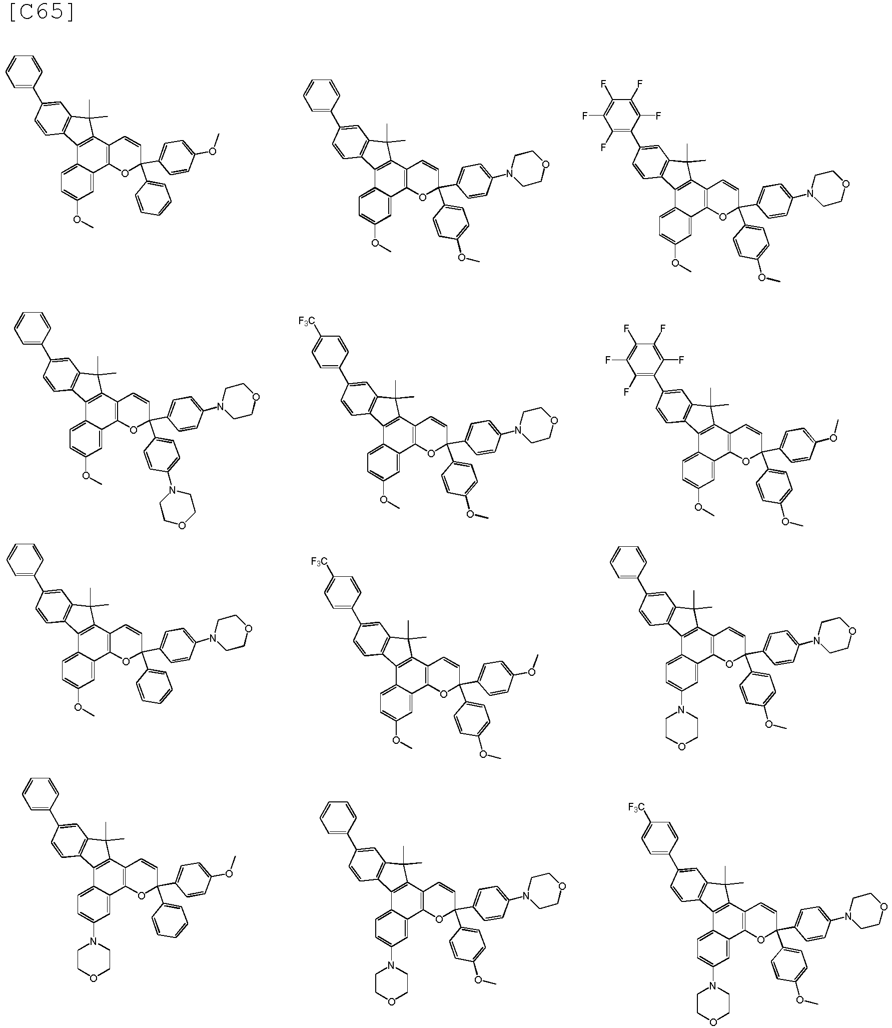

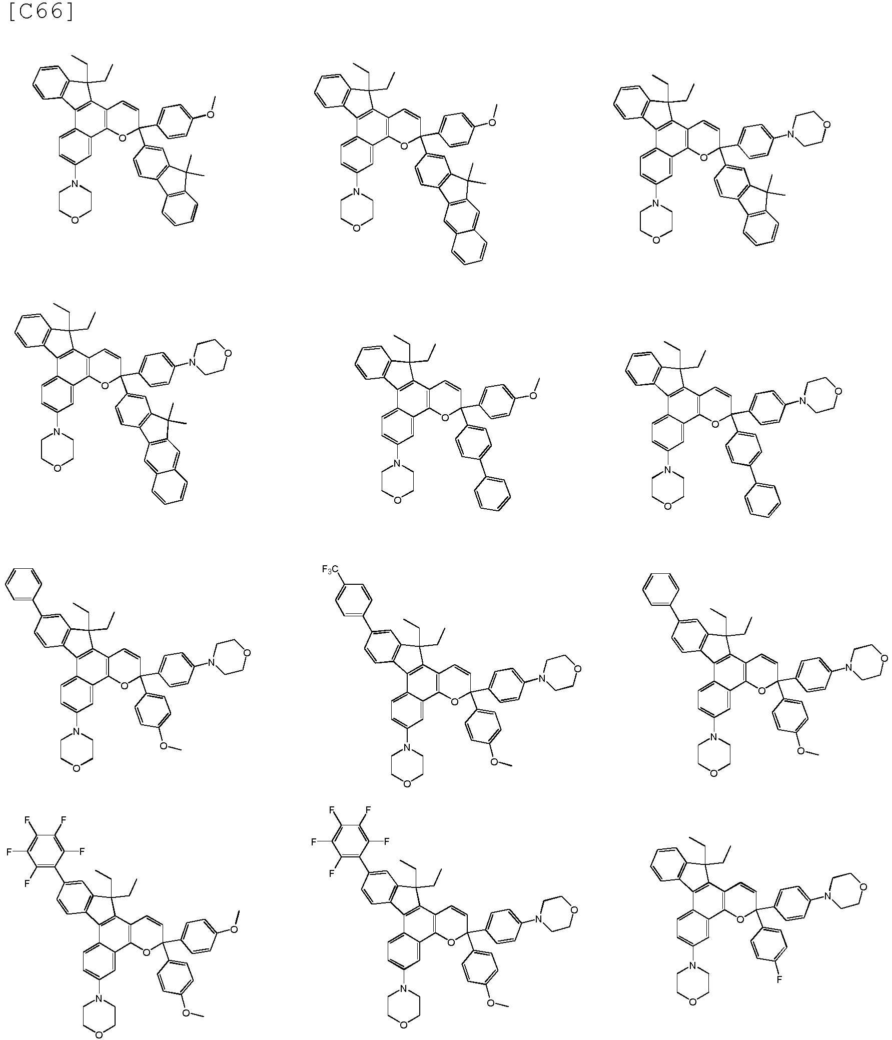

The compound represented by General Formula A, the compound represented by General Formula B, and the compound represented by General Formula C can be synthesized by a known method. For a synthesis method, the following documents can be referred to, for example. Japanese Patent No. 4884578, US 2006/0226402 A1, US 2006/0228557 A1, US 2008/0103301 A1, US 2011/0108781 A1, US 2011/0108781 A1, U.S. Patent Specification No. 7527754, U.S. Patent Specification No. 7556751, WO 2001/60811 A1, WO 2013/086248 A1, WO 1996/014596 A1, and WO 2001/019813 A1.

The photochromic article according to one aspect of the present invention and the photochromic composition according to one aspect of the present invention contain one or more compounds represented by General Formula A, one or more compounds represented by General Formula B, and one or more compounds represented by General Formula C.

For the compounds represented by General Formula A contained in the above-mentioned photochromic article and the above-mentioned photochromic composition, one type can be used alone, or two or more types (for example, two or more and four or less) can be used.

For the compounds represented by General Formula B contained in the above-mentioned photochromic article and the above-mentioned photochromic composition, one type can be used alone, or two or more types (for example, two or more and four or less) can be used.

For the compounds represented by General Formula C contained in the above-mentioned photochromic article and the above-mentioned photochromic composition, one type can be used alone, or two or more types (for example, two or more and four or less) can be used.

In the above-mentioned photochromic article and the above-mentioned photochromic composition, the total content of the compound represented by General Formula B and the compound represented by General Formula C is preferably more than the content of the compound represented by General Formula A on a mass basis. When the total of the compound represented by General Formula A, the compound represented by General Formula B, and the compound represented by General Formula C is 100 mass%, the total content of the compound represented by General Formula B and the compound represented by General Formula C is preferably more than 50 mass%, more preferably 60 mass% or more, further preferably 70 mass% or more, still further preferably 80 mass% or more, and even further preferably 90 mass% or more. With respect to the total (100 mass%) of the compound represented by General Formula A, the compound represented by General Formula B, and the compound represented by General Formula C, the total content of the compound represented by General Formula B and the compound represented by General Formula C can be less than 100 mass%, and can be 99 mass% or less, 98 mass% or less, 97 mass% or less, 96 mass% or less, or 95 mass% or less.

Regarding the mixing ratio of the compound represented by General Formula B and the compound represented by General Formula C, when the total of the compound represented by General Formula B and the compound represented by General Formula C is 100 mass%, the content of the compound represented by General Formula B can be 1 mass% or more or 99 mass% or more, and can be 25 mass% or less or 75 mass% or less. When the above-mentioned photochromic article and the above-mentioned photochromic composition contain two or more types of the compounds represented by General Formula B, the content of the compound represented by General Formula B is the total content of these compounds. The same also applies to the contents of various components in the present invention and the present specification.

In the above-mentioned photochromic article and the above-mentioned photochromic composition, when the total amount of the photochromic article and the photochromic composition is 100 mass%, the total content of the compound represented by General Formula A, the compound represented by General Formula B, and the compound represented by General Formula C can be about 0.1 to 15.0 mass%, for example. However, the above-mentioned total content is not limited to this range.

### [Photochromic Article and Photochromic Composition]

The above-mentioned photochromic article can have at least a substrate. In one aspect, the compound represented by General Formula A, the compound represented by General Formula B, and the compound represented by General Formula C can be contained in the substrate of the above-mentioned photochromic article. The above-mentioned photochromic article can have the substrate and a photochromic layer, and the compound represented by General Formula A, the compound represented by General Formula B, and the compound represented by General Formula C can be contained in the substrate and/or the photochromic layer. Regarding the substrate and the photochromic layer, the compound represented by General Formula A, the compound represented by General Formula B, and the compound represented by General Formula C can be contained only in the substrate in one aspect, can be contained only in the photochromic layer in another aspect, or can be contained in the substrate and the photochromic layer in still another aspect. In addition, in the substrate and the photochromic layer, only the compound represented by General Formula A, the compound represented by General Formula B, and the compound represented by General Formula C can be contained as photochromic compounds, or one or more of other photochromic compounds can also be contained. Examples of the other photochromic compounds include azobenzenes, spiropyrans, spirooxazines, naphthopyrans, indenonaphthopyrans, phenanthropyrans, hexaarylbisimidazoles, donor-acceptor Stenhouse adducts (DASA), salicylidene anilines, dihydropyrenes, anthracene dimers, fulgides, diarylethenes, phenoxynaphthacenequinones, and stilbenes.

<Substrate>

The above-mentioned photochromic article can contain a substrate selected according to the type of photochromic article. Examples of substrates include plastic lens substrates and glass lens substrates as spectacle lens substrates. The glass lens substrate can be a lens substrate made of inorganic glass, for example. Examples of plastic lens substrates include a styrene resin including a (meth)acrylic resin; an allyl carbonate resin such as a polycarbonate resin, an allyl resin, and a diethylene glycol bisallyl carbonate resin (CR-39); a vinyl resin; a polyester resin; polyether resin; a urethane resin obtained by reacting an isocyanate compound with a hydroxy compound such as diethylene glycol; a thiourethane resin obtained by reacting an isocyanate compound with a polythiol compound; and a cured product (generally called a transparent resin) obtained by curing a curable composition containing a (thio)epoxy compound having one or more disulfide bonds in the molecule. As the lens substrate, a undyed one (colorless lens) may be used, or a dyed one (dyed lens) may be used. The refractive index of the lens substrate can be about 1.50 to 1.75, for example. However, the refractive index of the lens substrate is not limited to the above-mentioned range, and the refractive index may be within the above-mentioned range or may be deviated from the above-mentioned range by plus or minus increment. The refractive index herein refers to the refractive index for light having a wavelength of 500 nm. In addition, the lens substrate may be a lens having refractive power (so-called prescription lens), or may be a lens not having refractive power (so-called non-prescription lens).

For example, the above-mentioned photochromic composition can be a polymerizable composition. In the present invention and the present specification, the term "polymerizable composition" is a composition containing one or more polymerizable compounds. By forming a polymerizable composition containing at least one or more compounds represented by General Formula A, one or more compounds represented by General Formula B, one or more compounds represented by General Formula C, and one or more polymerizable compounds by a known forming method, a cured product of such a polymerizable composition can be produced. Such a cured product can be contained as a substrate in the above-mentioned photochromic article and/or can be contained as a photochromic layer therein. A curing treatment can be an irradiation with light and/or heat treatment. The polymerizable compound is a compound having a polymerizable group, and as the polymerization reaction of the polymerizable compound proceeds, the polymerizable composition is cured, and thereby a cured product can be formed. The polymerizable composition can further contain one or more additives (for example, a polymerization initiator or the like).

Spectacle lenses can be various lenses such as monofocal lenses, multifocal lenses, and progressive power lenses. The type of lenses is determined by the surface shape of both surfaces of a lens substrate. In addition, the surface of the lens substrate may be any of a convex surface, a concave surface, and a flat surface. In a normal lens substrate and a spectacle lens, the object-side surface is a convex surface, and the eyeball-side surface is a concave surface. However, there is no particular limitation. The photochromic layer can usually be provided on the object-side surface of the lens substrate, but may be provided on the eyeball-side surface.

### <Photochromic Layer>

The photochromic layer can be a layer that is provided directly on the surface of the substrate or provided indirectly via one or more other layers. The photochromic layer can be, for example, a cured layer obtained by curing a polymerizable composition. The photochromic layer can be formed as a cured layer obtained by curing a polymerizable composition containing at least one or more compounds represented by General Formula A, one or more compounds represented by General Formula B, one or more compounds represented by General Formula C, and one or more polymerizable compounds. For example, by directly applying such a polymerizable composition onto the surface of the substrate or applying such a polymerizable composition onto the surface of a layer provided on the substrate and subjecting the applied polymerizable composition to a curing treatment, the photochromic layer can be formed as a cured layer containing one or more compounds represented by General Formula A, one or more compounds represented by General Formula B, and one or more compounds represented by General Formula C. As an application method, a known application method such as a spin coating method, a dip coating method, a spray coating method, an ink jet method, a nozzle coating method, and a slit coating method can be adopted. A curing treatment can be an irradiation with light and/or heat treatment. The polymerizable composition can further contain one or more additives (for example, a polymerization initiator or the like) in addition to one or more polymerizable compounds. As the polymerization reaction of the polymerizable compound proceeds, the polymerizable composition is cured, and thereby a cured layer can be formed.

The thickness of the photochromic layer can be 5 um or more, 10 um or more, or 20 um or more, for example, and can be 80 um or less, 70 um or less, or 50 um or less, for example.

### <Polymerizable Compound>

In the present invention and the present specification, a polymerizable compound refers to a compound having one or more polymerizable groups in one molecule, and the term "polymerizable group" refers to a reactive group capable of a polymerization reaction. Examples of polymerizable groups include an acryloyl group, a methacryloyl group, a vinyl group, a vinyl ether group, an epoxy group, a thiol group, an oxetane group, a hydroxy group, a carboxy group, an amino group, and an isocyanate group.

Examples of polymerizable compounds that can be used for forming the above-mentioned substrate and the above-mentioned photochromic layer include the following compounds.

### (Episulfide-Based Compound)

Episulfide-based compounds are compounds having two or more episulfide groups in one molecule. An episulfide group is a polymerizable group capable of ring-opening polymerization. Specific examples of episulfide-based compounds include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithiethane, and 2-[1,1-bis(2,3-epithiopropyldithiomethylthio)methyl]-1,3-dithiethane.

### (Thietanyl-Based Compound)

A thietanyl-based compound is a thietane compound having two or more thietanyl groups in one molecule. A thietanyl group is a polymerizable group capable of ring-opening polymerization. Some thietanyl-based compounds have multiple thietanyl groups and also an episulfide group. Examples of such compounds are described in the above-mentioned examples of episulfide-based compounds. Other thietanyl-based compounds include metal-containing thietane compounds having metal atoms in the molecule and non-metallic thietane compounds containing no metal.

Specific examples of non-metallic thietane compounds include bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bisthietanyl sulfide, bis(thietanylthio)methane, 3-[<(thietanylthio)methylthio>methylthio]thietane, bisthietanyl disulfide, bisthietanyl trisulfide, bisthietanyl tetrasulfide, bisthietanyl pentasulfide, 1,4-bis(3-thietanyldithio)-2,3-dithiabutane, 1,1,1-tris(3-thietanyldithio)methane, 1,1,1-tris(3-thietanyldithiomethylthio)methane, 1,1,2,2-tetrakis(3-thietanyldithio)ethane, and 1,1,2,2-tetrakis(3-thietanyldithiomethylthio)ethane.

Examples of metal-containing thietane compounds include compounds having, in the molecule as metal atoms, group 14 atoms such as Sn atoms, Si atoms, Ge atoms, and Pb atoms; group 4 elements such as Zr atoms and Ti atoms; group 13 atoms such as Al atoms; and group 12 atoms such as Zn atoms. Specific examples thereof include alkylthio(thietanylthio)tin, bis(alkylthio)bis(thietanylthio)tin, alkylthio(alkylthio)bis(thietanylthio)tin, bis(thietanylthio)cyclic dithiotin compounds, and alkyl(thietanylthio)tin compounds.

Specific examples of alkylthio(thietanylthio)tin include methylthiotris(thietanylthio)tin, ethylthiotris(thietanylthio)tin, propylthiotris(thietanylthio)tin, and isopropylthiotris(thietanylthio)tin.

Specific examples of bis(alkylthio)bis(thietanylthio)tin include bis(methylthio)bis(thietanylthio)tin, bis(ethylthio)bis(thietanylthio)tin, bis(propylthio)bis(thietanylthio)tin, and bis(isopropylthio)bis(thietanylthio)tin.

Specific examples of alkylthio(alkylthio)bis(thietanylthio)tin include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, and isopropylthio(propylthio)bis(thietanylthio)tin.

Specific examples of the bis(thietanylthio)cyclic dithiotin compounds include bis(thietanylthio)dithiastannetane, bis(thietanylthio)dithiastannolane, bis(thietanylthio)dithiastanninane, and bis(thietanylthio)trithiastannocane.

Specific examples of alkyl(thietanylthio)tin compounds include methyltris(thietanylthio)tin, dimethylbis(thietanylthio)tin, butyltris(thietanylthio)tin, tetrakis(thietanylthio)tin, tetrakis(thietanylthio)germanium, and tris(thietanylthio)bismuth.

### (Polyamine Compound)

A polyamine compound is a compound having two or more NH₂ groups in one molecule, and can form a urea bond by reaction with polyisocyanate and can form a thiourea bond by reaction with polyisothiocyanate. Specific examples of polyamine compounds include ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine, and 1,3,5-benzenetriamine.

### (Epoxy-Based Compound)

Epoxy-based compounds are compounds having an epoxy group in the molecule. An epoxy group is a polymerizable group capable of ring-opening polymerization. Epoxy compounds are generally classified into aliphatic epoxy compounds, alicyclic epoxy compounds, and aromatic epoxy compounds.

Specific examples of aliphatic epoxy compounds include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, and triglycidyl ether of tris(2-hydroxyethyl)isocyanurate.

Specific examples of alicyclic epoxy compounds include isophorone diol diglycidyl ether and bis-2,2-hydroxycyclohexylpropane diglycidyl ether.

Specific examples of aromatic epoxy compounds include resorcinol diglycidyl ether, bisphenol A diglycidyl ether, bisphenol F diglycidyl ether, bisphenol S diglycidyl ether, orthophthalic acid diglycidyl ester, phenol novolac polyglycidyl ether, and cresol novolac polyglycidyl ether.

In addition to the above-mentioned examples, an epoxy-based compound having a sulfur atom in the molecule can also be used together with the epoxy group. Such epoxy-based compounds containing a sulfur atom include linear aliphatic compounds and cycloaliphatic compounds.

Specific examples of linear aliphatic epoxy-based compounds containing a sulfur atom include bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, and 2,2-bis(2,3-epoxypropylthiomethyl)-1-(2,3-epoxypropylthio)butane.

Specific examples of cycloaliphatic epoxy-based compounds containing a sulfur atom include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, and 2,5-bis(2,3-epoxypropylthiomethyl)-2,5-dimethyl-1,4-dithiane.

### (Compound Having Radically Polymerizable Group)

A compound having a radically polymerizable group is a polymerizable group capable of radical polymerization. Examples of radically polymerizable groups include an acryloyl group, a methacryloyl group, an allyl group, and a vinyl group.

A compound having a polymerizable group selected from the group consisting of an acryloyl group and a methacryloyl group is hereinafter referred to as a "(meth)acrylate compound". Specific examples of (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl (meth)acrylate, bisphenol A di(meth)acrylate, 2,2-bis(4-(meth)acryloxyethoxyphenyl)propane, 2,2-bis(4-(meth)acroxydiethoxyphenyl)propane, 2,2-bis(4-(meth)acryloyloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bisphenol F di(meth)acrylate, 1,1-bis(4-(meth)acryloxyethoxyphenyl)methane, 1,1-bis(4-(meth)acroxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methylthio(meth)acrylate, phenylthio(meth)acrylate, benzylthio(meth)acrylate, xylylenedithiol di(meth)acrylate, mercaptoethyl sulfide di(meth)acrylate, and bifunctional urethane (meth)acrylate.

Specific examples of compounds (allyl compounds) having an allyl group include allyl glycidyl ether, diallyl phthalate, diallyl terephthalate, diallyl isophthalate, diallyl carbonate, diethylene glycol bisallyl carbonate, methoxypolyethylene glycol allyl ether, polyethylene glycol allyl ether, methoxypolyethylene glycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, and methacryloyloxy polyethylene glycol allyl ether.

Examples of compounds (vinyl compounds) having a vinyl group include α-methylstyrene, α-methylstyrene dimer, styrene, chlorostyrene, methylstyrene, bromostyrene, dibromostyrene, divinylbenzene, and 3,9-divinylspirobi(m-dioxane).

The above-mentioned photochromic article can include, at an arbitrary location, one or more layers known as functional layers of the photochromic article such as a protective layer for improving the durability of the photochromic article, an antireflection layer, a water-repellent or hydrophilic antifouling layer, an antifogging layer, and a primer layer for improving adhesiveness between layers.

The above-mentioned photochromic article can be an optical article. One aspect of the optical article is spectacle lenses. Such spectacle lenses can also be called photochromic lenses or photochromic spectacle lenses. In addition, as one aspect of the optical article, there are a lens for goggles, a visor (brim) portion of a sun visor, a shield member of a helmet, and the like. An optical article having an antiglare function can be obtained by applying the above-mentioned photochromic composition, which is a polymerizable composition, onto a substrate for these optical articles, and subjecting the applied composition to a curing treatment to form a photochromic layer.

### [Spectacles]

One aspect of the present invention relates to spectacles having a spectacle lens which is one aspect of the above-mentioned photochromic article. The details of the spectacle lens included in these spectacles is as described above. By providing such a spectacle lens, the above-mentioned spectacles can exhibit an antiglare effect as in the case of sunglasses by coloring of the photochromic compound upon irradiate with sunlight outdoors, and transmittance can be restored by fading of the photochromic compound when returned indoors. For the above-mentioned spectacles, a known technique can be applied to the configuration of a frame and the like.

### [Examples]

Hereinbelow, the present invention will be further described with reference to examples. However, the present invention is not limited to the embodiments described in the examples.

### [Synthesis of Compounds]

Compounds 1 to 15 shown below were synthesized by referring to the references described above regarding the synthesis method of the compounds. The compounds were identified in the same manner as described in the reference publication to confirm whether the compounds shown below were synthesized. Compounds 1 to 7 are compounds represented by General Formula A, compounds 8 to 15 are compounds represented by General Formula B, and compounds 16 to 23 are compounds represented by General Formula C.

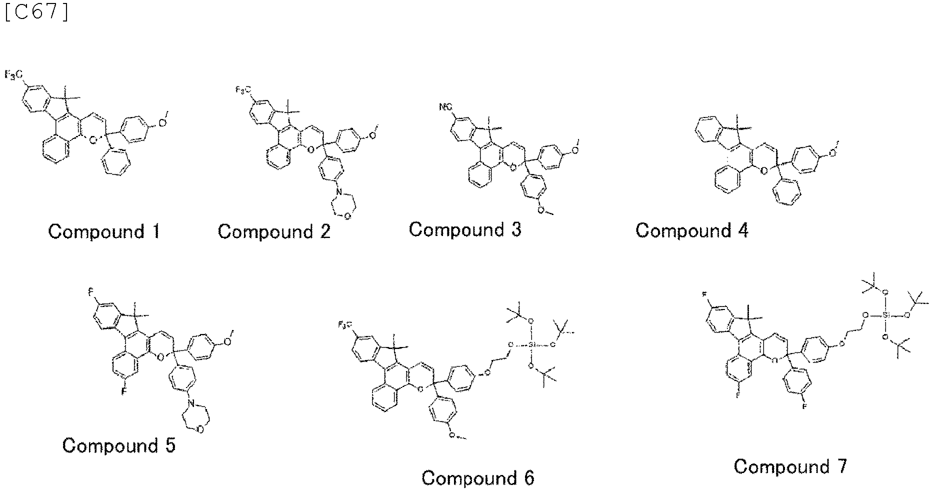

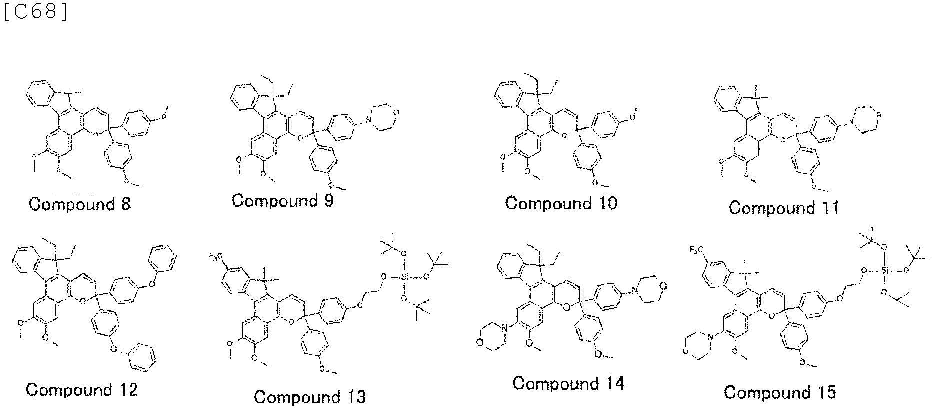

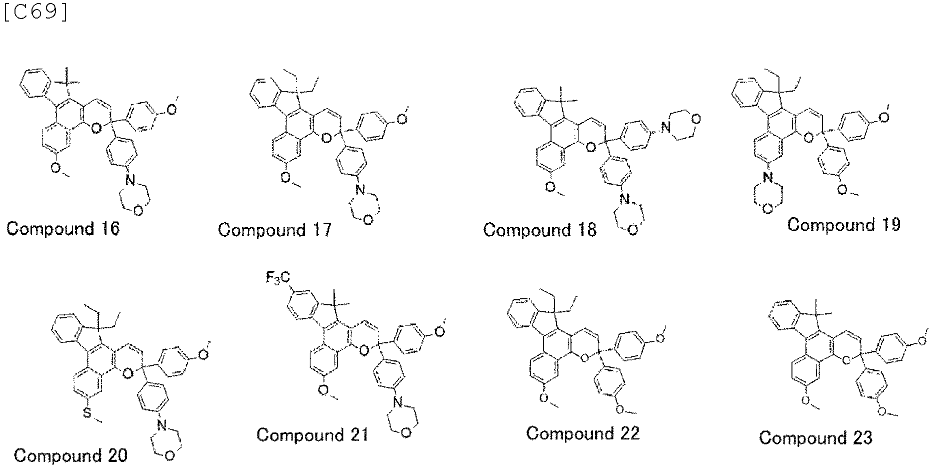

### [Examples 1 to 48 and Comparative Examples 1 to 3]

### <Preparation of Photochromic Composition (Polymerizable Composition)>

In a plastic container, with respect to a total of 100 parts by mass of (meth)acrylate, 68 parts by mass of polyethylene glycol diacrylate, 12 parts by mass of trimethylolpropane trimethacrylate, and 20 parts by mass of neopentyl glycol dimethacrylate were mixed to prepare a (meth)acrylate mixture. A photochromic compound was mixed to 100 parts by mass of this (meth)acrylate mixture such that the amount was 2.5 parts by mass. For compositions containing multiple photochromic compounds, Table 2 (Table 2-1 and Table 2-2) shows the mass ratio of each photochromic compound when the total amount of photochromic compounds was 10. Furthermore, a photopolymerization initiator (phenylbis(2,4,6-trimethylbenzoyl)phosphine oxide), an antioxidant [bis(3-tert-butyl-4-hydroxy-5-methylphenyl)propionic acid)] [ethylene bis(oxyethylene), and a light stabilizer (bis(1,2,2,6,6-pentamethyl-4-piperidyl)sebacic acid) were mixed, and after sufficient stirring, a silane coupling agent (γ-methacryloxypropyltrimethoxysilane) was added dropwise while stirring. Thereafter, defoaming was caused by an automatic revolution type stirring and defoaming device.

A photochromic composition was prepared by the method described above.

### <Formation of Primer Layer>

A plastic lens substrate (manufactured by HOYA CORPORATION, trade name: EYAS, center thickness: 2.5 mm, diameter: 75 mm, spherical lens power: -4.00) was immersed in an aqueous sodium hydroxide solution having a concentration of 10 mass% (liquid temperature 60°C) for 5 minutes to perform alkaline cleaning, further cleaning with pure water was performed, and drying was performed. Thereafter, in an environment of room temperature and relative humidity of 40% to 60% and by a spin coating method, an aqueous polyurethane resin liquid (polycarbonate polyol-based polyurethane emulsion, viscosity: 100 cPs, concentration of solid contents: 38 mass%) was applied to the convex surface of this plastic lens substrate at a rotation speed of 1500 rpm for 1 minute using a spin coater MS-B150 manufactured by Mikasa Corporation, and thereafter air drying was performed for 15 minutes to form a primer layer having a thickness of 5.5 µm.

### <Formation of Photochromic Layer>

The photochromic composition prepared as above was added dropwise onto the above-mentioned primer layer, and was applied by a spin coating method using a program to change the rotation speed from 500 rpm to 1500 rpm over 1 minute in slope mode and to further cause rotation at 1500 rpm for 5 seconds using MS-B150 manufactured by Mikasa Corporation. Thereafter, the photochromic composition applied onto the primer layer formed on the plastic lens substrate was irradiated with ultraviolet rays (main wavelength: 405 nm) for 40 seconds in a nitrogen atmosphere (oxygen concentration: 500 ppm or less), and this composition was cured to form a photochromic layer. The thickness of the formed photochromic layer was 45 um.

Thereby, a photochromic article (spectacle lens) was produced.

### [Evaluation Method]

### <Evaluation of Coloring Density>

The luminous reflectance was obtained by the following method in accordance with JIS T7333:2005.

The convex surface of each spectacle lens of the examples and the comparative examples was irradiated with light through an aeromass filter for 15 minutes using a xenon lamp as a light source to cause coloring of the photochromic layer. This irradiation with light was performed such that the irradiance and the tolerance of the irradiance were the values shown in Table 1 as defined in JIS T 7333:2005. The transmittance when coloring was measured with a spectrophotometer manufactured by Otsuka Electronics Co., Ltd. Table 2 shows the luminous transmittance T (%) obtained from the measurement results in the wavelength range of 380 nm to 780 nm. A smaller value of T (%) means that the photochromic compound colors at a higher density.

**[Table 1]**

| Wavelength region(nm) | Irradiance (W/m²) | Tolerance of irradiance (W/m²) |
|---|---|---|
| 300 to 340 | < 2.5 | |
| 340 to 380 | 5.6 | ± 1.5 |
| 380 to 420 | 12 | ± 3.0 |
| 420 to 460 | 12 | ± 3.0 |
| 460 to 500 | 26 | ± 2.6 |

### <Evaluation of Fading Speed>

The fading speed was evaluated by the following method.

The transmittance (measurement wavelength: 550 nm) of each spectacle lens of the examples and the comparative examples before irradiation with light (uncolored state) was measured with a spectrophotometer manufactured by Otsuka Electronics Co., Ltd. The transmittance measured herein is called "initial transmittance".

Each of the spectacle lenses was irradiated with light through an aeromass filter for 15 minutes using a xenon lamp as a light source to cause coloring of the photochromic layer. This irradiation with light was performed such that the irradiance and the tolerance of the irradiance were the values shown in Table 1 as defined in JIS T7333:2005. The transmittance when coloring was measured in the same manner as the initial transmittance. The transmittance measured herein is called "transmittance when coloring".

Thereafter, the time required for the transmittance to reach [(initial transmittance - transmittance when coloring)/2] from the time when irradiation with light was stopped was measured. This time is called "half-life time". It can be said that the shorter the half-life time, the faster the fading speed. Table 2 shows the obtained half-life time.

**[Table 2-1]**

| | Photochromic compound | Mass ratio | Coloring density T (%) | Fading speed half-life time (sec) |
|---|---|---|---|---|
| Example 1 | Compound 1/Compound 8/Compound 16 | 1/5/4 | 17.6 | 185 |
| Example 2 | Compound 1/Compound 9/Compound 16 | 1/5/4 | 17.2 | 170 |
| Example 3 | Compound 3/Compound 10/Compound 16 | 1/5/4 | 18.0 | 170 |
| Example 4 | Compound 3/Compound 12/Compound 16 | 2/4/4 | 18.0 | 185 |
| Example 5 | Compound 3/Compound 13/Compound 16 | 1/7/3 | 19.0 | 170 |
| Example 6 | Compound 7/Compound 10/Compound 16 | 2/5/3 | 18.8 | 180 |
| Example 7 | Compound 1/Compound 9/Compound 17 | 1/5/4 | 18.5 | 175 |
| Example 8 | Compound 1/Compound 11/Compound 17 | 1/3/7 | 18.0 | 190 |
| Example 9 | Compound 1/Compound 14/Compound 17 | 1/5/4 | 17.8 | 180 |
| Example 10 | Compound 3/Compound 10/Compound 17 | 1/5/4 | 19.0 | 170 |
| Example 11 | Compound 4/Compound 10/Compound 17 | 1/5/4 | 17.6 | 170 |
| Example 12 | Compound 7/Compound 13/Compound 17 | 1/5/4 | 19.0 | 165 |
| Example 13 | Compound 1/Compound 9/Compound 18 | 1/6/3 | 18.2 | 170 |
| Example 14 | Compound 1/Compound 10/Compound 18 | 1/5/4 | 18.4 | 175 |
| Example 15 | Compound 3/Compound 10/Compound 18 | 1/5/4 | 18.6 | 170 |
| Example 16 | Compound 4/Compound 15/Compound 18 | 1/7/2 | 17.2 | 160 |
| Example 17 | Compound 5/Compound 14/Compound 18 | 1/6/3 | 17.0 | 160 |
| Example 18 | Compound 7/Compound 15/Compound 18 | 2/6/2 | 16.9 | 160 |
| Example 19 | Compound 2/Compound 8/Compound 19 | 3/3/4 | 17.0 | 190 |
| Example 20 | Compound 3/Compound 9/Compound 19 | 1/5/4 | 18.5 | 150 |
| Example 21 | Compound 3/Compound 10/Compound 19 | 1/5/4 | 18.7 | 145 |
| Example 22 | Compound 5/Compound 10/Compound 19 | 3/4/3 | 18.9 | 150 |
| Example 23 | Compound 4/Compound 14/Compound 19 | 1/5/4 | 18.6 | 140 |
| Example 24 | Compound 6/Compound 10/Compound 19 | 2/4/4 | 19.0 | 135 |

**[Table 2-2]**

| | Photochromic compound | Mass ratio | Coloring density T (%) | Fading speed half-life time (sec) |
|---|---|---|---|---|
| Example 25 | Compound 1/Compound 8/Compound 20 | 1/3/6 | 18.2 | 175 |
| Example 26 | Compound 1/Compound 9/Compound 20 | 1/5/4 | 18.0 | 155 |
| Example 27 | Compound 3/Compound 10/Compound 20 | 1/5/4 | 19.0 | 140 |
| Example 28 | Compound 3/Compound 12/Compound 20 | 2/4/4 | 18.2 | 150 |
| Example 29 | Compound 7/Compound 13/Compound 20 | 1/5/4 | 17.8 | 140 |
| Example 30 | Compound 7/Compound 10/Compound 20 | 1/5/4 | 19.0 | 130 |
| Example 31 | Compound 1/Compound 13/Compound 21 | 1/6/3 | 18.8 | 135 |
| Example 32 | Compound 2/Compound 10/Compound 21 | 3/3/4 | 19.2 | 130 |
| Example 33 | Compound 5/Compound 9/Compound 21 | 2/4/4 | 18.0 | 125 |
| Example 34 | Compound 1/Compound 9/Compound 21 | 1/5/4 | 17.8 | 130 |
| Example 35 | Compound 1/Compound 13/Compound 21 | 1/5/4 | 19.4 | 140 |
| Example 36 | Compound 7/Compound 13/Compound 21 | 1/5/4 | 18.8 | 130 |
| Example 37 | Compound 1/Compound 9/Compound 22 | 1/5/4 | 16.8 | 180 |
| Example 38 | Compound 2/Compound 9/Compound 22 | 1/5/4 | 17.7 | 170 |
| Example 39 | Compound 3/Compound 14/Compound 22 | 1/4/5 | 17.8 | 165 |
| Example 40 | Compound 5/Compound 12/Compound 22 | 1/4/5 | 17.0 | 165 |
| Example 41 | Compound 5/Compound 14/Compound 22 | 1/5/4 | 17.8 | 155 |
| Example 42 | Compound 7/Compound 15/Compound 22 | 1/6/3 | 18.0 | 140 |
| Example 43 | Compound 1/Compound 9/Compound 23 | 3/5/2 | 17.0 | 175 |
| Example 44 | Compound 3/Compound 9/Compound 23 | 4/4/2 | 17.0 | 170 |
| Example 45 | Compound 3/Compound 10/Compound 23 | 2/6/2 | 18.0 | 165 |
| Example 46 | Compound 5/Compound 9/Compound 23 | 3/5/2 | 19.0 | 175 |
| Example 47 | Compound 5/Compound 10/Compound 23 | 1/7/2 | 18.8 | 170 |
| Example 48 | Compound 6/Compound 13/Compound 23 | 2/6/2 | 18.2 | 170 |
| Comparative Example 1 | Compound 1 | 10 | 25.0 | 150 |
| Comparative Example 2 | Compound 8 | 10 | 21.0 | 240 |
| Comparative Example 3 | Compound 16 | 10 | 23.0 | 190 |

From the results shown in Table 2, it can be confirmed that by combining the compound represented by General Formula A, the compound represented by General Formula B, and the compound represented by General Formula C, a photochromic article that colors at a high density in the visible range can be provided. Furthermore, from the results shown in Table 2, it can be confirmed that each of the spectacle lenses of the examples exhibits a fast fading speed.

Finally, each of aspects described above will be summarized.

According to one aspect, a photochromic article and a photochromic composition which contain one or more compounds represented by General Formula A, one or more compounds represented by General Formula B, and one or more compounds represented by General Formula C are provided.

In one aspect, R¹ and R² in General Formula A may each independently represent a substituted or unsubstituted alkyl group having 1 to 20 carbon atoms.

In one aspect, R¹ and R² in General Formula A may each independently represent a methyl group, an ethyl group, a propyl group, a butyl group, a pentyl group, or a hexyl group.

In one aspect, B¹ and B² in General Formula A, B³ and B⁴ in General Formula B, and B⁵ and B⁶ in General Formula C may each independently represent a substituted or unsubstituted phenyl group. When such a phenyl group has multiple substituents, these substituents may be bonded to form a ring.

In one aspect, R³ to R⁶ in General Formula A can each independently represent a hydrogen atom or an electron-withdrawing group (provided that one or more of R³ to R⁶ represent an electron-withdrawing group).

In one aspect, the above-mentioned electron-withdrawing group may be a halogen atom, a perfluoroalkyl group having 1 to 6 carbon atoms, a perfluorophenyl group, a perfluoroalkylphenyl group, or a cyano group.

In one aspect, the above-mentioned halogen atom may be a fluorine atom.

In one aspect, the above-mentioned perfluoroalkyl group may be a trifluoromethyl group.

In one aspect, one or more selected from the group consisting of R¹ to R⁶, B¹, and B² in General Formula A, R⁷ to R¹², B³, and B⁴ in General Formula B, and R¹⁵ to R²⁰, B⁵, and B⁶ in General Formula C can represent a substituent, and such a substituent is
a substituent R^{m} selected from the group consisting of a hydroxy group, a linear or branched alkyl group having 1 to 18 carbon atoms, a cycloaliphatic alkyl group of a single-ring type or a multi-ring type such as a bicyclic ring which has 5 to 18 carbon atoms, a linear or branched alkoxy group having 1 to 24 constituent atoms, a non-aromatic cyclic substituent having 1 to 24 constituent atoms, a linear or branched perfluoroalkyl group having 1 to 18 carbon atoms, a linear or branched perfluoroalkoxy group, a linear or branched alkylsulfide group having 1 to 24 constituent atoms, an aryl group, an aryloxy group, an arylsulfide group, a heteroaryl group, an amino group, a monoalkylamino group, a dialkylamino group, a monoarylamino group, a diarylamino group, a cyclic amino group such as a piperidino group, a morpholino group, a thiomorpholino group, a tetrahydroquinolino group, and a tetrahydroisoquinolino group, an ethynyl group, a mercapto group, a silyl group, a sulfonic acid group, an alkylsulfonyl group, a formyl group, a carboxy group, a cyano group, and a halogen atom;
a substituent in which R^{m} is further substituted with one or more same or different R^{m}'s; or
a solubilizing group.

In one aspect, R⁷ and R⁸ in General Formula B may each independently represent a substituted or unsubstituted alkyl group having 1 to 20 carbon atoms.

In one aspect, R⁷ and R⁸ in General Formula B may each independently represent a methyl group, an ethyl group, a propyl group, a butyl group, a pentyl group, or a hexyl group.

In one aspect, in General Formula B, R¹³ and R¹⁴ may each independently represent an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

In one aspect, R¹⁵ and R¹⁶ in General Formula C may each independently represent a substituted or unsubstituted alkyl group having 1 to 20 carbon atoms.

In one aspect, R¹⁵ and R¹⁶ in General Formula C may each independently represent a methyl group, an ethyl group, a propyl group, a butyl group, a pentyl group, or a hexyl group.

In one aspect, in General Formula C, an electron-donating group represented by R²¹ or R²² can be an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

In one aspect, in the above-mentioned photochromic article and the above-mentioned photochromic composition, the total content of the compound represented by General Formula B and the compound represented by General Formula C can be more than the content of the compound represented by General Formula A on a mass basis.

In one aspect, the above-mentioned photochromic article may be a photochromic article which has a substrate and a photochromic layer, and which contains, in this photochromic layer, one or more compounds represented by General Formula A, one or more compounds represented by General Formula B, and one or more compounds represented by General Formula C.

In one aspect, the above-mentioned photochromic layer may be a cured layer obtained by curing a polymerizable composition.

In one aspect, the above-mentioned photochromic composition may contain a polymerizable compound.

In one aspect, the above-mentioned photochromic article may be a spectacle lens.

In one aspect, the above-mentioned photochromic article may be a lens for goggles.

In one aspect, the above-mentioned photochromic article may be a visor portion of a sun visor.

In one aspect, the above-mentioned photochromic article may be a shield member of a helmet.

According to one aspect, spectacles including the above-mentioned spectacle lens are provided.

Two or more of the various aspects and various forms described in the present specification can be combined in any combination.

It should be considered that the embodiments described this time are examples in all respects and not restrictive. The scope of the present invention is indicated by the scope of the claims rather than the above description, and is intended to include all modifications within the meaning and scope equivalent to the scope of the claims.

### [Industrial Applicability]

One aspect of the present invention is useful in technical fields such as spectacles, goggles, sun visors, and helmets.

### [List of embodiments]

### [Embodiment 1]

A photochromic composition comprising:
one or more compounds represented by the following General Formula A;
one or more compounds represented by the following General Formula B; and
one or more compounds represented by the following General Formula C, 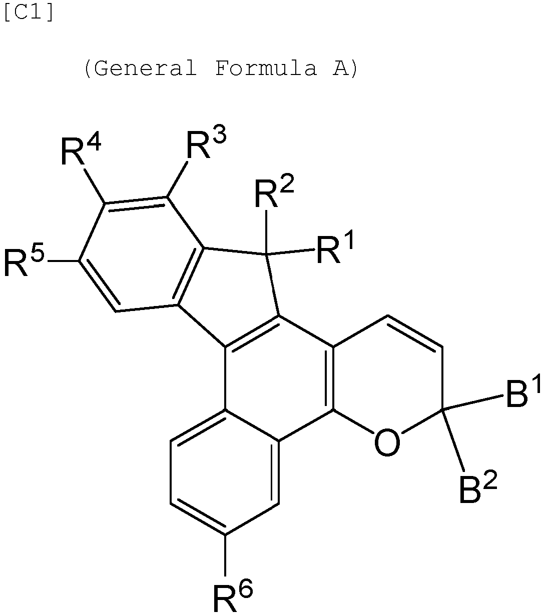
(in General Formula A, R¹ to R⁶, B¹, and B² each independently represent a hydrogen atom or a substituent) 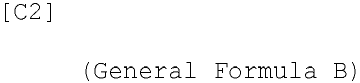 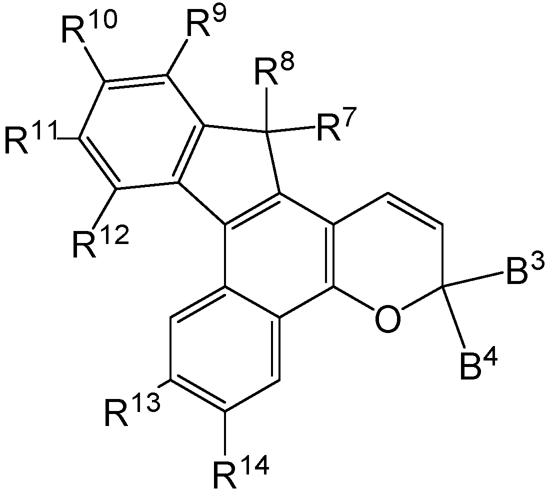
(in General Formula B,
R⁷ to R¹², B³, and B⁴ each independently represent a hydrogen atom or a substituent, and
R¹³ and R¹⁴ each independently represent an electron-donating group), and 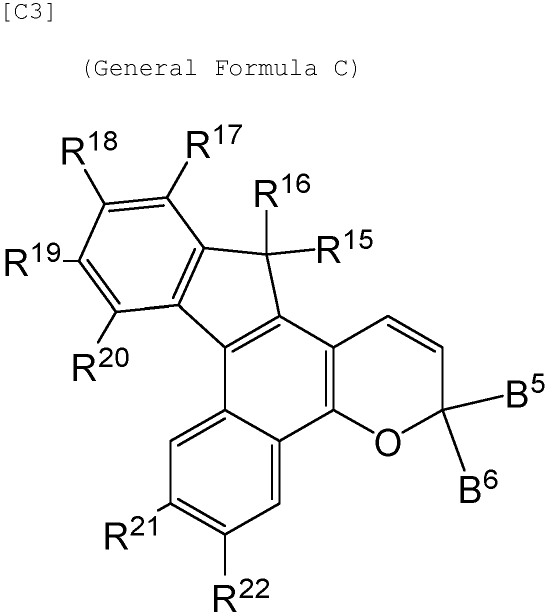
(in General Formula C,
R¹⁵ to R²⁰, B⁵, and B⁶ each independently represent a hydrogen atom or a substituent, and
one of R²¹ and R²² represents a hydrogen atom and the other represents an electron-donating group).

### [Embodiment 2]

The photochromic composition according to Embodiment 1, wherein R¹ and R² in General Formula A each independently represent a substituted or unsubstituted alkyl group having 1 to 20 carbon atoms.

### [Embodiment 3]

The photochromic composition according to Embodiment 1 or 2, wherein R¹ and R² in General Formula A each independently represent a methyl group, an ethyl group, a propyl group, a butyl group, a pentyl group, or a hexyl group.

### [Embodiment 4]

The photochromic composition according to any one of Embodiments 1 to 3, wherein B¹ and B² in General Formula A, B³ and B⁴ in General Formula B, and B⁵ and B⁶ in General Formula C each independently represent a substituted or unsubstituted phenyl group, provided that when the phenyl group has a plurality of substituents, the substituents may be bonded to form a ring.

### [Embodiment 5]

The photochromic composition according to any one of Embodiments 1 to 4, wherein R³ to R⁶ in General Formula A each independently represent a hydrogen atom or an electron-withdrawing group, provided that one or more of R³ to R⁶ represent an electron-withdrawing group.

### [Embodiment 6]

The photochromic composition according to Embodiment 5, wherein the electron-withdrawing group is a halogen atom, a perfluoroalkyl group having 1 to 6 carbon atoms, a perfluorophenyl group, a perfluoroalkylphenyl group, or a cyano group.

### [Embodiment 7]

The photochromic composition according to Embodiment 6, wherein the halogen atom is a fluorine atom.

### [Embodiment 8]

The photochromic composition according to Embodiment 6, wherein the perfluoroalkyl group is a trifluoromethyl group.

### [Embodiment 9]

The photochromic composition according to any one of Embodiments 1 to 8, wherein
one or more selected from the group consisting of R¹ to R⁶, B¹, and B² in General Formula A, R⁷ to R¹², B³, and B⁴ in General Formula B, and R¹⁵ to R²⁰, B⁵, and B⁶ in General Formula C represent a substituent, and
the substituent is
   a substituent R^{m} selected from the group consisting of a hydroxy group, a linear or branched alkyl group having 1 to 18 carbon atoms, a cycloaliphatic alkyl group of a single-ring type or a multi-ring type such as a bicyclic ring which has 5 to 18 carbon atoms, a linear or branched alkoxy group having 1 to 24 constituent atoms, a non-aromatic cyclic substituent having 1 to 24 constituent atoms, a linear or branched perfluoroalkyl group having 1 to 18 carbon atoms, a linear or branched perfluoroalkoxy group, a linear or branched alkylsulfide group having 1 to 24 constituent atoms, an aryl group, an aryloxy group, an arylsulfide group, a heteroaryl group, an amino group, a monoalkylamino group, a dialkylamino group, a monoarylamino group, a diarylamino group, a cyclic amino group such as a piperidino group, a morpholino group, a thiomorpholino group, a tetrahydroquinolino group, and a tetrahydroisoquinolino group, an ethynyl group, a mercapto group, a silyl group, a sulfonic acid group, an alkylsulfonyl group, a formyl group, a carboxy group, a cyano group, and a halogen atom;
   a substituent in which R^{m} is further substituted with one or more same or different R^{m}'s; or
   a solubilizing group.

### [Embodiment 10]

The photochromic composition according to any one of Embodiments 1 to 9, wherein R⁷ and R⁸ in General Formula B each independently represent a substituted or unsubstituted alkyl group having 1 to 20 carbon atoms.

### [Embodiment 11]

The photochromic composition according to any one of Embodiments 1 to 10, wherein R⁷ and R⁸ in General Formula B each independently represent a methyl group, an ethyl group, a propyl group, a butyl group, a pentyl group, or a hexyl group.

### [Embodiment 12]

The photochromic composition according to any one of Embodiments 1 to 11, wherein R¹³ and R¹⁴ in General Formula B each independently represent an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

### [Embodiment 13]

The photochromic composition according to any one of Embodiments 1 to 12, wherein R¹⁵ and R¹⁶ in General Formula C each independently represent a substituted or unsubstituted alkyl group having 1 to 20 carbon atoms.

### [Embodiment 14]

The photochromic composition according to any one of Embodiments 1 to 13, wherein R¹⁵ and R¹⁶ in General Formula C each independently represent a methyl group, an ethyl group, a propyl group, a butyl group, a pentyl group, or a hexyl group.

### [Embodiment 15]

The photochromic composition according to any one of Embodiments 1 to 14, wherein the electron-donating group represented by R²¹ or R²² in General Formula C is an electron-donating group selected from the group consisting of a methoxy group, an ethoxy group, a phenoxy group, a methylsulfide group, a phenylsulfide group, a dimethylamino group, a pyrrolidino group, a piperidino group, a morpholino group, and a thiomorpholino group.

### [Embodiment 16]

The photochromic composition according to any one of Embodiments 1 to 15, wherein a total content of the compound represented by General Formula B and the compound represented by General Formula C is more than a content of the compound represented by General Formula A on a mass basis.

### [Embodiment 17]

The photochromic composition according to any one of Embodiments 1 to 16, further comprising a polymerizable compound.

### [Embodiment 18]

A photochromic article comprising a cured product obtained by curing the photochromic composition according to Embodiment 17.

### [Embodiment 19]

The photochromic article according to Embodiment 18, further comprising:
a substrate; and
a photochromic layer which is the cured product.

### [Embodiment 20]

The photochromic article according to Embodiment 18 or 19, wherein the photochromic article is a spectacle lens.

### [Embodiment 21]

The photochromic article according to Embodiment 18 or 19, wherein the photochromic article is a lens for goggles.

### [Embodiment 22]

The photochromic article according to Embodiment 18 or 19, wherein the photochromic article is a visor portion of a sun visor.

### [Embodiment 23]

The photochromic article according to Embodiment 18 or 19, wherein the photochromic article is a shield member of a helmet.

### [Embodiment 24]

Spectacles comprising the spectacle lens according to Embodiment 19.

## Claims

1. A composition, which is a photochromic composition comprising one or more compounds of formula (A); one or more compounds of formula (B); and one or more compounds of formula (C): 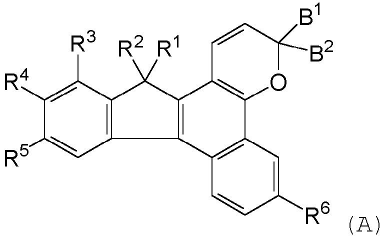 wherein R¹-R⁶, B¹ and B² each independently are H or a substituent, 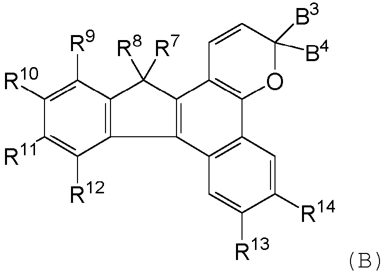 wherein R⁷-R¹², B³ and B⁴ each independently are H or a substituent, and R¹³ and R¹⁴ each independently are an electron-donating group, and 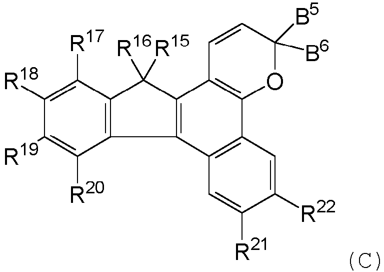 wherein R¹⁵-R²⁰, B⁵ and B⁶ each independently are H or a substituent, and one of R²¹ and R²² is H and the other is an electron-donating group.

2. The composition of claim 1, wherein R¹ and R² in formula (A) and/or R³ and R⁴ in formula (B) and/or R⁵ and R⁶ in formula (C) each independently are optionally substituted C₁₋₂₀-alkyl, preferably methyl, ethyl, propyl, butyl, pentyl or hexyl.

3. The composition of claim 1 or 2, wherein B¹ and B² in formula (A), B³ and B⁴ in formula (B), and B⁵ and B⁶ in formula (C) each independently are optionally substituted phenyl, and when the phenyl group has a plurality of substituents, the substituents may be bonded to form a ring.

4. The composition of any of claims 1-3, wherein R³-R⁶ in formula (A) each independently are H or an electron-withdrawing group, the electron-withdrawing group preferably being halogen, C₁₋₆-perfluoroalkyl, perfluorophenyl, perfluoroalkylphenyl or cyano, provided that at least one of R³-R⁶ is an electron-withdrawing group.

5. The composition of claim 4, wherein the halogen atom is F.

6. The composition of claim 4, wherein the perfluoroalkyl group is trifluoromethyl.

7. The composition of any of claims 1-6, wherein one or more selected from R²-R⁶, B¹ and B² in formula (A), R⁷-R¹², B³ and B⁴ in formula (B), and R¹⁵-R²⁰, B⁵ and B⁶ in formula (C) are
a substituent R^{m} selected from of hydroxy, linear or branched C₁₋₁₈-alkyl, single- or a multi-ring type cycloaliphatic alkyl (such as a bicyclic C₅₋₁₈-ring), linear or branched alkoxy having 1-24 constituent atoms, a non-aromatic cyclic substituent having 1-24 constituent atoms, linear or branched C₁₋₁₈-perfluoroalkyl, linear or branched perfluoroalkoxy, linear or branched alkylsulfide having 1-24 constituent atoms, aryl, aryloxy, arylsulfide, heteroaryl, amino, monoalkylamino, dialkylamino, monoarylamino, diarylamino, cyclic amino (such as piperidino, morpholino, thiomorpholino, tetrahydroquinolino and tetrahydroisoquinolino), ethynyl, mercapto, silyl, sulfonic acid, alkylsulfonyl, formyl, carboxy, cyano and halogen;
a substituent in which R^{m} is further substituted with one or more same or different group(s) R^{m}; or
a solubilizing group.

8. The composition of any of claims 1-7, wherein R¹³ and R¹⁴ in formula (B) each independently are an electron-donating group selected from methoxy, ethoxy, phenoxy, methylsulfide, phenylsulfide, dimethylamino, pyrrolidino, piperidino, morpholino and thiomorpholino.

9. The composition of any of claims 1-8, wherein the electron-donating group R²¹ or R²² in formula (C) is an electron-donating group selected from methoxy, ethoxy, phenoxy, methylsulfide, phenylsulfide, dimethylamino, pyrrolidino, piperidino, morpholino and thiomorpholino.

10. The composition of any of claims 1-9, wherein, on a mass basis, the total content of the compounds (B) and (C) is more than the content of the compound (A).

11. The composition of any of claims 1-10, further comprising a polymerizable compound.

12. A photochromic article comprising a cured product obtained by curing the composition of claim 11.

13. The photochromic article of claim 12, further comprising a substrate, and a photochromic layer which is the cured product.

14. The photochromic article of claim 12 or 13, wherein the photochromic article is a spectacle lens, a lens for goggles, a visor portion of a sun visor or a shield member of a helmet.

15. Spectacles comprising the spectacle lens of claim 14.
